# EUROPEAN PATENT APPLICATION

(11) **EP 4 477 069 A1**
(43) Date of publication of application: **18.12.2024**
(21) Application number: 23179909.9
(22) Date of filing: 16.06.2023
(51) Int. Cl.: A01H 1/02, A01H 6/82, C12N 9/22, C12N 15/82

(54) **UNREDUCED CLONAL GAMETE FORMATION AND POLYPLOID GENOME DESIGN IN THE SOLANACEAE**

(71) Applicant: Max-Planck-Gesellschaft zur Förderung der Wissenschaften e.V., 80539 München (DE)
(72) Inventor: UNDERWOOD, Charles J., 50829 Cologne (DE); WANG, Yazhong, 50829 Cologne (DE); MERCIER, Raphael, 50829 Cologne (DE)
(74) Representative: J A Kemp LLP

(57) **Abstract**

In contrast to meiosis, where meiotic recombination, random segregation of chromosomes and ploidy reduction occurs, *Mitosis instead of Meiosis* (*MiMe*), an engineered form of apomeiosis, leads to unreduced clonal gametes in the Solanaceae (nightshades), and, via selfing, to tetraploid offspring that stably inherit genome-wide heterozygosity and plant phenotypes. In the MiMe system, factors involved in meiotic recombination, sister chromatid cohesion, and meiosis II entry are knocked out or knocked down. Engineered clonal unreduced gamete formation by *MiMe* can be used as a component of synthetic apomixis, and to develop polyploid genome design.

## Description

### Field of the Invention

The present invention relates to the field of plant reproductive biology, and more particularly to plants from the Solanaceae (nightshade) family that are capable of apomeiosis.

### Background of the Invention

Hybrid vigour, otherwise known as heterosis, is a phenomenon that increases the yield and robustness of hybrid plants relative to their parents and is exploited in modern crop breeding (Hochholdinger, F. & Baldauf, J. A. Heterosis in plants. Current Biology vol. 28 R1089-R1092 (2018)). Hybrid varieties are preferred in most essential food categories, including grains (e.g., maize), vegetables (e.g., tomato), and oils (e.g., sunflower) (ter Steeg, E. M. S., Struik, P. C., Visser, R. G. F. & Lindhout, P. Crucial factors for the feasibility of commercial hybrid breeding in food crops. Nat. plants (2022) doi:10.1038/S41477-022-01142-W).

Autopolyploid progressive heterosis (APH) is a phenomenon where the combination of genomic segments from four distinct autopolyploid grandparents can have additional heterotic effects beyond biparental heterosis (Washburn, J. D. & Birchler, J. A. Polyploids as a 'model system' for the study of heterosis. Plant Reprod. 27, 1-5 (2014)). APH has been described in several crops including maize, potato and alfalfa, but has yet to be employed in commercial plant breeding because meiosis reassorts genotypes and therefore genetically consistent offspring that benefit from APH cannot be produced (Groose, R. W., Talbert, L. E., Kojis, W. P. & Bingham, E. T. Progressive heterosis in autotetraploid alfalfa: Studies using two types of inbreds. Crop Sci. 29, 1173-1177 (1989); Levings, C. S., Dudley, J. W. & Alexander, D. E. Inbreeding and Crossing in Autotetraploid Maize. Crop Sci. 7, 72-73 (1967); Mok, D. W. S. & Peloquin, S. J. Breeding value of 2n pollen (diplandroids) in tetraploid x diploid crosses in potatoes. Theor. Appl. Genet. 46, 307-314 (1975)). Routes to perform Polyploid Genome Design where several pre-defined genome haplotypes can be combined in a controlled manner could enable APH in crop species.

The *Mitosis instead of Meiosis (MiMe)* system previously established in Arabidopsis and rice leads to clonal, unreduced gametes (Mieulet, D. et al. Turning rice meiosis into mitosis. Nat. Publ. Gr. 26, 1242-1254 (2016); d'Erfurth, I. et al. Turning Meiosis into Mitosis. PLoS Biol. 7, e1000124 (2009); Wang, Y., van Rengs, W. M. J., Zaidan, M. W. A. M. & Underwood, C. J. Meiosis in crops: from genes to genomes. J. Exp. Bot. (2021) doi:10.1093/JXB/ERAB217). In Arabidopsis, MiMe can be established through the mutation of three meiotic genes involved in meiotic recombination initiation (*AtSP011-1*), sister chromatid segregation (*AtREC8*) and cell cycle control (*AtOSD1*) (d'Erfurth, I. et al. Turning Meiosis into Mitosis. PLoS Biol. 7, e1000124 (2009); Wang, Y., van Rengs, W. M. J., Zaidan, M. W. A. M. & Underwood, C. J. Meiosis in crops: from genes to genomes. J. Exp. Bot. (2021) doi:10.1093/JXB/ERAB217), and combined with CENH3-mediated maternal haploid induction to generate clonal offspring (Marimuthu, M. P. A. et al. Synthetic clonal reproduction through seeds. Science (80-. ). 331, 876 (2011); 11. Marimuthu, M. P. A. et al. Epigenetically mismatched parental centromeres trigger genome elimination in hybrids. Sci. Adv. 7, 1151 (2021)).

In rice, a similar MiMe system (involving mutations in *OsPAIR1, OsREC8, OsOSD1)* (Mieulet, D. et al. Turning rice meiosis into mitosis. Nat. Publ. Gr. 26, 1242-1254 (2016)) has also been combined with *MATL*-mediated maternal haploid induction (Wang, C. et al. Clonal seeds from hybrid rice by simultaneous genome engineering of meiosis and fertilization genes. Nat. Biotechnol. 37, 283-286 (2019)) and *OsBBM1-*mediated parthenogenetic activation of embryogenesis in egg cells (Khanday, I., Skinner, D., Yang, B., Mercier, R. & Sundaresan, V. A male-expressed rice embryogenic trigger redirected for asexual propagation through seeds. Nature 565, 91-95 (2019)) to generate clonal offspring. However, variation in *OSD1* copy number is found among plant species, representing a challenge for the establishment of *MiMe* in other plants.

Alternative mutants that skip the second meiotic division in Arabidopsis including *tam* (d'Erfurth, I. et al. The CYCLIN-A CYCA1;2/TAM Is Required for the Meiosis I to Meiosis II Transition and Cooperates with OSD1 for the Prophase to First Meiotic Division Transition. PLOS Genet. 6, e1000989 (2010)) and a *tdm1* (Cifuentes, M. et al. TDM1 Regulation Determines the Number of Meiotic Divisions. PLoS Genet. 12, (2016)) point mutant have yet to be explored in crops. Further to this, MiMe and synthetic apomixis systems have yet to be described in any dicot crop species, nor has the novel *dmp* maternal haploid inducer been tested as a component of synthetic apomixis (Wang, Y. & Underwood, C. J. Apomixis. Curr. Biol. 33, R293-295 (2023); Underwood, C. J. & Mercier, R. Engineering Apomixis: Clonal Seeds Approaching the Fields. https://doi.org/10.1146/annurev-arplant-102720-013958 73, (2022); Stokstad, E. Unmixed blessing. Science (80-. ). 380, 684-687 (2023)).

Relevant teaching can also be found in US 2012/0042408 A1, which discloses plants producing 2n gametes or apomeiotic gametes, in US 2014/0298507 A1, which disclosed synthetic clonal reproduction through seeds, and in US 2021/0363537 A1, which discloses a method for using plant heterosis.

The present inventors found that, in the Solanaceae (nightshades), loss-of-function *tam* mutants can skip the second meiotic cell division and can generate tetraploid offspring by selfing. On the other hand, the inventors also found that, unlike in Arabidopsis and rice, *osd1* mutants could not be generated in the Solanaceae (such as tomato) and therefore could not be used to skip the second meiotic division. The present inventors established a MiMe system in the Solanaceae, using inbred tomato as an example, by targeting *SISPO11-1, SIREC8* and *SITAM.* Subsequently, the present inventors implemented the system in three hybrid tomato genotypes and demonstrated its potential to propagate genome-wide heterozygosity through clonal gametes. By combining hybrid MiMe with the maternal haploid inducer *sldmp,* the present inventors were able to obtain a clonal plant via embryo rescue where genetic material was inherited from the mother alone. In addition, the present inventors established a paradigm for Polyploid Genome Design through the hybridization of two distinct hybrid MiMe genotypes. This led to the controlled production of "four-Haplotype" tetraploid plants that contain the complete genome sequences of their four inbred grandparents.

### Summary of the Invention

The invention provides a method of generating a parent plant capable of apomeiosis, so as to produce viable clonal male and female gametes having the same genome ploidy level as the parent plant, the method comprising: reducing the level or activity of an endogenous REC8 polypeptide in the parent plant or a cell thereof, so as to ablate sister chromatid cohesion; reducing the level or activity of an endogenous TAM polypeptide in the parent plant or a cell thereof, so as to prevent entry to the second meiotic division; and reducing the level or activity of an endogenous SPO11-1, SPO11-2, MTOPVIB, PRD1, PRD2, PRD3, or DFO polypeptide in the parent plant or a cell thereof, so as to ablate meiotic recombination; wherein the parent plant is from the Solanaceae (nightshade) family.

The invention further provides a parent plant obtainable by the method of the invention, as well as a plant part and plant cell thereof, and microspores and seeds obtained therefrom.

Yet further provided by the invention is parent plant capable of apomeiosis, so as to produce viable clonal male and female gametes having the same genome ploidy level as the parent plant, having: a reduced level or activity of an endogenous REC8 polypeptide, so as to ablate sister chromatid cohesion; a reduced level or activity of an endogenous TAM polypeptide, so as to prevent entry to the second meiotic division; and a reduced level or activity of an endogenous SPO11-1, SPO11-2, MTOPVIB, PRD1, PRD2, PRD3, or DFO polypeptide, so as to ablate meiotic recombination; wherein the parent plant is from the Solanaceae family.

The invention further provides a plant part or plant cell of the parent plant of the invention, as well as a plant part and plant cell thereof. Yet further provided by the invention are microspores and seeds obtained from such a plant part or plant cell. Also provided by the invention is a plant grown from such a microspore or seed.

### Brief Description of the Figures

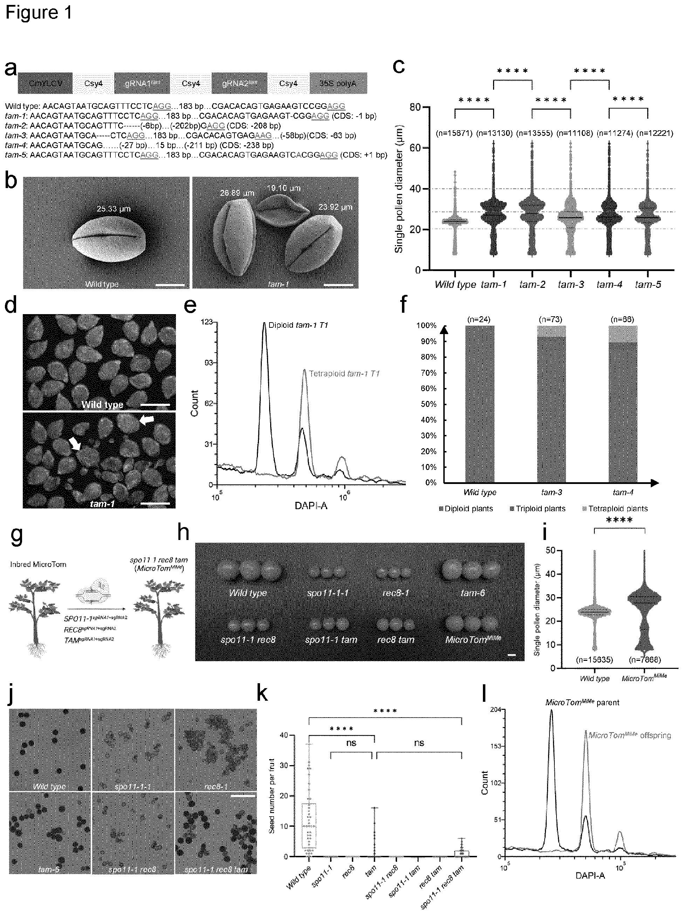

**Figure 1****.** Unreduced male and female gametes in *sltam* mutants and a *Mitosis instead of Meiosis* system in inbred tomato. (a) Schematic representation of CRISPR-Cas9 construct targeting *SITAM* gene in tomato. One Pol II promoter (CmYLCV) drives the expression of two sgRNAs (separated by a Csy4 spacer for gRNA production) that target the second exon of the *SITAM* gene. The Protospacer Adjacent Motif (PAM) motif NGG for both gRNA targets is highlighted with red and underlining. The sequence of wild type and five different alleles is shown. (b) scanning electron microscope for wild type pollen and *tam-1* single mutant pollen. Scale bar=10 µm. (c) Single pollen diameter distribution from single opening flower between wild type and five independent alleles of *Sltam.* Wild-type pollen size region (20.03-28.15 µm) and diploid pollen size region (28.15-40.23 µm) are highlighted by the red dotted lines. The data analysis was conducted with Ordinary one-way ANOVA followed by the Dunnett test for multiple comparisons. P value used Dunnett's multiple comparisons test (Definition of statical significance: P<0.05) and P value summary (****) <0.0001 means significantly different. The violin plot showed all points, medians showed in black solid lines, and quartiles showed in black dashed lines. (d) high resolution picture of wild type and *Sltam-1* mutant selfing seeds. The red arrows indicate bigger seeds that give rise to tetraploid offspring. Scale bars=3 mm. (e) Flow cytometry result of diploid (black) and tetraploid (red) selfing offspring of the *Sltam-1* T0 plant. Y axis of the histogram represents the events number and X axis of the histogram represents the intensity of DAPI. (f) Ploidy level of offspring from wild type and two different *Sltam* alleles. (g) Schematic workflow of the generation of *MiMe* (*spo11-1 rec8 tam)* triple mutants in Micro-Tom background. Six sgRNAs in were used to target *SISPO11-1, SIREC8* and *SITAM* in one single construct. (h) Fruits shape overview of wild type, *spo11-1-1, rec8-1-1, tam-6, spo11-1 rec8, spo11-1 tam, rec8 tam* and *spo11-1 rec8 tam* mutants. Scale bar=1 cm. (i) Single pollen diameter distribution from single flowers of wild type and triple *spo11-1 rec8 tam* mutant. The data analysis was conducted with unpaired t-tests and P value was used Two-tailed approach (Definition of statical significance: P<0.05) and P value summary (****) <0.0001 means significantly different. The violin plot showed all points, medians showed in black solid lines, and quartiles showed in black dashed lines. (j) Alexander staining results of wild type, single *spo11-1-1* mutants, single *rec8-1* mutants and triple *spo11-1 rec8 tam* mutants' pollen. Scale bar=200 µm. (k) Seed number per single fruit of wild type, *spo11-1, rec8, tam, spo11-1 rec8, spo11-1 tam, rec8 tam* and *spo11-1 rec8 tam* mutants. Each dot indicates the seed number of an individual fruit and the medians of the Box and whiskers for each genotype is represented by a solid line. P values are from Ordinary one-way ANOVA followed by the Sidak test for multiple comparisons. 'ns' means no significance, and P value (****) <0.0001 means significantly different. (l) Flow cytometry analysis of diploid parent *MicroTom^{MiMe}* (black) and tetraploid *MicroTom^{MiMe}* offspring (red). Y axis of the histogram represents the events number and X axis of the histogram represents the intensity of DAPI.

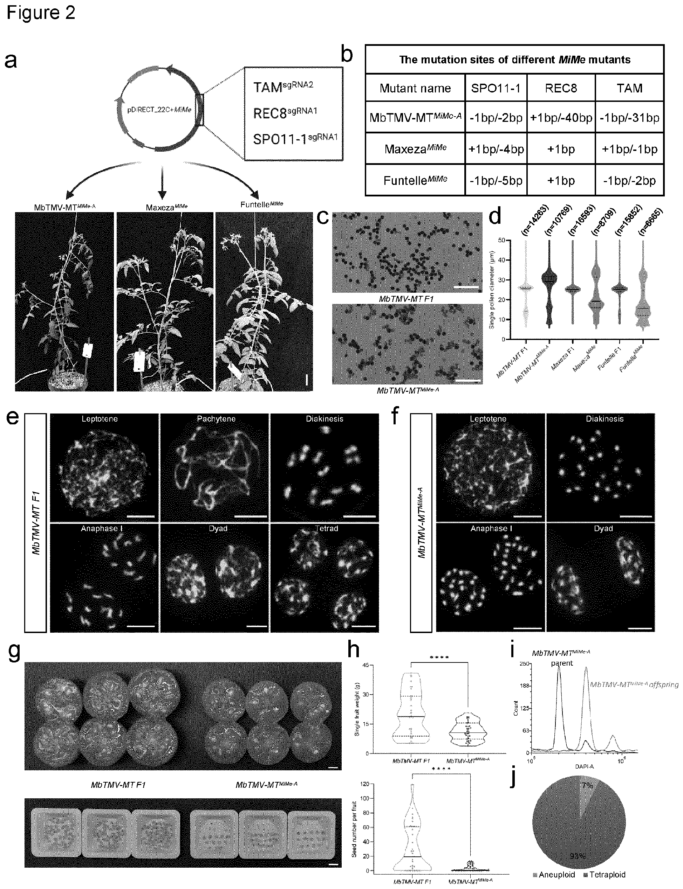

**Figure 2****.** *Mitosis instead of Meiosis* in three hybrid tomato genotypes. (a) Schematic workflow of generating MiMe systems in three hybrid tomato genotypes (MbTMV-MT F1, Funtelle F1 and Maxeza F1). A single construct that contained CAS9 and three single guide RNAs targeting *SITAM, SlREC8* and *SlSPO11-1* was transformed to the three different hybrid genotypes (MbTMV-MT F1, Funtelle F1 and Maxeza F1). Created with BioRender.com. (b) The sgRNA target site mutations in *SlSPO11-1, SIREC8* and *SITAM* in three hybrid tomato genotypes. (c) Alexander staining results of control MbTMV-MT F1 and *MbTMV-MT^{MiMe-A}* mutant pollen. Scale bar=200 µm. (d) Single pollen diameter distribution of single flowers in three F1 hybrid control plants and three hybrid MiMe mutants. (e) Chromosome behaviour of male meiocytes in control MbTMV-MT F1, including Leptotene, Pachytene, Diakinesis, Anaphase I, Dyad and Tetrad. Scale bar=10 µm. (f) Chromosome behaviour of male meiocytes in hybrid *MbTMV-MT^{MiMe-A}* including Leptotene, Diakinesis, Anaphase I and Dyad. Scale bar=10 µm. (g) Transverse anatomical view and seed number of fruits from MbTMV-MT F1 and hybrid *MbTMV-MT^{MiMe-A}.* Scale bar=1 cm. (h) Single fruit weight and seed number per fruit between hybrid MbTMV-MT F1 (n=74) and hybrid *MbTMV-MT^{MiMe-A}* (n=75) . P value was calculated using independent two-sample unpaired t-tests. Madian was showed in solid black line and quartiles were showed in dotted balck lines. P value summary (****) <0.0001 means significantly different. (i) Flow cytometry analysis of diploid parent *MbTMV-MT^{MiMe-A}* (black) and tetraploid *MbTMV-MT^{MiMe-A}* offspring (red). Y axis of the histogram represents the events number and X axis of the histogram represents the intensity of DAPI. (j) The ploidy level of *MbTMV-MT^{MiMe-A}* offspring. Tetraploid plants (n=77) are shown in blue and aneuploid plants (n=6) are showed in green.

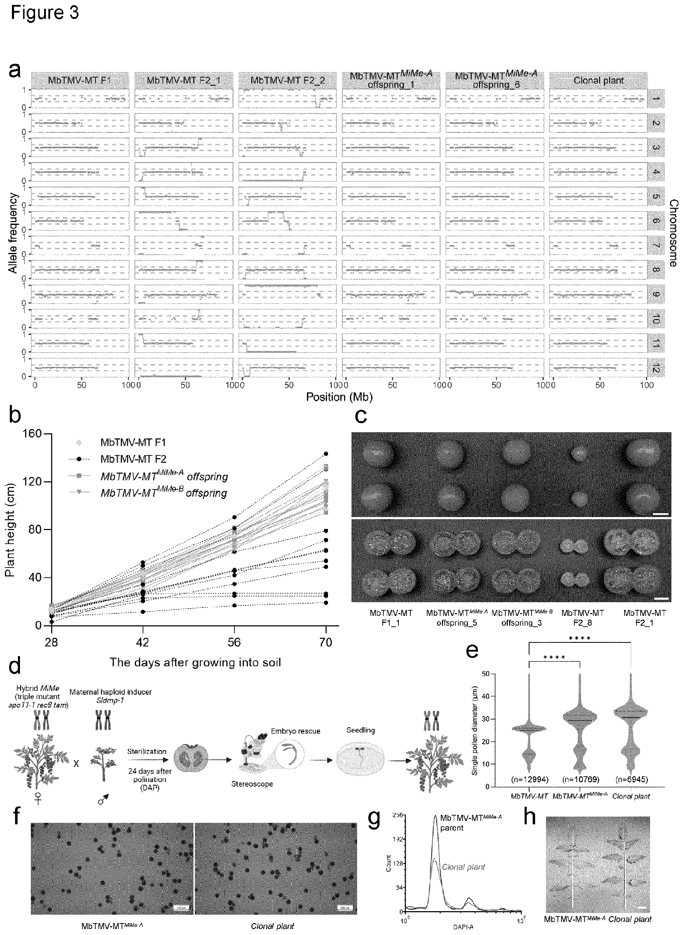

**Figure 3****.** Stable inheritance of genome-wide heterozygosity and plant phenotypes through seeds. (a) Whole genome sequencing and allele frequency analysis of a single diploid MbTMV-MT F1 plant, two diploid MbTMV-MT F2 plants, two tetraploid *MbTMV-MT^{MiMe-A}* offspring, and a diploid clonal plant (a product of *MbTMV-MT^{MiMe-A}* pollination by *sldmp* mutant). The allele frequency distribution of Moneyberg TMV (0 on Y axis) and Micro-Tom (1 on Y axis) is shown along the 12 chromosomes of tomato variety MbTMV (van Rengs et al., 2022). (b) Plant height measurements of MbTMV-MT F1, MbTMV-MT F2 and two different *MbTMV-MT^{MiMe}* offspring populations. (c) The fruit morphology and transverse anatomical view of MbTMV-MT F1, *MbTMV-MT^{MiMe-A}* offspring; *MbTMV-MT^{MiMe-B}* offspring and MbTMV-MT F2 offspring population. Scale bars=2 cm. (d) Schematic outline for isolating a diploid clonal offspring from *MbTMV-MT^{MiMe-A}.* The pollen of *Sldmp* mutant (maternal haploid inducer) was used to pollinate emasculated flowers of *MbTMV-MT^{MiMe-A}* and 24 days after the pollination the fruits were collected. The embryos were rescued from the fruit and cultured *in vitro* and allowed to develop into plants. Created with BioRender.com. (e) Single pollen diameter distribution for MbTMV-MT F1, *MbTMV-MT^{MiMe-A}* and the clonal plant. The data analysis was conducted with Ordinary one-way ANOVA followed by the Dunnett test for multiple comparisons. P value was used Dunnett's multiple comparisons test (Definition of statical significance: P<0.05) and P value summary (****) <0.0001 means significantly different. The violin plot showed all points, medians showed in black solid lines, and quartiles showed in black dashed lines. (f) Alexander staining results of pollen from *MbTMV-MT^{MiMe-A}* and the clonal plant. Scale bars=100 µm. (g) Flow cytometry analysis of diploid parent *MbTMV-MT^{MiMe-A}* (black) and the diploid clonal plant (red). Y axis of the histogram represents the events number and X axis of the histogram represents the intensity of DAPI. (h) The leaf morphology of *MbTMV-MT^{MiMe-A}* and the clonal plant, scale bar=2 cm.

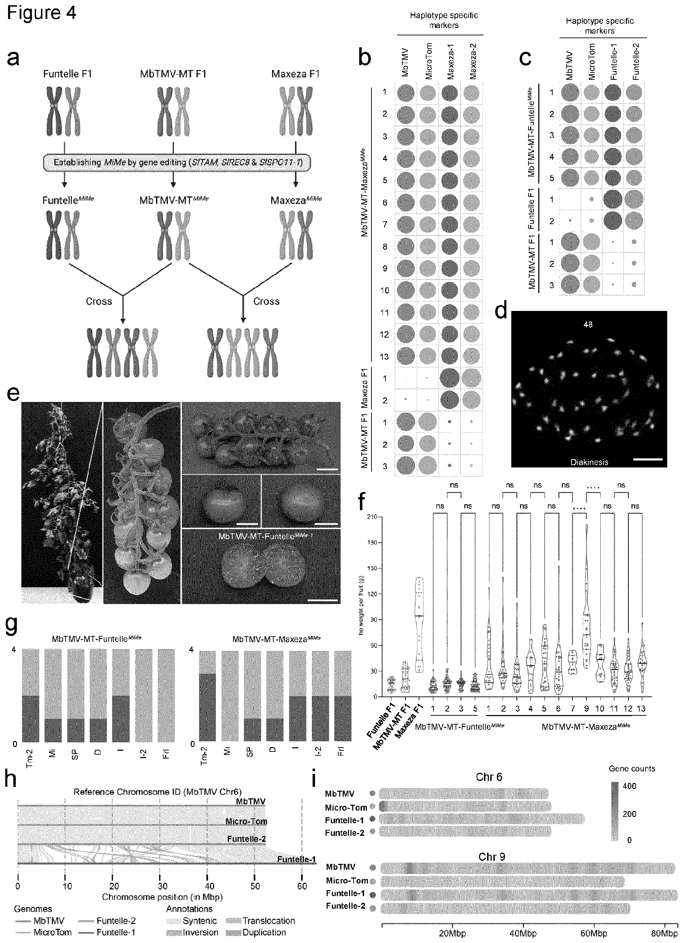

**Figure 4****.** Polyploid Genome Design and generation of tetraploid hybrid plants containing four unique genome haplotypes. (a) Schematic workflow for generating tetraploid "4-Haplotype" (4-H) plants that contained the complete genetic repertoire of their four inbred grandparents. Hybrid MiMe plants were used for hybridization (MbTMV-MT*^{MiMe}* × Maxeza*^{MiMe-A}*; MbTMV-MT*^{MiMe}* × Funtelle*^{MiMe}*) to produce two different types of tetraploid hybrid plants in tomato. Created with BioRender.com. (b) Presence of haplotype specific markers (MbTMV, Micro-Tom, Maxeza haplotype 1, Maxeza haplotype 2) in 13 MbTMV-MT*^{MiMe}* × Maxeza*^{MiMe}* offspring, 2 Maxeza F1 plants and 3 MbTMV-MT F1 plants. Colours represent each haplotype tested and size of circle represents the % of markers found (a complete circle filling the whole square illustrates 100% of all markers were found while no circle represents 0% of markers were found). (c) Presence of haplotype specific markers (MbTMV, Micro-Tom, Funtelle haplotype 1, Funtelle haplotype 2) in 5 MbTMV-MT*^{MiMe}* x Funtelle*^{MiMe}* offspring, 2 Funtelle F1 plants and 3 MbTMV-MT F1 plants. Colours represent each haplotype tested and size of circle represents the % of markers found (a complete circle filling the whole square illustrates 100% of all markers were found while no circle represents 0% of markers were found). (d) Chromosome spreading results of male meiocytes from a tetraploid MbTMV-MT*^{MiMe}* x Funtelle*^{MiMe}* offspring plant at Diakinesis stage. 48 chromosomes are clearly identifiable. Scale bar=10 µm. (e) Images of a tetraploid MbTMV-MT*^{MiMe}* x Funtelle*^{MiMe}* offspring plant. Whole plant morphology (left), the structure of a branch with ripening tomato fruits (middle), fully ripened fruits (top right), harvested fruits (middle right), cut fruits (bottom right). Scale bars=2.5 cm. (f) Single fruit weights of MbTMV-MT F1, Funtelle F1, Maxeza F1, 12 tetraploid MbTMV-MT-Maxeza*^{MiMe}* plants and 4 tetraploid MbTMV-MT-Funtelle*^{MiMe}* plants. (g) The gene dosage distribution within 4-H plants regarding Tomato mosaic virus resistance (*Tm*-2², Solyc09g018220), Meloidogyne incognita (Mi, Solyc06g008720), Self-pruning (SP, Solyc06g074350), dwarf (*D*, Solyc02g089160), *l* gene (Solyc11g011180), *l-*2 gene (Solyc11g071430) and Fusarium oxysporum f. sp. radicis-lycopersici (*Frl*) resistances. (h) Genomic rearrangements on Chr6 for Meloidogyne incognita (*Mi*) resistance. Moneyberg TMV (blue), Micro-Tom (yellow), Funtelle-1 haplotype (purple) and Funtelle-2 haplotype (green) are shown in coloured lines. The specific structures including Syntenic, Inversion, Translocation and Duplication are highlighted with grey, yellow, light green and blue boxes respectively. (i) The MbTMV-MT-Funtelle*^{MiMe}* 4-H plant gained a large number of wild genes due to the *Mi* introgession region on chromosome 6 and the TMV introgression region on chromosome 9.

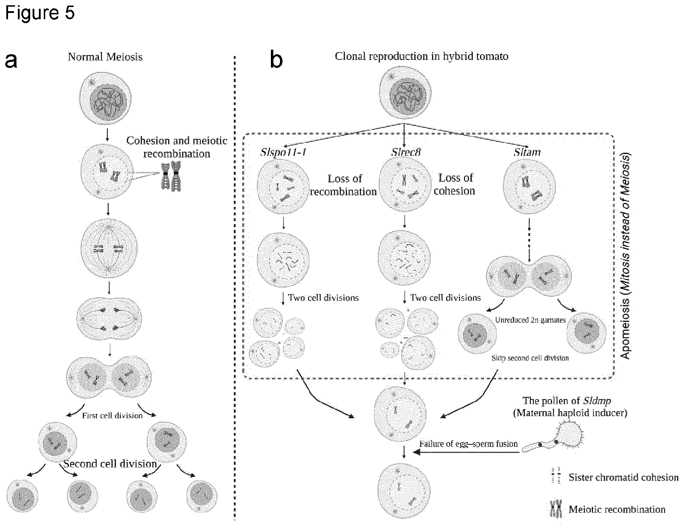

**Figure 5****.** Proposed model for clonal reproduction in hybrid tomato. (a) Normal meiosis process with meiotic recombination. (b) Clonal reproduction in hybrid tomato. Created with BioRender.com.

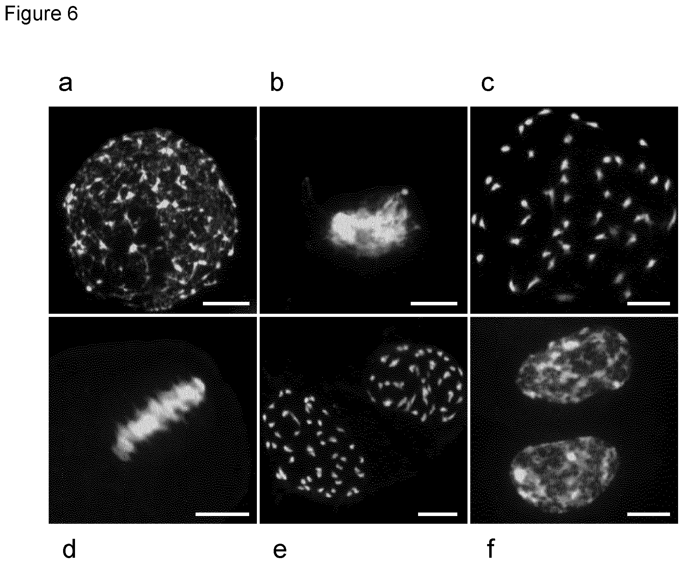

**Figure 6****.** The chromosome behaviour of male meiocytes in a MbTMV-MT-Maxeza*^{MiMe-6}* mutant. Chromosome behaviors of male meiocytes about MbTMV-MT-Maxeza*^{MiMe-6}* mutant during (a) Leptotene; (b) Meiosis-to-Mitosis transition stage; (c) Diakinesis; (d) Metaphase I; (e) Anaphase I and (f) Dyad. Scale bar= 10 µm.

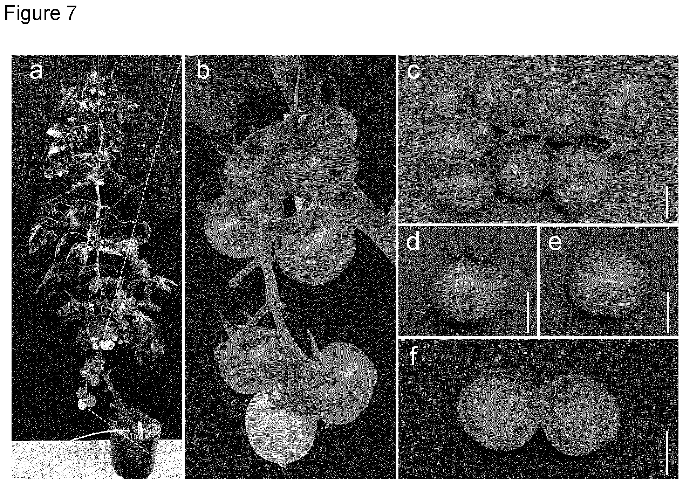

**Figure 7****.** Plant morphology of MbTMV-MT-Maxeza*^{MiMe-6}* mutant. a) Plant morphology of MbTMV-MT-Maxeza*^{MiMe}*.-6. b) The structure of a single branch with ripening fruits. c) Single branch with fully mature fruits. Scale bar= 2.5 cm. d) Overview image of a single ripe fruit with pedicel and sepal. Scale bar= 2.5 cm. e) Overview image of a single ripe fruit without pedicel and sepal. Scale bar= 2.5 cm. f) Transverse anatomical view of single fruit. Scale bar= 2.5 cm.

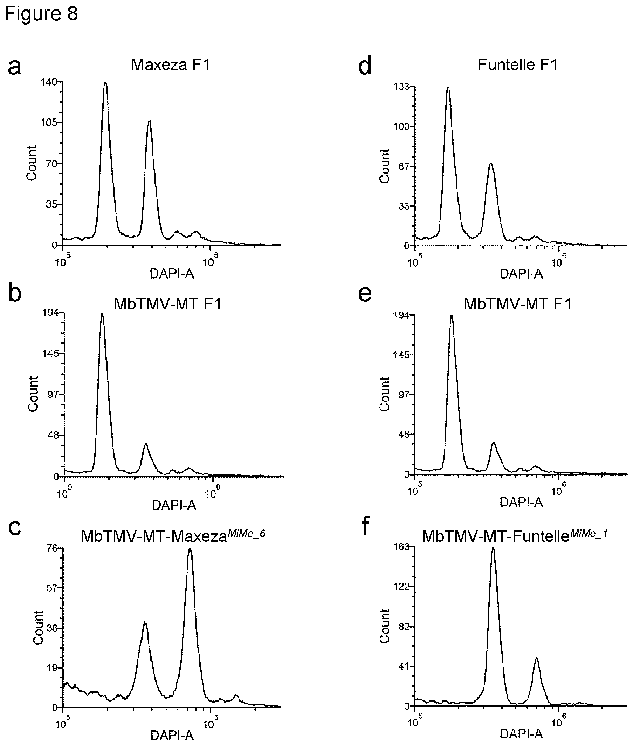

**Figure 8****.** The ploidy level of wild type and 4-haplotype plants. The ploidy level of diploid F1 hybrid wild type (a. Maxeza F1, b,e. MbTMV-MT F1, d. Funtelle F1), and c. tetraploid MbTMV-MT-Maxeza*^{MiMe_6}* and f. tetraploid MbTMV-MT-Funtelle*^{MiMe_1}.*

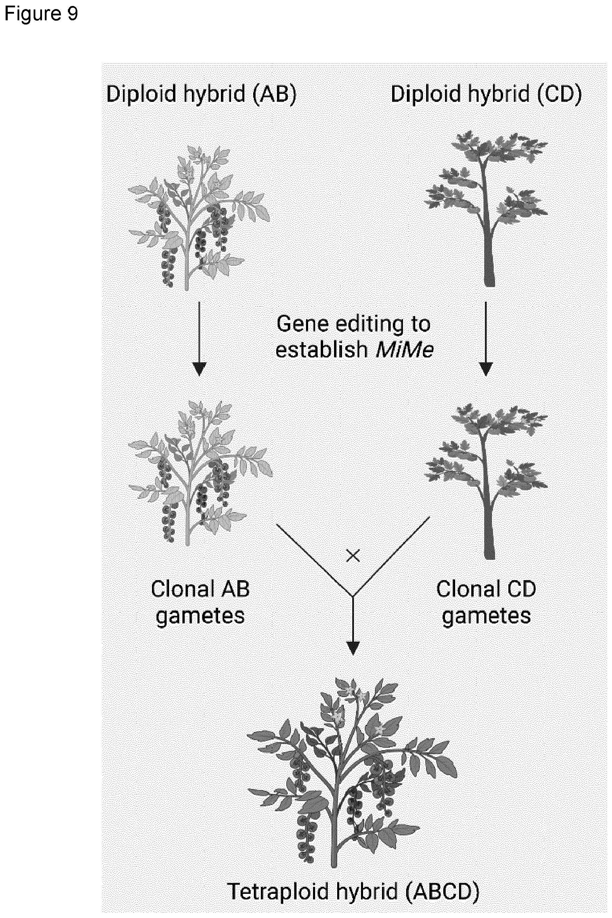

**Figure 9****.** The proposed working model of application of Polyploid Genome Design in plant breeding. Through the controlled production of clonal unreduced gametes via *MiMe* (or other apomeiosis approaches) combined with hybridization it is possible to generate 4-haplotype (4-H) plants. These plants will maintain the complete heterozygosity of the immediate parents, and if the parents were created from inbred parental lines, the complete genetic repertoire of the four grandparental lines. This approach opens up new opportunities for increasing ploidy level while maintaining genetic diversity, for the creation of seedless triploids with high heterozygosity, and facilitating the improvement of hybrid allotetraploid crops. Created with BioRender.com.

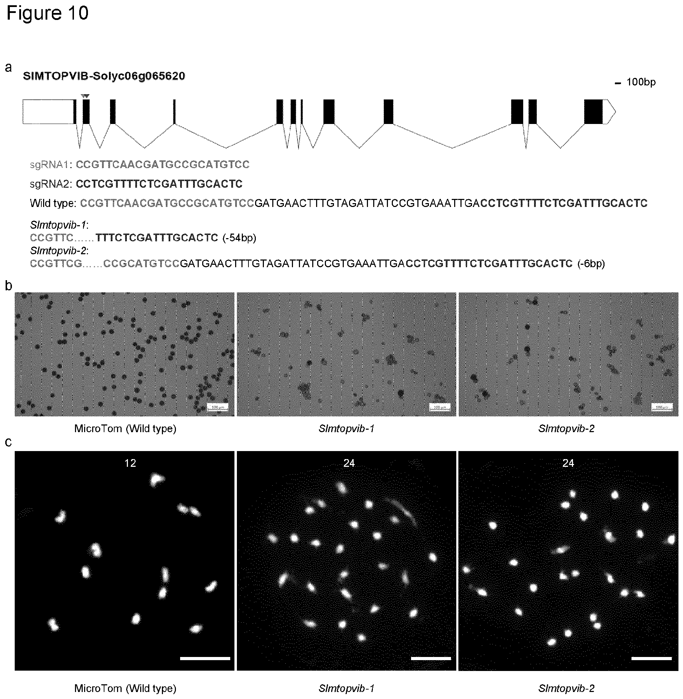

**Figure 10****.** *Slmtopvib* is required for DNA double strand break (DSB) formation in tomato. a. The gene structure of *SIMTOPVIB* and specific mutation sites of two *Slmtopvib* alleles. b. Alexander staining results of wild type and two *Slmtopvib* mutants' pollen. Scale bars= 100 µm. c. Chromosome spreading result between wild type and *Slmtopvib* two mutants' meiocytes at Diakinesis. Scale bars= 10 µm.

### Brief Description of the Sequences

**Table 1 - Overview of genomic DNA, cDNA, and protein sequences.**

| | | **Tomato *(Solanum lycopersicum)*** | **Potato *(Solanum tuberosum)*** | **Eggplant *(Solanum melongena)*** | **Pepper *(Capsicum annuum)*** |
|---|---|---|---|---|---|
| **SPO11-1** | Genomic DNA | SEQ ID NO: 1 | SEQ ID NO: 28 | SEQ ID NO: 55 | SEQ ID NO: 82 |
| | cDNA | SEQ ID NO: 2 | SEQ ID NO: 29 | SEQ ID NO: 56 | SEQ ID NO: 83 |
| | Protein | SEQ ID NO: 3 | SEQ ID NO: 30 | SEQ ID NO: 57 | SEQ ID NO: 84 |
| **REC8** | Genomic DNA | SEQ ID NO: 4 | SEQ ID NO: 31 | SEQ ID NO: 58 | SEQ ID NO: 85 |
| | cDNA | SEQ ID NO: 5 | SEQ ID NO: 32 | SEQ ID NO: 59 | SEQ ID NO: 86 |
| | Protein | SEQ ID NO: 6 | SEQ ID NO: 33 | SEQ ID NO: 60 | SEQ ID NO: 87 |
| **TAM** | Genomic DNA | SEQ ID NO: 7 | SEQ ID NO: 34 | SEQ ID NO: 61 | SEQ ID NO: 88 |
| | cDNA | SEQ ID NO: 8 | SEQ ID NO: 35 | SEQ ID NO: 62 | SEQ ID NO: 89 |
| | Protein | SEQ ID NO: 9 | SEQ ID NO: 36 | SEQ ID NO: 63 | SEQ ID NO: 90 |
| **SPO11-2** | Genomic DNA | SEQ ID NO: 10 | SEQ ID NO: 37 | SEQ ID NO: 64 | SEQ ID NO: 91 |
| | cDNA | SEQ ID NO: 11 | SEQ ID NO: 38 | SEQ ID NO: 65 | SEQ ID NO: 92 |
| | Protein | SEQ ID NO: 12 | SEQ ID NO: 39 | SEQ ID NO: 66 | SEQ ID NO: 93 |
| **MTOPVIB** | Genomic DNA | SEQ ID NO: 13 | SEQ ID NO: 40 | SEQ ID NO: 67 | SEQ ID NO: 94 |
| | cDNA | SEQ ID NO: 14 | SEQ ID NO: 41 | SEQ ID NO: 68 | SEQ ID NO: 95 |
| | Protein | SEQ ID NO: 15 | SEQ ID NO: 42 | SEQ ID NO: 69 | SEQ ID NO: 96 |
| **PRD1** | Genomic DNA | SEQ ID NO: 16 | SEQ ID NO: 43 | SEQ ID NO: 70 | SEQ ID NO: 97 |
| | cDNA | SEQ ID NO: 17 | SEQ ID NO: 44 | SEQ ID NO: 71 | SEQ ID NO: 98 |
| | Protein | SEQ ID NO: 18 | SEQ ID NO: 45 | SEQ ID NO: 72 | SEQ ID NO: 99 |
| **PRD2** | Genomic DNA | SEQ ID NO: 19 | SEQ ID NO: 46 | SEQ ID NO: 73 | SEQ ID NO: 100 |
| | cDNA | SEQ ID NO: 20 | SEQ ID NO: 47 | SEQ ID NO: 74 | SEQ ID NO: 101 |
| | Protein | SEQ ID NO: 21 | SEQ ID NO: 48 | SEQ ID NO: 75 | SEQ ID NO: 102 |
| **PRD3** | Genomic DNA | SEQ ID NO: 22 | SEQ ID NO: 49 | SEQ ID NO: 76 | SEQ ID NO: 103 |
| | cDNA | SEQ ID NO: 23 | SEQ ID NO: 50 | SEQ ID NO: 77 | SEQ ID NO: 104 |
| | Protein | SEQ ID NO: 24 | SEQ ID NO: 51 | SEQ ID NO: 78 | SEQ ID NO: 105 |
| **DFO** | Genomic DNA | SEQ ID NO: 25 | SEQ ID NO: 52 | SEQ ID NO: 79 | SEQ ID NO: 106 |
| | cDNA | SEQ ID NO: 26 | SEQ ID NO: 53 | SEQ ID NO: 80 | SEQ ID NO: 107 |
| | Protein | SEQ ID NO: 27 | SEQ ID NO: 54 | SEQ ID NO: 81 | SEQ ID NO: 108 |
| **DMP** | Genomic DNA | SEQ ID NO: 145 | SEQ ID NO: 148 | SEQ ID NO: 151 | SEQ ID NO: 154 |
| | cDNA | SEQ ID NO: 146 | SEQ ID NO: 149 | SEQ ID NO: 152 | SEQ ID NO: 155 |
| | Protein | SEQ ID NO: 147 | SEQ ID NO: 150 | SEQ ID NO: 153 | SEQ ID NO: 156 |

All of the genomic DNA sequences provided herein include 1kb upstream and 1kb downstream with respect to the coding sequence. Although not explicitly included in the Sequence Listing, genomic DNA sequences that exclude 1 kb upstream and/or 1kb downstream of genomic DNA with respect to the coding sequence are also provided herein and may find use in any of the embodiments of the invention.

Although not explicitly included in the Sequence Listing, reverse complement versions of any one of the cDNA sequences listed above are also provided herein and may find use in any of the embodiments of the invention.

**Table 2 - Overview of exemplary guide RNA sequences.**

| | **SPO11-1** | | **REC8** | | **TAM** | |
|---|---|---|---|---|---|---|
| | sgRNA1 | sgRNA2 | sgRNA1 | sgRNA2 | sgRNA1 | sgRNA2 |
| **Tomato *(Solanum lycopersicum)*** | SEQ ID NO: 109 | SEQ ID NO: 110 | SEQ ID NO: 111 | SEQ ID NO: 112 | SEQ ID NO: 113 | SEQ ID NO: 114 |
| **Potato *(Solanum tuberosum)*** | SEQ ID NO: 127 | SEQ ID NO: 128 | SEQ ID NO: 129 | SEQ ID NO: 130 | SEQ ID NO: 131 | SEQ ID NO: 132 |
| | | | | | | |

| | **SPO11-2** | | **MTOPVIB** | | **PRD1** | |
|---|---|---|---|---|---|---|
| | sgRNA1 | sgRNA2 | sgRNA1 | sgRNA2 | sgRNA1 | sgRNA2 |
| **Tomato *(Solanum lycopersicum)*** | SEQ ID NO: 115 | SEQ ID NO: 116 | SEQ ID NO: 117 | SEQ ID NO: 118 | SEQ ID NO: 119 | SEQ ID NO: 120 |
| **Potato *(Solanum tuberosum)*** | SEQ ID NO: 133 | SEQ ID NO: 134 | SEQ ID NO: 135 | SEQ ID NO: 136 | SEQ ID NO: 137 | SEQ ID NO: 138 |
| | | | | | | |

| | **PRD2** | | **PRD3** | | **DFO** | |
|---|---|---|---|---|---|---|
| | sgRNA1 | sgRNA2 | sgRNA1 | sgRNA2 | sgRNA1 | sgRNA2 |
| **Tomato *(Solanum lycopersicum)*** | SEQ ID NO: 121 | SEQ ID NO: 122 | SEQ ID NO: 123 | SEQ ID NO: 124 | SEQ ID NO: 125 | SEQ ID NO: 126 |
| **Potato *(Solanum tuberosum)*** | SEQ ID NO: 139 | SEQ ID NO: 140 | SEQ ID NO: 141 | SEQ ID NO: 142 | SEQ ID NO: 143 | SEQ ID NO: 144 |
| | | | | | | |

| | **DMP** | | | | | |
|---|---|---|---|---|---|---|
| | sgRNA1 | sgRNA2 | | | | |
| **Tomato *(Solanum lycopersicum)*** | SEQ ID NO: 157 | SEQ ID NO: 158 | | | | |
| **Potato *(Solanum tuberosum)*** | SEQ ID NO: 159 | SEQ ID NO: 160 | | | | |

### Detailed Description of the Invention

Many important crop species are cultivated as polyploids (including potato, wheat, rapeseed, sugarcane, sugar beet, banana, coffee, peanut and strawberry). Polyploidy presents possibilities for genetic novelty and phenotypic robustness, but polyploid breeding is difficult using traditional breeding methods due to the increased genetic complexity of working with three or more sets of homologous chromosomes.

Engineered clonal unreduced gamete formation can be used as a component of synthetic apomixis, yet a wider potential for the generation of highly heterozygous and heterotic polyploid genotypes remains untested in any plant species. The present invention prevents the breakdown of optimal parental genotypes and phenotypes that occurs during meiosis (due to recombination and random segregation of chromosomes) and could thereby expedite the breeding of polyploid crop varieties. This application of clonal unreduced gametes can preserve genetic heterozygosity and heterosis, which are important for the vigor of crop varieties.

Conventional double-cross systems between four different tetraploid grandparents can achieve autopolyploid progressive heterosis (APH) but the reshuffling of genetic information occurs (ter Steeg, E. M. S., Struik, P. C., Visser, R. G. F. & Lindhout, P. Crucial factors for the feasibility of commercial hybrid breeding in food crops. Nat. plants (2022) doi:10.1038/S41477-022-01142-W; Washburn JD, Birchler JA. Polyploids as a "model system" for the study of heterosis. Plant Reprod. 2014 Mar;27(1):1-5. Doi: 10.1007/s00497-013-0237-4. Epub 2013 Nov 8. PMID: 24202960).

Therefore, APH has yet to be exploited in commercial plant breeding because genetically consistent offspring that benefit from APH cannot be produced on a large scale. In the Polyploid Genome Design paradigm of the invention, the formation of clonal unreduced gametes produced by *MiMe* plants is exploited to generate "four-haplotype" plants that contain the full genetic inheritance of their four inbred grandparents.

Compared to the state of the art, the *MiMe* system and, specifically, its application in Polyploid Genome Design, offers several advantages. The *MiMe* system in the Solanaceae (nightshades), such as tomato, enables the controlled production of tetraploid plants that contain the complete genetic repertoire of their four inbred grandparents, thus maximizing genetic heterozygosity and potential heterotic effects. This represents the first step towards controlled polyploid genome design and has the potential to maximize genetic heterozygosity and thereby allow autopolyploid progressive heterosis to be actually exploited in agriculture. The implementation of polyploid genome design in plant breeding will require the development of four-way heterotic groups which could be driven by using genomic selection to identify higher- order combining abilities between grandparental lines.

Beyond this, the genetic space for introgression of wild alleles is essentially doubled in tetraploids, meaning that breeders could incorporate more unique characteristics (e.g., abiotic and biotic stress tolerance) in elite lines that were previously abandoned due to polygenic inheritance or prohibitive linkage drag. The inventors found that thousands of genes derived from wild relatives of tomato were gained in the novel tetraploid plants generated. This blueprint can facilitate the introgression of a complete "wild" genome in a background with three "domesticated" genomes. Further still, four genomes from four distinct species, such as tomato species, could be combined within a single plant. Given that some important pathways for plant defense involve a plethora of genes, this can lead to the development of plant genotypes that combine several novel phenotypes (e.g., plants that have high resistance to abiotic and biotic stresses, and are capable of high yield). Furthermore, polyploidy can allow the more acute control of genes that are very sensitive to gene dosage due to the buffering epistatic effect of a genome containing four complete haplotypes. From a wider perspective, polyploid genome design can allow the controlled engineering of organisms with three or more sets of chromosomes with pre-defined DNA sequences. The approach of the present invention can also be useful for the clonal transfer of genomes between diploid wild materials that are related to current autopolyploid crops, for instance in potato. Maximizing genetic diversity whist increasing genome ploidy through polyploid genome design has the potential to enable heterosis in crops and lead to novel breeding schemes.

The present invention will be described with respect to particular embodiments and with reference to certain drawings, but the disclosure is not limited thereto, but only by the claims. Any reference signs in the claims shall not be construed as limiting the scope. Of course, it is to be understood that not necessarily all aspects or advantages may be achieved in accordance with any particular embodiment. Those skilled in the art will recognize that the disclosed embodiments may be embodied or carried out in a manner that achieves or optimizes one advantage or group of advantages as taught herein without necessarily achieving other aspects or advantages as may be taught or suggested herein. The disclosure may best be understood by reference to the following detailed description when read in conjunction with the accompanying drawings.

The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one disclosed embodiment. Thus, appearances of the phrases "in one embodiment" or "in an embodiment" in various places throughout this specification are not necessarily all referring to the same embodiment, but may do so. Similarly, it should be appreciated that in the description of exemplary disclosed embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. It should be appreciated that "embodiments" of the disclosure can be specifically combined together unless the context indicates otherwise. The specific combinations of all disclosed embodiments (unless implied otherwise by the context) are further disclosed embodiments of the claimed invention.

In addition, as used in this specification and the appended claims, the singular forms "a", "an", and "the" include plural referents unless the content clearly dictates otherwise. Thus, for example, reference to "a polynucleotide" includes two or more polynucleotides, reference to "a polypeptide" includes two or more polypeptides, and the like. "About" as used herein when referring to a measurable value such as an amount is meant to encompass variations of ± 20 % or ± 10 %, more preferably ± 5 %, even more preferably ± 1 %, and still more preferably ± 0.1 % from the specified value, as such variations are appropriate.

Meanwhile, the terms *"comprises", "comprising", "includes", "including", "having",* and their conjugates mean "including but not limited to". The term *"consisting of"* means *"including and limited to".* The term *"consisting essentially of"* means that the composition, method or structure may include additional ingredients, steps and/or parts, but only if the additional ingredients, steps and/or parts do not materially alter the basic and novel characteristics of the claimed composition, method or structure.

In one aspect, the invention provides a method of generating a parent plant capable of apomeiosis, so as to produce viable clonal male and female gametes having the same genome ploidy level as the parent plant, the method comprising: reducing the level or activity of an endogenous REC8 polypeptide in the parent plant or a cell thereof, so as to ablate sister chromatid cohesion; reducing the level or activity of an endogenous TAM polypeptide in the parent plant or a cell thereof, so as to prevent entry to the second meiotic division; and reducing the level or activity of an endogenous SPO11-1, SPO11-2, MTOPVIB, PRD1, PRD2, PRD3, or DFO polypeptide in the parent plant or a cell thereof, so as to ablate meiotic recombination; wherein the parent plant is from the Solanaceae (nightshade) family.

In the context of the present invention, "apomeiosis" is a process that leads to viable unreduced and non-recombined clonal gametes, so the gametes retain a full parental chromosome set, without any reduction in genetic material or loss of any heterozygosity. This is in contrast to meiosis, where meiotic recombination, random segregation of chromosomes and ploidy reduction occurs. Viable unreduced and non-recombined clonal gametes can be used in synthetic "apomixis". "Apomixis" is asexual reproduction leading to the production of seeds without fertilization, that is to offspring genetically identical to the parent plant (Koltunow, et al., (1995) Plant Physiol. 108:1345-1352; Koltunow and Grossniklaus (2003) Ann. Rev. Plant Devl. 54: 547-74; Ravi, et al., (2008) Nature 451:1121-4).

Apomixis increases the opportunity for developing superior gene combinations and facilitates the rapid incorporation of desirable traits. Apomixis not only provides reproductive assurance, but also avoids a loss of heterozygosity in the offspring because the offspring maintains the parental genotype. Apomixis therefore avoids the effects of loss of vigour due to inbreeding and may additionally confer some advantages because of the heterosis effects. "Apomeiosis" also has utility in polyploid genome design. For instance, two parental hybrid plant lines capable of apomeiosis can be crossed so as to generate four-haploid plants. In contrast to sexual breeding schemes that would lead to, on average, 25% of grandparental DNA, four-haplotype plants contain the complete (100%) genetic repertoire of all four of their inbred grandparents. Hence, the method has utility in exploiting genetic variation and autopolyploid progressive heterosis in the breeding of current and new polyploid crops.

As used herein, "endogenous" means native to the genome of the parent plant or cell thereof, and at the native position within the genome.

In the context of the present invention, any plant from the "Solanaceae" (nightshade) family of plants may be used, including, but not limited to, tomato, potato, eggplant, and pepper.

In some embodiments of the method of the invention, the parent plant is a tomato plant. In some embodiments, the endogenous REC8 polypeptide: has at least 90%, 91%, 92%, 93%, 94%, 95%, 96%, 97%, 98%, 99%, or complete sequence identity to the polypeptide of SEQ ID NO.: 6, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID Nos.: 4 and 5, or a fragment thereof. In some embodiments, the endogenous TAM polypeptide: has at least 90%, 91%, 92%, 93%, 94%, 95%, 96%, 97%, 98%, 99%, or complete sequence identity to the polypeptide of SEQ ID NO.: 9, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID Nos.: 7 and 8, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID Nos.: 3, 12, 15, 18, 21, 24, and 27, or a fragment thereof. In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID Nos.: 1, 2, 10, 11, 13, 14, 16, 17, 19, 20, 22, 23, 25, and 26, or a fragment thereof.

In some embodiments of the method of the invention, the tomato plant comprises a genome selected from the group consisting of: *Solanum lycopersicum, Solanum pimpinellifolium, Solanum galapagense, Solanum cheesmaniae, Solanum arcanum, Solanum chmielewskii, Solanum neorickii, Solanum chilense, Solanum corneliomulleri, Solanum habrochaites, Solanum huaylasense, Solanum peruvianum,* and *Solanum pennellii.* In some embodiments, the tomato plant comprises a genome selected from any other species in the *Solanum* genus that can be hybridized with any of the species listed above (such as *Solanum lycopersicoides*). In some embodiments, the tomato plant is a hybrid of any of the above, in the first F1 hybrid generation or any generation thereafter.

In some embodiments of the method of the invention, the parent plant is a potato plant. In some embodiments, the endogenous REC8 polypeptide: has at least 90%, 91%, 92%, 93%, 94%, 95%, 96%, 97%, 98%, 99%, or complete sequence identity to the polypeptide of SEQ ID NO.: 33, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 31 and 32, or a fragment thereof. In some embodiments, the endogenous TAM polypeptide has at least 90%, 91%, 92%, 93%, 94%, 95%, 96%, 97%, 98%, 99%, or complete sequence identity to the polypeptide of SEQ ID NO.: 36, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 34 and 35, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 30, 39, 42, 45, 48, 51, and 54, or a fragment thereof. In some embodiments, the endogenous REC8 polypeptide SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 28, 29, 37, 38, 40, 41, 43, 44, 46, 47, 49, 50, 52, and 53, or a fragment thereof.

In some embodiments of the method of the invention, the potato plant comprises a genome selected from the group consisting of: *Solanum tuberosum, Solanum tuberosum* group stenotomum, *Solanum tuberosum* group phureja, *Solanum tuberosum* group goniocalyx, *Solanum tuberosum* group ajanhuiri, *Solanum candolleanum, Solanum pinnatisectum, Solanum andreanum, Solanum burkartii, Solanum lignicaule, Solanum buesii, Solanum multiinterruptum, Solanum brevicaule, Solanum jamesii, Solanum piurae, Solanum morelliforme, Solanum chomatophilum, Solanum paucissectum, Solanum sogarandinum, Solanum vernei, Solanum chacoense, Solanum commersonii, Solanum boliviense, Solanum bulbocastanum, Solanum cajamarquense, Solanum neorossii, Solanum gourlayi, Solanum spegazzinii, Solanum avilesii, Solanum berthaultii, Solanum venturii, Solanum stoloniferum, Solanum hougasii,* and *Solanum demissum.* In some embodiments, the potato plant comprises a genome of any other species in the *Solanum* genus that can be hybridized with any of the species listed above (such as *Solanum etuberosum*). In some embodiments, the potato plant is a hybrid of any of the above, in the first F1 hybrid generation or any generation thereafter.

In some embodiments of the method of the invention, the parent plant is an eggplant plant. In some embodiments, the endogenous REC8 polynucleotide:
has at least 90%, 91%, 92%, 93%, 94%, 95%, 96%, 97%, 98%, 99%, or complete sequence identity to the polypeptide of SEQ ID NO.: 60, or a fragment thereof. In some embodiments, the endogenous REC8 polynucleotide: is encoded by a polynucleotide having at least 90%, 91%, 92%, 93%, 94%, 95%, 96%, 97%, 98%, 99%, or complete sequence identity to a polynucleotide selected from SEQ ID NOs.: 58 and 59, or a fragment thereof. In some embodiments, the endogenous TAM polypeptide: has at least 90%, 91%, 92%, 93%, 94%, 95%, 96%, 97%, 98%, 99%, or complete sequence identity to a polypeptide of SEQ ID NO.: 63, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 61 and 62, or a fragment thereof. In some embodiments, the endogenous SPO11 -1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 57, 66, 69, 72, 75, 78, and 81, or a fragment thereof. In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 55, 56, 64, 65, 67, 68, 70, 71, 73, 74, 76, 77, 79, and 80, or a fragment thereof.

In some embodiments of the method of the invention, the eggplant plant comprises a genome selected from the group consisting of: *Solanum melongena, Solanum macrocarpon, Solanum aethiopicum, Solanum aculeatissimum, Solanum anguivi, Solanum atropurpureum, Solanum campylacanthum, Solanum capense, Solanum capsicoides, Solanum dasyphylium, Solanum elaeagnifolium, Solanum ferox, Solanum incanum, Solanum indicum, Solanum insanum, Solanum lasiocarpum, Solanum linnaeanum, Solanum pectinatum, Solanum pseudocapsicum, Solanum quitonese, Solanum repandum, Solanum rostratum, Solanum supinum, Solanum sessiliflorum, Solanum sisymbriifolium, Solanum stramoniifolium, Solanum torvum, Solanum viarum, Solanum violaceum, Solanum virginianum,* and *Solanum xanthocarpum.* In some embodiments, the eggplant plant comprises a genome of any other species in the *Solanum* genus that can be hybridized with any of the species listed above. In some embodiments, the eggplant plant is a hybrid of any of the above, in the first F1 hybrid generation or any generation thereafter.

In some embodiments of the method of the invention, the parent plant is a pepper plant. In some embodiments, the endogenous REC8 polypeptide: has at least 90%, 91%, 92%, 93%, 94%, 95%, 96%, 97%, 98%, 99%, or complete sequence identity to a polypeptide of SEQ ID NO.: 87, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 85 and 86, or a fragment thereof. In some embodiments, the endogenous TAM polypeptide: has at least 90%, 91%, 92%, 93%, 94%, 95%, 96%, 97%, 98%, 99%, or complete sequence identity to a polypeptide of SEQ ID NO.: 90, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 88 and 89, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 84, 93, 96, 99, 102, 105, and 108, or a fragment thereof. In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 82, 83, 91, 92, 94, 95, 97, 98, 100, 101, 103, 104, 106, and 107, or a fragment thereof.

In some embodiments of the method of the invention, the pepper plant comprises a genome selected from the group consisting of: *Capsicum annuum, Capsicum baccatum, Capsicum chinense, Capsicum frutescens, Capsicum pubescens, Capsicum buforum, Capsicum campylopodium, Capsicum cardenasii, Capsicum chacoense, Capsicum coccineum, Capsicum cornutum, Capsicum dimorphum, Capsicum dusenii, Capsicum eximium, Capsicum flexuosum, Capsicum galapagoense, Capsicum geminifolium, Capsicum hookerianum, Capsicum lanceolatum, Capsicum leptopodum, Capsicum lycianthoides, Capsicum minutiflorum, Capsicum mirabile, Capsicum mositicum, Capsicum parvifolium, Capsicum rhomboideum, Capsicum schottianum, Capsicum scolnikianum, Capsicum tovarii,* and *Capsicum villosum.* In some embodiments, the pepper plant comprises a genome of any other species in the *Capsicum* genus that can be hybridized with any of the species listed above. In some embodiments, the pepper plant is a hybrid of any of the above, in the first F1 hybrid generation or any generation thereafter.

As used herein, the terms "polynucleotide", "nucleic acid", and variations thereof shall be generic to polydeoxyribonucleotides (containing 2-deoxy-D-ribose), to polyribonucleotides (containing D-ribose), to any other type of polynucleotide that is an N-glycoside of a purine or pyrimidine base, and to other polymers containing nonnucleotidic backbones, provided that the polymers contain nucleobases in a configuration that allows for base pairing and base stacking, as found in DNA and RNA. Thus, these terms include known types of nucleic acid sequence modifications, for example, substitution of one or more of the naturally occurring nucleotides with an analog, and inter-nucleotide modifications. As used herein, the symbols for nucleotides and polynucleotides are those recommended by the IUPAC-IUB Commission of Biochemical Nomenclature.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

Sequences of the polynucleotides of the present invention may be prepared by various suitable methods known in the art, including, for example, direct chemical synthesis or cloning. For direct chemical synthesis, formation of a polymer of nucleic acids typically involves sequential addition of 3'-blocked and 5'-blocked nucleotide monomers to the terminal 5'-hydroxyl group of a growing nucleotide chain, wherein each addition is effected by nucleophilic attack of the terminal 5'-hydroxyl group of the growing chain on the 3'-position of the added monomer, which is typically a phosphorus derivative, such as a phosphotriester or phosphoramidite. Such methodology is known to those of ordinary skill in the art and is described in the pertinent texts and literature (e.g., in WO 2018/175635; PCT/US2018/023630; U.S. Pat. Nos. 4,500,707; 5,436,327; and 5,700,637). In addition, the desired sequences may be isolated from natural sources by splitting DNA using appropriate restriction enzymes, separating the fragments using gel electrophoresis, and thereafter, recovering the desired polynucleotide sequence from the gel via techniques known to those of ordinary skill in the art, such as utilization of polymerase chain reactions (PCR; see e.g. U.S. Pat. No. 4,683,195).

The nucleic acids employed herein may be codon optimized relative to a parental template for expression in a particular host cell. Cells differ in their usage of particular codons, and codon bias corresponds to relative abundance of particular tRNAs in a given cell type. By altering codons in a sequence so that they are tailored to match with the relative abundance of corresponding tRNAs, it is possible to increase expression of a product (e.g. a polypeptide) from a nucleic acid. Similarly, it is possible to decrease expression by deliberately choosing codons corresponding to rare tRNAs. Thus, codon optimization/deoptimization can provide control over nucleic acid expression in a particular cell type (e.g. bacterial cell, plant cell, mammalian cell, etc.). Methods of codon optimizing a nucleic acid for tailored expression in a particular cell type are well-known to those of skill in the art.

Various methods are known to those of skill in the art for identifying similar (e.g. homologs, orthologs, paralogs, etc.) polypeptide and/or polynucleotide sequences, including phylogenetic methods, sequence similarity analysis, and hybridization methods. Phylogenetic trees may be created for a gene family by using a program such as CLUSTAL (Thompson et al. Nucleic Acids Res. 22: 4673-4680 (1994); Higgins et al. Methods Enzymol 266: 383-402 (1996)) or MEGA (Tamura et al. Mol. Biol. & Evo. 24:1596-1599 (2007)). Once an initial tree for genes from one species is created, potential orthologous sequences can be placed in the phylogenetic tree and their relationships to genes from the species of interest can be determined. Evolutionary relationships may also be inferred using the Neighbor-Joining method (Saitou and Nei, Mol. Biol. & Evo. 4:406-425 (1987)). Homologous sequences may also be identified by a reciprocal BLAST strategy. Evolutionary distances may be computed using the Poisson correction method (Zuckerkandl and Pauling, pp. 97-166 in Evolving Genes and Proteins, edited by V. Bryson and H.J. Vogel. Academic Press, New York (1965)).

In addition, evolutionary information may be used to predict gene function. Functional predictions of genes can be greatly improved by focusing on how genes became similar in sequence *(i.e.* by evolutionary processes) rather than on the sequence similarity itself (Eisen, Genome Res. 8: 163-167 (1998)). Many specific examples exist in which gene function has been shown to correlate well with gene phylogeny (Eisen, Genome Res. 8: 163-167 (1998)). By using a phylogenetic analysis, one skilled in the art would recognize that the ability to deduce similar functions conferred by closely-related polypeptides is predictable.

When a group of related sequences are analysed using a phylogenetic program such as CLUSTAL, closely related sequences typically cluster together or in the same clade (a group of similar genes). Groups of similar genes can also be identified with pair-wise BLAST analysis (Feng and Doolittle, J. Mol. Evol. 25: 351-360 (1987)). Analysis of groups of similar genes with similar function that fall within one clade can yield sub-sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount, Bioinformatics: Sequence and Genome Analysis Cold Spring Harbor Laboratory Press, Cold Spring Harbor, N.Y., page 543 (2001)).

To find sequences that are homologous to a reference sequence, BLAST [0155] nucleotide searches can be performed with the BLASTN program, score=100, wordlength=12, to obtain nucleotide sequences homologous to a nucleotide sequence encoding a polypeptide of the invention. BLAST protein searches can be performed with the BLASTX program, score=50, wordlength=3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) *supra.* When utilizing BLAST, Gapped BLAST, or PSI-BLAST, the default parameters of the respective programs (e.g., BLASTN for nucleotide sequences, BLASTX for proteins) can be used Methods for the alignment of sequences and for the analysis of similarity and identity of polypeptide and polynucleotide sequences are well-known in the art.

As used herein, "sequence identity" refers to the percentage of residues that are identical in the same positions in the sequences being analysed. As used herein "sequence similarity" refers to the percentage of residues that have similar biophysical / biochemical characteristics in the same positions (e.g. charge, size, hydrophobicity) in the sequences being analysed.

Methods of alignment of sequences for comparison are well-known in the art, including manual alignment and computer assisted sequence alignment and analysis. This latter approach is a preferred approach, due to the increased throughput afforded by computer assisted methods. As noted below, a variety of computer programs for performing sequence alignment are available, or can be produced by one of skill in the art.

The determination of percent sequence identity and/or similarity between any two sequences can be accomplished using a mathematical algorithm. Examples of such mathematical algorithms are the algorithm of Myers and Miller, CABIOS 4:11-17 (1988); the local homology algorithm of Smith et al., Adv. Appl. Math. 2:482 (1981); the homology alignment algorithm of Needleman and Wunsch, J. Mol. Biol. 48:443-453 (1970); the search-for-similarity-method of Pearson and Lipman, Proc. Natl. Acad. Sci. 85:2444-2448 (1988); the algorithm of Karlin and Altschul, Proc. Natl. Acad. Sci. USA 87:2264-2268 (1990), modified as in Karlin and Altschul, Proc. Natl. Acad. Sci. USA 90:5873-5877 (1993).

Computer implementations of these mathematical algorithms can be utilized for comparison of sequences to determine sequence identity and/or similarity. Such implementations include, for example: CLUSTAL in the PC/Gene program (available from Intelligenetics, Mountain View, Calif); the AlignX program, versionl0.3.0 (Invitrogen, Carlsbad, CA) and GAP, BESTFIT, BLAST, PASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described by Higgins et al. Gene 73:237-244 (1988); Higgins et al. CABIOS 5:151-153 (1989); Corpet et al. Nucleic Acids Res. 16:10881-90 (1988); Huang et al. CABIOS 8:155-65 (1992); and Pearson et al., Meth. Mol. Biol. 24:307-331 (1994). The BLAST programs of Altschul et al. J. Mol. Biol. 215:403-410 (1990) are based on the algorithm of Karlin and Altschul (1990) supra.

Polynucleotides homologous to a reference sequence can be identified by hybridization to each other under stringent or under highly stringent conditions. Single stranded polynucleotides hybridize when they associate based on a variety of well characterized physical-chemical forces, such as hydrogen bonding, solvent exclusion and base stacking. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in references cited below (e.g., Sambrook et al., Molecular Cloning: A Laboratory Manual, 2nd Ed., Vol. 1-3, Cold Spring Harbor Laboratory, Cold Spring Harbor, N.Y. ("Sambrook") (1989); Berger and Kimmel, Guide to Molecular Cloning Techniques, Methods in Enzymology, vol. 152 Academic Press, Inc., San Diego, Calif ("Berger and Kimmel") (1987); and Anderson and Young, "Quantitative Filter Hybridisation." In: Harnes and Higgins, ed., Nucleic Acid Hybridisation, A Practical Approach. Oxford, TRL Press, 73-111 (1985)).

The invention also employs polynucleotide sequences that are capable of hybridizing to the disclosed polynucleotide sequences and fragments thereof under various conditions of stringency (see, for example, Wahl and Berger, Methods Enzymol. 152: 399-407 (1987); and Kimmel, Methods Enzymo. 152: 507-511, (1987)). Full length cDNA, homologs, orthologs, and paralogs of polynucleotides of the present invention may be identified and isolated using well-known polynucleotide hybridization methods.

With regard to hybridization, conditions that are highly stringent, and means for achieving them, are well known in the art. See, for example, Sambrook et al. (1989) *supra*; Berger and Kimmel (1987) pp. 467-469 *supra*; and Anderson and Young (1985) *supra.* Hybridization experiments are generally conducted in a buffer of pH between 6.8 to 7.4, although the rate of hybridization is nearly independent of pH at ionic strengths likely to be used in the hybridization buffer (Anderson and Young (1985) *supra*)*.* In addition, one or more of the following may be used to reduce non-specific hybridization: sonicated salmon sperm DNA or another non-complementary DNA, bovine serum albumin, sodium pyrophosphate, sodium dodecylsulfate (SDS), polyvinyl-pyrrolidone, ficoll and Denhardt's solution. Dextran sulfate and polyethylene glycol 6000 act to exclude DNA from solution, thus raising the effective probe DNA concentration and the hybridization signal within a given unit of time. In some instances, conditions of even greater stringency may be desirable or required to reduce non-specific and/or background hybridization. These conditions may be created with the use of higher temperature, lower ionic strength and higher concentration of a denaturing agent such as formamide.

Stringency conditions can be adjusted to screen for moderately similar fragments such as homologous sequences from distantly related organisms, or to highly similar fragments such as genes that duplicate functional enzymes from closely related organisms. The stringency can be adjusted either during the hybridization step or in the post-hybridization washes. Salt concentration, formamide concentration, hybridization temperature and probe lengths are variables that can be used to alter stringency. As a general guideline, high stringency is typically performed at Tm 50 degrees Celsius to Tm 20 degrees Celsius, moderate stringency at Tm 20 degrees Celsius to Tm 35 degrees Celsius and low stringency at Tm 35 degrees Celsius to Tm 50 degrees Celsius for duplex >150 base pairs. Hybridization may be performed at low to moderate stringency (25-50°C below Tm), followed by post-hybridization washes at increasing stringencies. Maximum rates of hybridization in solution are determined empirically to occur at Tm-25 degrees Celsius for DNA-DNA duplex and Tm-15 degrees Celsius for RNA-DNA duplex. Optionally, the degree of dissociation may be assessed after each wash step to determine the need for subsequent, higher stringency wash steps.

High stringency conditions may be used to select for nucleic acid sequences with high degrees of identity to the disclosed sequences. An example of stringent hybridization conditions obtained in a filter-based method such as a Southern or northern blot for hybridization of complementary nucleic acids that have more than 100 complementary residues is about 5°C to 20°C lower than the thermal melting point (Tm) for the specific sequence at a defined ionic strength and pH.

Hybridization and wash conditions that may be used to bind and remove polynucleotides with less than the desired homology to the nucleic acid sequences or their complements include, for example: 6X SSC and 1% SDS at 65°C; 50% formamide, 4X SSC at 42°C; 0.5X SSC to 2.0 X SSC, 0.1% SDS at 50°C to 65°C; or 0.1X SSC to 2X SSC, 0.1% SDS at 50°C - 65°C; with a first wash step of, for example, 10 minutes at about 42°C with about 20% (v/v) formamide in 0. IX SSC, and with, for example, a subsequent wash step with 0.2 X SSC and 0.1% SDS at 65°C for 10, 20 or 30 minutes. For identification of less closely related homologs, wash steps may be performed at a lower temperature, e.g., 50° C. An example of a low stringency wash step employs a solution and conditions of at least 25°C in 30 mM NaCl, 3 mM trisodium citrate, and 0.1% SDS over 30 min. Greater stringency may be obtained at 42°C in 15 mM NaCl, with 1.5 mM trisodium citrate, and 0.1% SDS over 30 min. Wash procedures will generally employ at least two final wash steps. Additional variations on these conditions will be readily apparent to those skilled in the art (see, for example, US Patent Application No. 20010010913).

If desired, one may employ wash steps of even greater stringency, including conditions of 65°C-68°C in a solution of 15 mM NaCl, 1.5 mM trisodium citrate, and 0.1% SDS, or about 0.2X SSC, 0.1% SDS at 65° C and washing twice, each wash step of 10, 20 or 30 min in duration, or about 0.1 X SSC, 0.1% SDS at 65° C and washing twice for 10, 20 or 30 min. Hybridization stringency may be increased further by using the same conditions as in the hybridization steps, with the wash temperature raised about 3°C to about 5°C, and stringency may be increased even further by using the same conditions except the wash temperature is raised about 6°C to about 9°C.

In the context of the present invention, the amount of "sequence identity" when used in connection with a polynucleotide sequence includes total lengths and/or regions having unit integral values in the ranges of about 1-20 base pairs (bp), 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kilobases (kb), 1.5-3 kb, 2-4 kb, 2.5-5 kb, 3-6 kb, 3.5-7 kb, 4-8 kb, 5-10 kb, or up to and including the total length of the endogenous polynucleotide sequence. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bp. Genomic endogenous polynucleotide sequences provided herein include 1 kb upstream and downstream of genomic DNA with respect to the coding sequence. In some embodiments, a genome modification is introduced within 1 kb upstream and/or downstream of any one of the coding sequences provided herein. All of the genomic DNA sequences provided herein include 1kb upstream and 1kb downstream with respect to the coding sequence. Although not explicitly included in the Sequence Listing, genomic DNA sequences that exclude 1 kb upstream and/or 1kb downstream of genomic DNA with respect to the coding sequence are also provided herein and may find use in any of the embodiments of the invention. In some embodiments, a genome modification is introduced within any one of the coding sequences provided herein.

In some embodiments of the invention, the polynucleotide encodes a polypeptide or fragment thereof that has an amino acid sequence that is at least 90%, at least 91%, at least 92%, at least 93%, at least 94%, at least 95%, at least 96%, at least 97%, at least 98%, at least 99%, or 100% identical to any one of the polypeptide sequences of the invention. "Fragment" as used herein refers to a portion of the nucleic acid sequence encoding a polypeptide. A fragment of a nucleic acid sequence may encode a biologically active portion of a polypeptide or it may be a fragment that can be used as a hybridization probe or PCR primer using methods disclosed below. Nucleic acid molecules that are fragments of a nucleic acid sequence encoding a polypeptide may comprise at least 150, 180, 210, 240, 270, 300, 330 or 360, contiguous nucleotides or up to the number of nucleotides present in a full-length nucleic acid sequence encoding a polypeptide disclosed herein, depending upon the intended use. "Contiguous nucleotides" is used herein to refer to nucleotide residues that are immediately adjacent to one another.

In some embodiments of the invention, a fragment of a polynucleotide sequence encoding a polypeptide of the invention will encode at least about 15, 20, 30, 50, 75, 100, 125, contiguous amino acids or up to the total number of amino acids present in a full-length polypeptide of the invention. In some embodiments, the fragment is an N-terminal and/or a C-terminal truncation of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more amino acids from the N-terminus and/ or C-terminus relative to any of the polypeptides of the invention, e.g., by proteolysis, insertion of a start codon, deletion of the codons encoding the deleted amino acids with the concomitant insertion of a stop codon or by insertion of a stop codon in the coding sequence. In some embodiments, the fragments encompassed herein result from the removal of the N-terminal 1, 2, 3, 4, 5, 6, 7, 8, 9, 10, 11, 12, 13, 14, 15, 16, 17, 18, 19, 20, 21, 22, 23, 24, 25 or more amino acids from the N-terminus relative to any of the polypeptides of the invention, e.g., by proteolysis or by insertion of a start codon in the coding sequence.

The amount of "sequence identity" can also be described by percent sequence identity over the full aligned length of two polynucleotides, which includes percent sequence identity of at least 90%, 91%, 92%, 93%, 94%, 95%, 96%, 97%, 98%, 99%, or 100%. In some embodiments, the "sequence identity" is calculated using ClustalW algorithm in the ALIGNX module of the Vector NTI Program Suite (Invitrogen Corporation, Carlsbad, Calif.) with all default parameters. In some embodiments, the sequence identity is across the entire length of polypeptide calculated using ClustalW algorithm in the ALIGNX module of the Vector NTI Program Suite (Invitrogen Corporation, Carlsbad, Calif.) with all default parameters. Sufficient homology includes any combination of polynucleotide length, global percent sequence identity, and optionally conserved regions of contiguous nucleotides or local percent sequence identity. For example, sufficient homology can be described as a region of 75-150 bp having at least 90% sequence identity to a region of an endogenous polynucleotide sequence. Sufficient homology can also be described by the predicted ability of two genes or polynucleotides to specifically hybridize under high stringency conditions. In this regard, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology - Hybridization with Nucleic Acid Probes, (Elsevier, New York).

As used herein, a "polypeptide" is an amino acid sequence including a plurality of consecutive polymerized amino acid residues (e.g., at least about 15 consecutive polymerized amino acid residues). "Polypeptide" refers to an amino acid sequence, oligopeptide, peptide, protein, or portions thereof, and the terms "polypeptide" and "protein" are used interchangeably. Polypeptides as described herein also include polypeptides having various amino acid additions, deletions, or substitutions relative to the native amino acid sequence of a polypeptide of the present disclosure. In some embodiments of the invention, polypeptides that are homologs of a polypeptide of the present disclosure contain non-conservative changes of certain amino acids relative to the native sequence of a polypeptide of the present disclosure. In some embodiments of the invention, polypeptides that are homologs of a polypeptide of the invention contain conservative changes of certain amino acids relative to the native sequence of a polypeptide of the invention, and thus may be referred to as conservatively modified variants. A conservatively modified variant may include individual substitutions, deletions or additions to a polypeptide sequence which result in the substitution of an amino acid with a chemically similar amino acid. Conservative substitution tables providing functionally similar amino acids are well-known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). A modification of an amino acid to produce a chemically similar amino acid may be referred to as an analogous amino acid.

In the context of the present invention, the amount of "sequence identity" when used in connection with a polypeptide sequence may expressed as a percentage in relation to the full-length polypeptides of the invention. In some embodiments, the polypeptide has an amino acid sequence with at least 90%, at least 91%, at least 92%, at least 93%, at least 94%, at least 95%, at least 96%, at least 97%, at least 98%, at least 99%, or 100% amino acid identity to any one of the full-length polypeptide sequences of the invention.

"Sequence identity", as used herein in connection with polypeptides, may also describe fragments of the full-length polypeptides of the invention. For instance, a fragment of any one of the full-length polypeptides of the invention may have 20 consecutive amino acids, at least 30 consecutive amino acids, at least 40 consecutive amino acids, at least 50 consecutive amino acids, at least 60 consecutive amino acids, at least 70 consecutive amino acids, at least 80 consecutive amino acids, at least 90 consecutive amino acids, at least 100 consecutive amino acids, at least 120 consecutive amino acids, at least 140 consecutive amino acids, at least 160 consecutive amino acids, at least 180 consecutive amino acids, at least 200 consecutive amino acids, at least 220 consecutive amino acids, at least 240 consecutive amino acids, or more consecutive amino acids of a full-length polypeptide of the invention.

In some embodiments, polypeptides or fragments thereof include sequences with one or more amino acids removed from the consecutive amino acid sequence of a full-length polypeptide of the invention. In some embodiments, polypeptides or fragments thereof include sequences with one or more amino acids replaced/substituted with an amino acid different from the endogenous amino acid present at a given amino acid position in a consecutive amino acid sequence of a full-length polypeptide of the invention. In some embodiments, polypeptides or fragments thereof include sequences with one or more amino acids added to an otherwise consecutive amino acid sequence of a full-length polypeptide of the invention.

In any of the embodiments of the method of the invention, the parent plant or cell thereof may be haploid, diploid or polyploid. If the parent plant is polyploid, it may be triploid, tetraploid, or hexaploid.

In any of the embodiments of the method of the invention, the parent plant or cell thereof may be a hybrid plant or cell thereof.

In any of the embodiments of the method of the invention, the method may comprise reducing the level of the endogenous REC8, TAM, and/or SPO11-1, SPO11-2, MTOPVIB, PRD1, PRD2, PRD3, or DFO polypeptide/s in the parent plant or a cell thereof by about 10%, 20%, 30%, 40%, 50%, 60%, 70%, 80%, 90%, or completely, as compared to in a control plant or cell thereof. In some embodiments, the control plant or cell thereof is a wild type equivalent of the parent plant or cell thereof.

In any of the embodiments of the method of the invention, the method may comprise reducing the activity of the endogenous REC8, TAM, and/or SPO11 -1, SPO11-2, MTOPVIB, PRD1, PRD2, PRD3, or DFO polypeptide/s in the parent plant or a cell thereof by about 10%, 20%, 30%, 40%, 50%, 60%, 70%, 80%, 90%, or completely, as compared to in a control plant or cell thereof. In some embodiments, the control plant or cell thereof is a wild type equivalent of the parent plant or cell thereof.

As used herein, the term "reducing the level or activity of" one or more endogenous polypeptides means that the phenotype conferred by the one or more endogenous polypeptides in the parent plant or cell thereof or the function of the one or more endogenous polypeptides in the parent plant or cell thereof is reduced as compared to in a "control plant" or cell thereof that is a wild type equivalent of the parent plant or cell thereof. In some embodiments, the wild type equivalent may have the same genetic background and a comparable developmental stage. In some embodiments, the expression level of a gene encoding the one or more endogenous polypeptides in the parent plant or cell thereof is reduced as compared to in a "control plant" or cell thereof that is a wild type equivalent of the parent plant or cell thereof. In some embodiments, the wild type equivalent may have the same genetic background and a comparable developmental stage. Expression level can be mRNA or protein. Such reduction may be by at least 10%, 20%, 30%, 40%, 50%, 60%, 70%, 80%, 90%, or 100%. According to some embodiments, reduction is complete loss of function. In this context, in some embodiments, a "control" plant or plant cell is one in which the activity of the endogenous REC8, TAM, and/or SPO11-1, SPO11-2, MTOPVIB, PRD1, PRD2, PRD3, or DFO polypeptide/s is not reduced. In some embodiments, a "control" plant or plant cell lacks the genome modifications in the polynucleotides encoding the endogenous REC8, TAM, and/or SPO11-1, SPO11-2, MTOPVIB, PRD1, PRD2, PRD3, or DFO polypeptide/s.

"Reducing" the expression level of an endogenous polynucleotide or an endogenous polypeptide encoded thereby means that the polynucleotide or polypeptide level of the target sequence is lower than the polynucleotide level or polypeptide level of the same target sequence in an appropriate control (such as a wild type equivalent). In particular aspects, reducing the polynucleotide level and/or the polypeptide level of the target sequence results in less than 95%, less than 90%, less than 80%, less than 70%, less than 60%, less than 50%, less than 40%, less than 30%, less than 20%, less than 10%, or less than 5% of the polynucleotide level, or the level of the polypeptide encoded thereby, of the same target sequence in an appropriate control (such as a wild type equivalent). In some embodiments, the method of the invention results in a reduction in function of the at least one endogenous polypeptide in the parent plant or cell thereof. In other embodiments, the method results in a loss of function of the at least one polypeptide in the parent plant or cell thereof.

In some embodiments of the method of the invention, the method comprises reducing the level or activity of the endogenous REC8, TAM, and/or SPO11-1, SPO11-2, MTOPVIB, PRD1, PRD2, PRD3, or DFO polypeptide/s in the parent plant or a cell thereof by RNA silencing.

"RNA silencing" makes use of a polynucleotide sequence (or "silencing element") capable of reducing the level or expression of a polynucleotide being targeted or the polypeptide encoded thereby. The silencing element can decrease the expression level of the target sequence by influencing the level of the target RNA transcript or, alternatively, by influencing translation and thereby affecting the level of the encoded polypeptide. A single polynucleotide employed in the methods can comprise one or more silencing elements to the same or different polynucleotides being targeted. The silencing element can be produced *in vivo* or *in vitro.* In some aspects, the silencing element has no effect on the parts of the parent plant that do not constitute the germline.

Silencing elements can include, but are not limited to, a sense suppression element, an antisense suppression element, a double stranded RNA, a siRNA, an amiRNA, a miRNA, or a hairpin suppression element. Non-limiting examples of silencing elements that can be employed to decrease expression of target sequences comprise fragments and variants of the sense or antisense sequence or consist of the sense or antisense sequence of any of the polynucleotide sequences disclosed herein. The silencing element can further comprise additional sequences that advantageously effect transcription and/or the stability of a resulting transcript. For example, the silencing elements can comprise at least one thymine residue at the 3' end. This can aid in stabilization. Thus, the silencing elements can have at least 1, 2, 3, 4, 5, 6, 7, 8, 9, 10 or more thymine residues at the 3' end. Enhancer suppressor elements can also be employed.

In some embodiments of the method of the invention, decreasing expression of any of the polypeptides disclosed herein may be obtained by sense suppression or cosuppression. For cosuppression, an expression cassette is designed to express an RNA molecule corresponding to all or part of a messenger RNA encoding any of the polypeptides disclosed herein in the "sense" orientation. Overexpression of the RNA molecule may result in decreased expression of the endogenous polynucleotide. Plants transformed with the cosuppression expression cassette can be screened to identify those that show the desired degree of inhibition of polypeptide expression.

Typically, a sense suppression element has substantial sequence identity to the endogenous polynucleotide being targeted, typically greater than about 65% sequence identity, greater than about 85% sequence identity, about 90%, 91%, 92%, 93%, 94%, 95%, 96%, 97%, 98% or 99% sequence identity. The sense suppression element can be any length, so long as it allows for the suppression of the targeted sequence. The sense suppression element can be, for example, 15, 16, 17, 18 19, 20, 22, 25, 30, 50, 100, 150, 200, 250, 300, 350, 400, 450, 500, 600, 700, 900, 1000, 1100, 1200, 1300 nucleotides or longer of the endogenous polynucleotides disclosed herein.

The polynucleotide used for cosuppression may correspond to all or part of the sequence encoding the polypeptide, all or part of the 5' and/or 3' untranslated region of a polypeptide transcript or all or part of both the coding sequence and the untranslated regions of a transcript encoding a polypeptide. Where the polynucleotide comprises all or part of the coding region for the polypeptide, the expression cassette can be designed to eliminate the start codon of the polynucleotide so that no protein product will be translated.

Cosuppression may be used to inhibit the expression of plant genes to produce plants having undetectable protein levels for the proteins encoded by these genes. See, for example, Broin, et al., (2002) Plant Cell 14:1417-1432. Cosuppression may also be used to inhibit the expression of multiple proteins in the same plant. See, for example, US Patent Number 5,942,657. Methods for using cosuppression to inhibit the expression of endogenous genes in plants are described in Flavell, et al., (1994) Proc. Natl. Acad. Sci. USA 91:3490-3496; Jorgensen, et al., (1996) Plant Mol. Biol. 31:957-973; Johansen and Carrington, (2001) Plant Physiol. 126:930-938; Broin, et al., (2002) Plant Cell 14:1417-1432; Stoutjesdijk, et al., (2002) Plant Physiol. 129:1723-1731; Yu, et al., (2003) Phytochemistry 63:753-763 and US Patent Numbers 5,034,323, 5,283,184 and 5,942,657. The efficiency of cosuppression may be increased by including a poly-dT region in the expression cassette at a position 3' to the sense sequence and 5' of the polyadenylation signal.

In some embodiments of the method of the invention, inhibition of the expression of the polypeptide may be obtained by antisense suppression. For antisense suppression, the expression cassette is designed to express an RNA molecule complementary to all or part of a messenger RNA encoding the endogenous polypeptide. Overexpression of the antisense RNA molecule may result in decreased expression of the endogenous polynucleotide being targeted. Plants transformed with the antisense suppression expression cassette can be screened to identify those that show the desired degree of inhibition of polypeptide expression.

The polynucleotide for use in antisense suppression may correspond to all or part of the complement of the sequence encoding the endogenous polypeptide, all or part of the complement of the 5' and/or 3' untranslated region of the target transcript, or all or part of the complement of both the coding sequence and the untranslated regions of a transcript encoding the polypeptide. In addition, the antisense polynucleotide may be fully complementary *(i.e.* 100% identical to the complement of the target sequence) or partially complementary *(i.e.* less than 100% identical to the complement of the target sequence) to the target sequence. In addition, the antisense suppression element may be fully complementary or partially complementary to the target polynucleotide. In specific aspects of the disclosure, the antisense suppression element comprises at least 85%, 90%, 91%, 92%, 93%, 94%, 95%, 96%, 97%, 98%, or 99% sequence complementarity to the target polynucleotide.

Antisense suppression may be used to inhibit the expression of multiple proteins in the same plant. Furthermore, the antisense suppression element can be complementary to a portion of any of the endogenous polynucleotides disclosed herein. Methods for using antisense suppression to inhibit the expression of endogenous genes in plants are described, for example, in Liu et at (2002) Plant Physiol. 129:1732-1743 and U.S. Pat. Nos. 5,759,829 and 5,942,657. The efficiency of antisense suppression may be increased by including a poly-dT region in the expression cassette at a position 3' to the antisense sequence and 5' of the polyadenylation signal.

In some embodiments of the method of the invention, inhibition of the expression of an endogenous polypeptide disclosed herein may be obtained by double-stranded RNA (dsRNA) interference. A "double stranded RNA silencing element" or "dsRNA" comprises at least one transcript that is capable of forming a dsRNA. Thus, a "dsRNA silencing element" includes a dsRNA, a transcript or polyribonucleotide capable of forming a dsRNA or more than one transcript or polyribonucleotide capable of forming a dsRNA. "Double stranded RNA" or "dsRNA" refers to a polyribonucleotide structure formed either by a single self-complementary RNA molecule or a polyribonucleotide structure formed by the expression of least two distinct RNA strands. The dsRNA molecule(s) that may be employed mediate the decrease of expression of a target sequence, for example, by mediating RNA interference "RNAi" or gene silencing in a sequence-specific manner. The dsRNA is capable of decreasing or eliminating the level or expression of an endogenous polynucleotide or endogenous polypeptide as disclosed herein.

The dsRNA can decrease or eliminate the expression level of the target sequence by influencing the level of the target RNA transcript, by influencing translation and thereby affecting the level of the encoded polypeptide, or by influencing expression at the pre-transcriptional level *(i.e.* via the modulation of chromatin structure, methylation pattern, etc., to alter gene expression). See, for example, Verdel et al., (2004) Science 303:672-676; Pal-Bhadra et al., (2004) Science 303:669-672; Allshire (2002) Science 297:1818-1819; Volpe et al., (2002) Science 297:1833-1837; Jenuwein (2002) Science 297:2215-2218; and Hall et al., (2002) Science 297:2232-2237. Accordingly, as used herein, the term "dsRNA" is meant to encompass other terms used to describe nucleic acid molecules that are capable of mediating RNA interference or gene silencing, including, for example, shortinterfering RNA (siRNA), double-stranded RNA (dsRNA), micro-RNA (miRNA), hairpin RNA, short hairpin RNA (shRNA), post-transcriptional gene silencing RNA (ptgsRNA), and others.

For dsRNA interference, a sense RNA molecule like that described above for cosuppression and an antisense RNA molecule that is fully or partially complementary to the sense RNA molecule are expressed in the same cell, resulting in inhibition of the expression of the corresponding endogenous messenger RNA.

Expression of the sense and antisense molecules may be accomplished by designing the expression cassette to comprise both a sense sequence and an antisense sequence. Alternatively, separate expression cassettes may be used for the sense and antisense sequences. Methods for using dsRNA interference to inhibit the expression of endogenous plant genes are described in Waterhouse, et al., (1998) Proc. Natl. Acad. Sci. USA 95:13959-13964, Liu, et al., (2002) Plant Physiol. 129:1732-1743 and WO 1999/49029, WO 1999/53050, WO 1999/61631 and WO 2000/49035.

In some embodiments of the method of the invention, the inhibition of the expression of an endogenous polypeptide disclosed herein may be obtained by hairpin RNA (hpRNA) interference or intron-containing hairpin RNA (ihpRNA) interference. These methods are highly efficient at inhibiting the expression of endogenous genes. See, Waterhouse and Helliwell, (2003) Nat. Rev. Genet. 4:29-38.

For hpRNA interference, the expression cassette is designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially complementary to the sense sequence. Alternatively, the base-paired stem region may correspond to a portion of a promoter sequence controlling expression of the gene whose expression is to be inhibited. Thus, the base-paired stem region of the molecule generally determines the specificity of the RNA interference. hpRNA molecules are highly efficient at inhibiting the expression of endogenous genes and the RNA interference they induce is inherited by subsequent generations of plants. See, for example, Chuang and Meyerowitz, (2000) Proc. Natl. Acad. Sci. USA 97:4985-4990; Stoutjesdijk, et al., (2002) Plant Physiol. 129:1723-1731; and Waterhouse and Helliwell, (2003) Nat. Rev. Genet. 4:29-38. Methods for using hpRNA interference to inhibit or silence the expression of genes are described, for example, in Chuang and Meyerowitz, (2000) Proc. Natl. Acad. Sci. USA 97:4985-4990; Stoutjesdijk, et al., (2002) Plant Physiol. 129:1723-1731; Waterhouse and Helliwell, (2003) Nat. Rev. Genet. 4:29-38; Pandolfini et al., BMC Biotechnology 3:7; and US Patent Application Publication Number 2003/0175965. A transient assay for the efficiency of hpRNA constructs to silence gene expression *in vivo* has been described by Panstruga, et al., (2003) Mol. Biol. Rep. 30:135-140.

For ihpRNA, the interfering molecules have the same general structure as for hpRNA, but the RNA molecule additionally comprises an intron that is capable of being spliced in the cell in which the ihpRNA is expressed. The use of an intron minimizes the size of the loop in the hairpin RNA molecule following splicing, and this increases the efficiency of interference. See, for example, Smith, et al., (2000) Nature 407:319-320. In fact, Smith, *et al*., show 100% suppression of endogenous gene expression using ihpRNA-mediated interference. Methods for using ihpRNA interference to inhibit the expression of endogenous plant genes are described, for example, in Smith, et al., (2000) Nature 407:319-320; Wesley, et al., (2001) Plant J. 27:581-590; Wang and Waterhouse, (2001) Curr. Opin. Plant Biol. 5:146-150; Waterhouse and Helliwell, (2003) Nat. Rev. Genet. 4:29-38; Helliwell and Waterhouse, (2003) Methods 30:289-295; and US Patent Application Publication Number 2003/0180945.

Any region of the target polynucleotide can be used to design the domain of the silencing element that shares sufficient sequence identity to allow expression of the hairpin transcript to decrease the level of the target polynucleotide. For instance, the domain can be designed to share sequence identity to the 5' untranslated region of the target polynucleotide(s), the 3' untranslated region of the target polynucleotide(s), exonic regions of the target polynucleotide(s), intronic regions of the target polynucleotide(s), and any combination thereof. In specific aspects of the disclosure, a domain of the silencing element shares sufficient homology to at least about 15, 16, 17, 18, 19, 20, 22, 25 or 30 consecutive nucleotides from about nucleotides 1-50, 25-75, 75-125, 50-100, 125-175, 175-225, 100-150, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, etc., of the target sequence.

In some instances to optimize the siRNA sequences employed in the hairpin, the synthetic oligodeoxyribonucleotide/RNAse H method can be used to determine sites on the target mRNA that are in a conformation that is susceptible to RNA silencing. See, for example, Vickers et al., (2003) J. Biol. Chem. 278:7108-7118 and Yang et al., (2002) Proc. Natl. Acad. Sci. USA 99:9442-9447. These studies indicate that there is a significant correlation between the RNase-H-sensitive sites and sites that promote efficient siRNA-directed mRNA degradation.

The expression cassette for hpRNA interference may also be designed such that the sense sequence and the antisense sequence do not correspond to an endogenous RNA. In such a case, the sense and antisense sequence flank a loop sequence that comprises a nucleotide sequence corresponding to all or part of the endogenous messenger RNA of the target gene. Thus, it is the loop region that determines the specificity of the RNA interference. See, for example, WO 2002/00904; Mette, et al., (2000) EMBO J 19:5194-5201; Matzke, et al., (2001) Curr. Opin. Genet. Devel. 11:221-227; Scheid, et al., (2002) Proc. Natl. Acad. Sci. USA 99:13659-13662; Aufsaftz, et al., (2002) Proc. Natl. Acad. Sci. 99:16499-16506; Sijen, et al., Curr. Biol. (2001) 11:436-440.

In addition, transcriptional gene silencing (TGS) may be accomplished through use of a hairpin suppression element where the inverted repeat of the hairpin shares sequence identity with the promoter region of a target polynucleotide to be silenced. See, for example, Aufsatz et al., (2002) Proc. Natl. Acad. Sci. 99:16499-16506 and Mette et al., (2000) EMBO J. 19:5194-5201.

Amplicon expression cassettes comprise a plant virus-derived sequence that contains all or part of the target gene but generally not all of the genes of the native virus. The viral sequences present in the transcription product of the expression cassette allow the transcription product to direct its own replication. The transcripts produced by the amplicon may be either sense or antisense relative to the target sequence *(i.e.* the messenger RNA for the polypeptide). Methods of using amplicons to inhibit the expression of endogenous plant genes are described, for example, in Angell and Baulcombe, (1997) EMBO J. 16:3675-3684; Angell and Baulcombe, (1999) Plant J. 20:357-362; and US Patent Number 6,646,805.

In some embodiments of the method of the invention, inhibition of the expression of an endogenous polypeptide as disclosed herein may be obtained by RNA interference by expression of a gene encoding a micro RNA (miRNA) or shortinterfering RNA (siRNA) (Meister and Tuschl (2004) Nature 431:343-349 and Bonetta et al., (2004) Nature Methods 1:79-86). miRNAs are regulatory agents consisting of about 22 ribonucleotides. miRNA are highly efficient at inhibiting the expression of endogenous genes. See, for example Palatnik et al., (2003) Nature 425:257-263. The miRNA can be an "artificial miRNA" or "amiRNA" which comprises a miRNA sequence that is synthetically designed to silence a target sequence.

For miRNA interference, the expression cassette is designed to express an RNA molecule that is modelled on an endogenous miRNA gene. For example, the miRNA gene encodes an RNA that forms a hairpin structure containing a 22-nucleotide sequence that is complementary to another endogenous gene (target sequence). In some aspects of the disclosure, the 22-nucleotide sequence is selected from a transcript sequence from any of the endogenous polynucleotides disclosed herein and contains 22 nucleotides of the same in sense orientation and 21 nucleotides of a corresponding antisense sequence that is complementary to the sense sequence. Additional genes may also be targeted. miRNA molecules are highly efficient at inhibiting the expression of endogenous genes, and the RNA interference they induce is inherited by subsequent generations of plants.

The heterologous polynucleotide being expressed need not form the dsRNA by itself, but can interact with other sequences in the plant cell to allow the formation of the dsRNA. For example, a chimeric polynucleotide that can selectively silence the target polynucleotide can be generated by expressing a chimeric construct comprising the target sequence for a miRNA or siRNA to a sequence corresponding to all or part of the gene or genes to be silenced. In this aspect of the disclosure, the dsRNA is "formed" when the target for the miRNA or siRNA interacts with the miRNA present in the cell. The resulting dsRNA can then decrease the level of expression of the gene or genes to be silenced. See, for example, US Application Publication 2007-0130653, entitled "Methods and Compositions for Gene Silencing". The construct can be designed to have a target for an endogenous miRNA or, alternatively, a target for a heterologous and/or synthetic miRNA can be employed in the construct. If a heterologous and/or synthetic miRNA is employed, it can be introduced into the cell on the same nucleotide construct as the chimeric polynucleotide or on a separate construct. Any method can be used to introduce the construct comprising the heterologous miRNA.

In some embodiments of the method of the invention, the expression level of the identified genes in meiotic cells is reduced in floral buds using RNAi approaches including artificial microRNAs (amiRNAs), MiRNA-induced gene silencing (MIGS), Virusinduced gene silencing (VIGS), co-suppression constructs, or other RNA interferencebased approaches. In such embodiments, the targeted knockdown of genes by amiRNAs can be carried out by stable genetic transformation (e.g., Agrobacterium or particle gun) of recombinant DNA containing cassettes that express amiRNAs.

In some embodiments of the invention, the method comprises reducing the level or activity of the endogenous REC8, TAM, and/or SPO11-1, SPO11-2, MTOPVIB, PRD1, PRD2, PRD3, or DFO polypeptide/s in the parent plant or a cell thereof by introducing genome modifications into the endogenous polynucleotides encoding the REC8, TAM, and/or SPO11-1, SPO11-2, MTOPVIB, PRD1, PRD2, PRD3, or DFO polypeptide/s.

In some embodiments, a "genome modification" can be selected from the group consisting of a nucleotide insertion; a nucleotide deletion; an insertion-deletion (indel); an inversion; a nucleotide substitution; and any combination thereof. The effect of the genome modification is to disrupt the endogenous polynucleotide encoding the endogenous polypeptide, so as to reduce (or eliminate) the level or activity of the endogenous polypeptide. The genome modification may be introduced by any method known in the art, for example, by genome editing, transposon tagging, or mutagenizing plants using random or targeted mutagenesis, and subsequently selecting for plants that have the desired genome modification. The genome modification may be in a homozygous form or in a heterozygous form. The genome modification may result in a frameshift (e.g. any insertion or deletion that is not a multiple of three), failures in splicing (e.g. mutation of GT splice donor, a splicing branch point or an AG splice acceptor) or the introduction of a premature stop codon (TAA, TAG, TGA), a missense mutation, a loss-of-function mutation, or a nonsense mutation. Any other genetic insertions or deletions that cause modifications to the protein sequence may also lead to a considerable loss of function (e.g. due to the loss of even just a single critical amino acid). The genome modification has the result that the endogenous polynucleotide being targeted is not expressed or expressed at a reduced level. The size of the genome modification can be smaller than 1kb or even smaller than 0.1 kb. In some embodiments, the loss-of-function mutation is in the 5' region of the endogenous polynucleotide so as to inhibit the production of any expression product (for example in exon 1). However, the loss-of-function mutation may be in any part of the endogenous polynucleotide, such as, but not limited to, in a regulatory element (for example its promoter).

In some embodiments of the method of the invention, the genome modification is introduced by: genome editing; transposon activation; or mutagenesis. In particular embodiments, the genome modification is a loss-of-function mutation.

"Genome editing" is the modification of an endogenous polynucleotide as disclosed herein by based on targeted mutation of genes by engineered nucleases. For instance, the targeted mutation of genes by engineered nucleases can be carried out by stable genetic transformation (e.g., Agrobacterium or particle gun) of recombinant DNA containing cassettes that express engineered nucleases, or by transient delivery of protein/ribonucleoprotein engineered nuclease complexes into plant protoplasts/cells/tissues/organs.

"Transposon activation" or "transposon tagging" may also be used to decrease or eliminate the activity of one or more polypeptides of the invention. This method comprises inserting a transposon within an endogenous gene in the pathway to decrease or eliminate expression of the polypeptide. In this aspect of the invention, the expression of one or more polypeptides is decreased or eliminated by inserting a transposon within a regulatory region or coding region of the gene encoding the polypeptide. A transposon that is within an exon, intron, 5' or 3' untranslated sequence, a promoter or any other regulatory sequence of a gene may be used to decrease or eliminate the expression and/or activity of the encoded polypeptide. Methods for the transposon tagging of specific genes in plants are well known in the art. See, for example, Maes, et al., (1999) Trends Plant Sci. 4:90-96; Dharmapuri and Sonti, (1999) FEMS Microbiol. Lett. 179:53-59; Meissner et al., (2000) Plant J. 22:265-274; Phogat, et al., (2000) J. Biosci. 25:57-63; Walbot, (2000) Curr. Opin. Plant Biol. 2:103-107; Gai et al., (2000) Nucleic Acids Res. 28:94-96; Fitzmaurice et al., (1999) Genetics 153:1919-1928. In addition, the TUSC process for selecting *Mu* insertions in selected genes has been described in Bensen et al., (1995) Plant Cell 7:75-84; Mena et al., (1996) Science 274:1537-1540; and US Patent Number 5,962,764.

When "mutagenesis" is used, this may be by using ethyl methanesulfonate or by gamma irradiation. Mutations in conserved residues are particularly effective in inhibiting the activity of an encoded protein. Conserved residues of plant polypeptides suitable for mutagenesis with the goal to eliminate activity have been described. Parent plants in which the endogenous gene has been mutated or deleted are then identified. For examples of these methods see Ohshima et al., (1998) Virology 243:472-481; Okubara et al., (1994) Genetics 137:867-874; and Quesada et al., (2000) Genetics 154:421-436. In addition, a fast and automatable method for screening for chemically induced mutations, TILLING (Targeting Induced Local Lesions In Genomes), using denaturing HPLC or selective endonuclease digestion of selected PCR products is also applicable. See McCallum et al., (2000) Nat. Biotechnol. 18:455-457. If the mutagenesis is not carried out in a combinatorial manner, but by individual mutagenesis of each gene or polynucleotide of the invention, then the different mutations can be combined by hybridization and genotyping, for instance to establish triple mutant plants. See, for example, Gruis et al., (2002) Plant Cell 14:2863-2882.

In some embodiments of the method of the invention, the genome editing is by means of a DNA editing system selected from the group consisting of: CRISPR/Cas9, CRISPR/Cas12a (Cpf1), a base editor (e.g. cytidine base editors), a prime editor (e.g. e.g. fusion proteins between Cas9 H840A nickase and a reverse transcriptase enzyme), a transcription-activator like effector nuclease (TALEN), a zinc finger nuclease (ZFN), a homing endonuclease, and a meganuclease. In some embodiments, the prime editor is a fusion protein between Cas9 H840A nickase and a reverse transcriptase enzyme.

"Endonucleases" are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site. Endonucleases allow for precision genetic engineering of eukaryotic genomes, such as plant genomes.

As used herein, "meganucleases" are commonly grouped into four families: the LAGLIDADG family, the GIY-YIG family, the His-Cys box family and the HNH family. These families are characterized by structural motifs, which affect catalytic activity and recognition sequence. For instance, members of the LAGLIDADG family are characterized by having either one or two copies of the conserved LAGLIDADG motif. The four families of meganucleases are widely separated from one another with respect to conserved structural elements and, consequently, DNA recognition sequence specificity and catalytic activity. Meganucleases are found commonly in microbial species and have the unique property of having very long recognition sequences (>14bp) thus making them naturally very specific for cutting at a desired location. This can be exploited to make site-specific double- stranded breaks in genome editing. One of skill in the art can use these naturally occurring meganucleases, but the number of such naturally occurring meganucleases is limited. In order to overcome this challenge, mutagenesis and high throughput screening methods have been used to create meganuclease variants that recognize unique sequences. For example, various meganucleases have been fused to create hybrid enzymes that recognize a new sequence. Alternatively, DNA-interacting amino acids of the meganuclease can be altered to design sequence specific meganucleases (see e.g., US Patent 8,021,867). Meganucleases can be designed using the methods described in e.g. Certo, MT et al. Nature Methods (2012) 9:073-975; U.S. Patent Nos. 8,304,222; 8,021,867; 8,119,381; 8,124,369; 8,129,134; 8,133,697; 8,143,015; 8,143,016; 8,148,098; or 8,163,514. Alternatively, meganucleases with site-specific cutting characteristics can be obtained using commercially available technologies e.g. Precision Biosciences' Directed Nuclease Editor^{™} genome editing technology.

"Zinc finger nucleases" (or "ZFNs") and "transcription-activator like effector nucleases" (or "TALENs"), as used herein, have proven to be effective at producing targeted double-stranded breaks (see Christian M, Cermak T, Doyle EL, et al. Targeting DNA double-strand breaks with TAL effector nucleases. Genetics. 2010;186(2):757-761. doi:10.1534/genetics.110.120717). In essence, ZFNs and TALENs restriction endonuclease technology utilizes a non-specific DNA cutting enzyme linked to a specific DNA binding domain (either a series of zinc finger domains or TALE repeats, respectively). Typically, a restriction enzyme whose DNA recognition site and cleaving site are separate from each other is selected. The cleaving portion is separated and then linked to a DNA binding domain, thereby yielding an endonuclease with very high specificity for a desired sequence. An exemplary restriction enzyme with such properties is Fokl. Additionally, Fokl has the advantage of requiring dimerization to have nuclease activity, which means that the specificity increases, because each nuclease partner recognizes a unique DNA sequence. To enhance this effect, Fokl nucleases have been engineered that can only function as heterodimers and have increased catalytic activity. Such nucleases avoid the possibility of unwanted homodimer activity and increase specificity of the double-stranded break. Thus, to target a specific site, ZFNs and TALENs are constructed as nuclease pairs, with each member of the pair designed to bind adjacent sequences at the targeted site. Upon transient expression in cells, the nucleases bind to their target sites and the Fokl domains heterodimerize to create a double-strand break. Repair of these double-stranded breaks through the "non-homologous end-joining" (or "NHEJ") pathway often results in small deletions or small sequence insertions. Since each repair made by NHEJ is unique, the use of a single nuclease pair can produce an allelic series with a range of different deletions at the target site. The deletions typically range anywhere from a few base pairs to a few hundred base pairs in length, but larger deletions have been successfully generated in cell culture by using two pairs of nucleases simultaneously (Carlson DF, Fahrenkrug SC, Hackett PB. Targeting DNA With Fingers and TALENs. Mol Ther Nucleic Acids. 2012;1(1):e3. Published 2012 Jan 24. doi:10.1038/mtna.2011.5). In addition, when a fragment of DNA with homology to the targeted region is introduced in conjunction with the nuclease pair, the double-strand break or double-stranded break can be repaired via homologous recombination (HR) to generate specific modifications (Urnov, F., Miller, J., Lee, Y. et al. Highly efficient endogenous human gene correction using designed zinc-finger nucleases. Nature 435, 646-651 (2005). https://doi.org/10.1038/nature03556). Although the nuclease portions of both ZFNs and TALENs have similar properties, the difference between these engineered nucleases is in their DNA recognition peptide. ZFNs rely on Cys2-His2 zinc fingers, and TALENs on TALEs. Both of these DNA recognizing peptide domains have the characteristic that they are naturally found in combinations in their proteins. Cys2-His2 Zinc fingers are typically found in repeats that are 3bp apart, and are found in diverse combinations in a variety of nucleic acid interacting proteins. TALEs, on the other hand, are found in repeats with a one-to-one recognition ratio between the amino acids and the recognized nucleotide pairs. Because both zinc fingers and TALEs happen in repeated patterns, different combinations can be tried to create a wide variety of sequence specificities. Approaches for making site-specific zinc finger endonucleases include, for example, modular assembly (where Zinc fingers correlated with a triplet sequence are attached in a row to cover the required sequence), OPEN (low-stringency selection of peptide domains vs. triplet nucleotides followed by high-stringency selections of peptide combination vs. the final target in bacterial systems), and bacterial one-hybrid screening of zinc finger libraries, among others. ZFNs can also be designed and obtained commercially from, for example, Sangamo Biosciences^{™} (Richmond, CA). Methods for designing and obtaining TALENs are described in Reyon et al. Nature Biotechnology (2012) 30(5): 460-465; Miller et al. Nature Biotechnology (2011) 29: 143-148; Cermak et al. Nucleic Acids Research (2011) 39(12): e82, and Zhang et al. Nature Biotechnology (2011) 29(2): 149-153. A recently developed web-based program named *"Mojo Hand"* was introduced by Mayo Clinic for designing TAL and TALEN constructs for genome editing applications (can be accessed through www.talendesign.org).

As used herein, "homing endonucleases" are double-stranded DNases that have large, asymmetric recognition sites (12 to 40 base pairs (bp)) and coding sequences that are usually embedded in either introns or inteins (Belfort, M. and Roberts, R.J. (1997) Nucleic Acids Research, 25, 3379-3388). Introns are spliced out of precursor RNAs, while inteins are spliced out of precursor proteins (Dujon, B. et al. (1989) Gene, 82, 115-118; Perler, F.B. et al. (1994) Nucleic Acids Research, 22, 1125-1127). Homing endonucleases are named using conventions similar to those of restriction endonucleases with intron-encoded endonucleases containing the prefix, "1-" and intein endonucleases containing the prefix, "PI-" (Belfort, M. and Roberts, R.J. (1997) Nucleic Acids Research, 25, 3379-3388; Roberts, R.J. et al. (2003) Nucleic Acids Research, 31, 1805-1812). Homing endonuclease recognition sites are rare. For example, an 18-base pair (bp) recognition sequence will occur only once in every 7 × 10¹⁰ base pairs of random sequence. However, unlike restriction endonucleases, homing endonucleases tolerate some sequence degeneracy within their recognition sequence (Gimble, F.S. and Wang, J. (1996) Journal of Molecular Biology, 263, 163-180; Argast, M.G. et al. (1998) Journal of Molecular Biology, 280, 345-353). That is, single base changes do not abolish cleavage but reduce its efficiency to variable extents. As a result, their observed sequence specificity is typically in the range of 10 to 12 base pairs.

As used herein, a "CRISPR-associated endonuclease" (or "Cas") refers to an endonuclease having an RNA-guided polynucleotide-editing activity and is one of the components of the CRISPR/Cas system for genome editing, which uses at least one additional component, a "guide RNA" (gRNA). In some embodiments of the invention, the "CRISPR-associated endonuclease" is a "Cas9 endonuclease" (or "Cas9"). According to some embodiments, the "CRISPR-associated endonuclease" may be any Cas9 known in the art, such as, but not limited to, SpCas9, SaCas9, FnCas9, NmCas9, St1Cas9, BlatCas9 (Shota Nakade, Takashi Yamamoto & Tetsushi Sakuma (2017), Cas9, Cpf1 and C2c1/2/3-What's next?, Bioengineered, 8:3, 265-273, and references therein). In other embodiments, the *"CRISPR-associated endonuclease"* may be Cpf1, such as, but not limited to, AsCpf1 or LbCpf1 (Shota Nakade, Takashi Yamamoto & Tetsushi Sakuma (2017), Cas9, Cpf1 and C2c1/2/3-What's next?, Bioengineered, 8:3, 265-273, and references therein).

The terms "guide RNA" or "gRNA" as used herein may be used interchangeably and refer to a polynucleotide which facilitates the specific targeting of a CRISPR-associated endonuclease or a modified CRISPR-associated endonuclease to a target sequence such as a genomic or episomal sequence in a cell. According to some embodiments, gRNAs can be chimeric/uni-molecular (comprising a single RNA molecule, also referred to as single guide RNA or sgRNA) or modular (comprising more than one separate RNA molecule, typically a crRNA and tracrRNA which may be linked, for example by duplexing). According to some embodiments, a gRNA is an sgRNA.

The sgRNA is an RNA molecule which includes both the tracrRNA and crRNA (and a connecting loop). The sgRNA comprises a nucleotide sequence encoding the target homologous sequence (crRNA) and the endogenous bacterial RNA that links the crRNA to the Cas nuclease (tracrRNA) in a single chimeric transcript. This region of the crRNA, known as the variable region, confers the cutting specificity of the associated endonuclease and is typically 20 nucleotides in length, but can be between about 17 to 20 nucleotides in length. The gRNA/Cas complex is recruited to the target sequence by the base-pairing between the gRNA sequence and the complement genomic DNA. For successful binding of Cas, the genomic target sequence must also contain the correct Protospacer Adjacent Motif (PAM) sequence immediately following the target sequence. The binding of the gRNA/Cas complex localizes the Cas to the genomic target sequence so that the Cas can cut both strands of the DNA causing a double-strand or double-stranded break. Just as with ZFNs and TALENs, the double-stranded breaks produced by CRISPR/Cas can be repaired by HR (homologous recombination) or NHEJ (non-homologous end-joining), and are susceptible to specific sequence modification during DNA repair. The Cas nuclease has two functional domains: RuvC and HNH, each cutting a different DNA strand. When both of these domains are active, the Cas causes double strand breaks in the genomic DNA. A significant advantage of CRISPR/Cas is the high efficiency of this system coupled with the ability to easily create synthetic gRNAs. This creates a system that can be readily modified to target different genomic sites and/or to target different modifications at the same site. Additionally, protocols have been established which enable simultaneous targeting of multiple genes. The majority of cells carrying the mutation present biallelic mutations in the targeted genes. However, apparent flexibility in the base-pairing interactions between the gRNA sequence and the genomic DNA target sequence allows imperfect matches to the target sequence to be cut by Cas.

Modified versions of the Cas enzyme containing a single inactive catalytic domain, either RuvC- or HNH-, are called 'nickases'. With only one active nuclease domain, the Cas nickase cuts only one strand of the target DNA, creating a single-strand break or *"nick".* A single-strand break or single-stranded break, or nick, is mostly repaired by single strand break repair mechanism involving proteins such as but not only, PARP (sensor) and XRCCl/LIG III complex (ligation). If a single strand break (SSB) is generated by topoisomerase I poisons or by drugs that trap PARP1 on naturally occurring SSBs then these could persist and when the cell enters into S-phase and the replication fork encounter such SSBs they will become single ended DSBs which can only be repaired by HR. However, two proximal, opposite strand nicks introduced by a Cas nickase are treated as a double-strand break, in what is often referred to as a "double nick" CRISPR system. A double-nick which is basically non-parallel DSB can be repaired like other DSBs by HR or NHEJ depending on the desired effect on the gene target and the presence of a donor sequence and the cell cycle stage (HR is of much lower abundance and can only occur in S and G2 stages of the cell cycle). Thus, if specificity and reduced off-target effects are crucial, using the Cas nickase to create a double-nick by designing two gRNAs with target sequences in close proximity and on opposite strands of the genomic DNA would decrease off-target effect as either gRNA alone will result in nicks that are not likely to change the genomic DNA, even though these events are not impossible.

As used herein, a "modified CRISPR-associated endonuclease" (or "modified Cas") refers to a Cas in which the catalytic domain has been altered and/or which are fused to additional domain. According to some embodiments, a "modified Cas" refers to a Cas which contains inactive catalytic domains (dead Cas, or dCas) and has no nuclease activity while still being able to bind to DNA based on gRNA specificity. According to some embodiments, a "modified Cas" refers to a Cas which has a nickase activity ("nCas9"), thus inducing a single strand break. In some embodiments, the modified CRISPR-associated endonuclease is a "modified Cas9 endonuclease", possibly a catalytically inactive Cas9 (or "dCas9") or a nickase Cas9 ("nCas9"). The dCas can be utilized as a platform for DNA transcriptional regulators to activate or repress gene expression by fusing the inactive enzyme to known regulatory domains. For example, the binding of dCas alone to a target sequence in genomic DNA can interfere with gene transcription. There are a number of publically available tools available to help choose and/or design target sequences as well as lists of bioinformatically determined unique gRNAs for different genes in different species such as the Feng Zhang lab's Target Finder, the Michael Boutros lab's Target Finder "E-CRISP", the RGEN Tools: "Cas-OFFinder", the CasFinder: Flexible algorithm for identifying specific Cas9 targets in genomes and the CRISPR Optimal Target Finder.

In the context of the invention, modified Cas, such as dCas or nCas9, can also be used according to some embodiments together with other enzymes (possibly as a fusion protein) for base-editing. Base editing is a genome editing approach that uses components from CRISPR systems together with other enzymes to directly install point mutations into cellular DNA or RNA without making double-stranded DNA breaks. DNA base editors comprise a catalytically disabled nuclease fused to a nucleobase deaminase enzyme and, in some cases, a DNA glycosylase inhibitor. RNA base editors achieve analogous changes using components that target RNA. Base editors directly convert one base or base pair into another, enabling the efficient installation of point mutations in non-dividing cells without generating excess undesired editing byproducts (Rees and Liu (2018), "Base Editing: Precision Chemistry on the Genome and Transcriptome of Living Cells", Nature Reviews Genetics, 19(12): 770-788). According to some embodiments, the modified Cas9 is an nCas fused to a base editor enzyme such as an adenosine or cytidine deaminase. Particular base editors contemplated include APOBEC, BE1, BE2, BE3, HF-BE3, BE4, BE4max, BE4-GAM, YE1-BE3, EE-BE3, YE-BE3, YEE-BE3, VQR-BE3, VRER-BE3, Sa-BE3, Sa-BE4, SaBE4-Gam, SaKKH-BE3, Cas12a-BE, Target-AID, Target-AID-NG, xBE3, eA3A-BE3, A3A-BE3, BE-PLUS, TAM, CRISPR-X, ABE7.9, ABE7.10, ABE7.10*, xABE, ABESa, VQR-ABE, VRER-ABE, SaKKH-ABE (Rees and Liu (2018), "Base Editing: Precision Chemistry on the Genome and Transcriptome of Living Cells", Nature Reviews Genetics, 19(12): 770-788).

As used herein, the "guide RNA" (or "gRNA") is not limited to a particular sequence, provided that the sequence is either specific to the polynucleotide or polynucleotides of the invention being targeted. Example 5'-target sequences to introduce into single guide RNAs (where a single guide RNA contains a 5'-target sequence and a 3'-scaffold sequence) for any of the genome modifications of the invention are provided herein. Example guide RNA sequences for introducing such genome modifications are also provided herein. In some embodiments of the method of the invention, at least one guide RNA comprises a variable region having a sequence selected from the group consisting of SEQ ID NOs.: 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, and 144, and any combination of the foregoing.

In some embodiments of the method of the invention, the method comprises: providing to a parent plant cell a CRISPR/Cas9 endonuclease and guide RNAs specific to each of the endogenous polynucleotides encoding the endogenous REC8, TAM, and/or SPO11-1, SPO11-2, MTOPVIB, PRD1, PRD2, PRD3, or DFO polypeptide/s; allowing a ribonucleoprotein complex formed by the CRISPR/Cas9 endonuclease and guide RNAs to introduce a double-strand break at the endogenous polynucleotides encoding the endogenous REC8, TAM, and/or SPO11-1, SPO11-2, MTOPVIB, PRD1, PRD2, PRD3, or DFO polypeptide/s so as to introduce the genome modifications in the genome of the parent plant cell; and regenerating the parent plant from the plant cell.

In this context, "regenerating" may comprise growing parent plant cells (including protoplasts) into parent plants by first growing the parent plant cells into groups that develop into a callus, followed by the regeneration of shoots (caulogenesis) from the callus using plant tissue culture methods. The growth of protoplasts into callus and subsequent regeneration of shoots requires the proper balance of plant growth regulators in the tissue culture medium that must be customised. Protoplasts may also be used for plant breeding, using a technique called protoplast fusion. Protoplasts can be induced to fuse by using an electric field or a solution of polyethylene glycol. This technique may be used to generate somatic hybrids in tissue culture. Methods of protoplast regeneration are well known in the art. Several factors affect the isolation, culture, and regeneration of protoplasts, namely the genotype, the donor tissue and its pre-treatment, the enzyme treatment for protoplast isolation, the method of protoplast culture, the culture, the culture medium, and the physical environment (see Maheshwari et al. (1986), "Differentiation of Protoplasts and of Transformed Plant Cells": 3-36. Springer-Verlag, Berlin). The regenerated parent plants can be subjected to selection.

In some embodiments of the method of the invention, the CRISPR/Cas9 endonuclease and guide RNAs are provided to the parent plant cell by stable transformation of at least one recombinant DNA construct. In such embodiments, the CRISPR/Cas9 endonuclease can be provided to the parent plant cell by stable transformation of a recombinant DNA construct, and the guide RNAs can be provided to the parent plant cell in RNA form.

In this context, "recombinant" refers to a nucleic acid sequence comprising a combination of nucleic acid molecules that would not otherwise exist in nature. Recombinant nucleic acids as referred to herein may be synthesised recombinant nucleic acids.

In this context, "stable transformation" refers to the introduction of a heterologous polynucleotide by a transformation step. The heterologous polynucleotide can be stably integrated within the genome such that the polynucleotide is heritable (capable of being passed on to successive generations). The heterologous polynucleotide may be integrated into the genome alone or as part of a recombinant DNA construct. A transgenic plant can also comprise more than one heterologous polynucleotide within its genome.

In contrast, "transient transformation" refers to the transfer of a nucleic acid fragment into the nucleus, or other DNA-containing organelle, of a host plant resulting in gene expression without integration or stable inheritance. Host plants containing the transformed nucleic acid fragments are referred to as "transgenic" plants.

"Transformation" methods include the use of liposomes, electroporation, chemicals that increase free DNA uptake, injection of the DNA directly into the plant, particle gun bombardment, Agrobacterium mediate transformation, transformation using viruses or pollen, or microprojection. Methods may be selected from the calcium/polyethylene glycol method for protoplasts, electroporation of protoplasts, microinjection into plant material, DNA or RNA-coated particle bombardment, and infection with (non-integrative) viruses. Methods for coating DNA or RNA onto microparticles are well known to those of ordinary skill in the art, for example the methods described in Ismagul et al BMC Plant Biology 2018 18: 135, or Kikkert JR. Cell biology: a laboratory handbook, vol. 4. San Diego: Academic Press; 1998. p. 157-61, or Sanford JC, et al. Methods Enzymol. 1993 217:483-509. Accordingly, suitable microparticles complexed with the polynucleotide and/or the vector of the composition are known to the skilled person. Transgenic plants, including transgenic crop plants, are preferably produced via *Agrobacterium tumefaciens* mediated transformation.

Thus, a polynucleotide may be "introduced" into a plant such that it is expressed as a transgene. Nucleic acids are "introduced" into a plant through transformation. Transformation encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide and/or vector may be transiently or stably introduced into a host cell and may be maintained non-integrated, for example, as a plasmid. Alternatively, it may be integrated into the host genome. The resulting transformed plant cell may then be used to regenerate a transformed plant in a manner well known in the art.

"Introduced" includes reference to the incorporation of a nucleic acid into a cell where the nucleic acid may be incorporated into the genome of the cell, and includes reference to the transient provision of a nucleic acid or protein to the cell. Introduced includes reference to stable or transient transformation methods, as well as sexually crossing. Thus, "introduced" in the context of inserting a nucleic acid fragment (e.g., a recombinant DNA construct/expression construct) into a cell, means "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

To select transformed plants, plant material obtained in the transformation is, as a rule, subjected to selective conditions so that transformed plants can be distinguished from untransformed plants. For example, seeds obtained in the above-described manner can be planted and, after an initial growing period, subjected to a suitable selection by spraying. A further possibility is growing the seeds, if appropriate after sterilization, on agar plates using a suitable selection agent so that only the transformed seeds can grow into plants. Alternatively, the transformed plants are screened for the presence of a selectable marker either visually using fluorescent of color based reporters, or using molecular techniques to detect the presence of the polynucleotide of the invention. Following DNA transfer and regeneration, putatively transformed plants may also be evaluated, for instance using Southern blot analysis or PCR analysis, for the presence of the gene of interest, copy number and/or genomic organisation. Alternatively or additionally, expression levels of the newly introduced DNA may be monitored using Northern and/or Western analysis, or by rtPCR or RNA-Seq all such techniques being well known in the art.

In some embodiments of the method of the invention, the at least one recombinant DNA construct, guide RNAs, or ribonucleoprotein complex can be provided to the parent plant cell using a method selected from the group consisting of: particle bombardment; *Agrobacterium* transformation; protoplast transfection; electroporation; and nanoparticle-mediated transfection.

In alternative embodiments of the method of the invention, the CRISPR/Cas9 endonuclease and guide RNAs can be provided to the parent plant cell by transient delivery of a ribonucleoprotein complex.

In some embodiments of the method of the invention, the method further comprises validating the presence of the genome modifications. In particular embodiments, the validating may comprise PCR amplification and DNA sequencing; or PCR amplification and enzymatic digestion.

As used herein, "validating" can include any technique known in the art capable of detecting the genome modification, such as, but not limited to, DNA sequencing (e.g. Sanger/Illumina/PacBio sequencing), electrophoresis, an enzymebased mismatch-detection assay, and a hybridization assay such as PCR, RT-PCR, RNase protection, *in-situ* hybridization, primer extension, Southern blot, Northern Blot and dot blot analysis. Various methods used for detection of single nucleotide polymorphisms (SNPs) can also be used, such as PCR based T7 endonuclease, Heteroduplex and Sanger sequencing. In particular embodiments, genome modifications are identified by PCR amplification followed by DNA sequencing or enzymatic digestion (e.g. dCAPS).

Another method of validating the presence of a genome modification (such as insertion-deletion events (or *"indels"*)) comprises a mismatch cleavage assay that makes use of a structure selective enzyme (e.g. endonuclease) that recognizes and cleaves mismatched DNA. The mismatch cleavage assay is a simple method for the detection of indels and is therefore the typical procedure to detect mutations induced by genome editing. The assay uses enzymes that cleave heteroduplex DNA at mismatches and extrahelical loops formed by multiple nucleotides, yielding two or more smaller fragments. A PCR product of about 300 to 1000 bp is generated with the predicted nuclease cleavage site off-centre so that the resulting fragments are dissimilar in size and can easily be resolved by conventional gel electrophoresis or high-performance liquid chromatography (HPLC). End-labelled digestion products can also be analysed by automated gel or capillary electrophoresis. The frequency of indels at the locus can be estimated by measuring the integrated intensities of the PCR amplicon and cleaved DNA bands. The digestion step takes 15 to 60 minutes, and when the DNA preparation and PCR steps are added, the entire assays can be completed in less than 3 hours. Two alternative enzymes are typically used in this assay. T7 endonuclease 1 (T7E1) is a resolvase that recognises and cleaves imperfectly matched DNA at the first, second or third phosphodiester bond upstream of the mismatch. The sensitivity of a T7E1 -based assay is 0.5 to 5 %. In contrast, Surveyor^{™} nuclease (Transgenomic Inc., Omaha, NE, USA) is a member of the CEL family of mismatch-specific nucleases derived from celery. It recognizes and cleaves mismatches due to the presence of single nucleotide polymorphisms (SNPs) or small indels, cleaving both DNA strands downstream of the mismatch. It can detect indels of up to 12 nucleotides and is sensitive to mutations present at frequencies as low as about 3%, *i.e.* 1 in 32 copies.

Yet another method of validating the presence of a genome modification comprises high-resolution melting analysis. High-resolution melting analysis (HRMA) involves the amplification of a DNA sequence spanning the genomic target (90 to 200 bp) by real-time PCR with the incorporation of a fluorescent dye, followed by melt curve analysis of the amplicons. HRMA is based on the loss of fluorescence when intercalating dyes are released from double-stranded DNA during thermal denaturation. It records the temperature-dependent denaturation profile of amplicons and detects whether the melting process involves one or more molecular species.

Yet another method of validating the presence of a genome modification is the heteroduplex mobility assay. Mutations can also be detected by analysing rehybridized PCR fragments directly by native polyacrylamide gel electrophoresis (PAGE). This method takes advantage of the differential migration of heteroduplex and homoduplex DNA in polyacrylamide gels. The angle between matched and mismatched DNA strands caused by an indel means that heteroduplex DNA migrates at a significantly slower rate than homoduplex DNA under native conditions, and they can easily be distinguished based on their mobility. Fragments of 140 to 170 bp can be separated in a 15% polyacrylamide gel. The sensitivity of such assays can approach 0.5% under optimal conditions, which is similar to T7E1. After reannealing the PCR products, the electrophoresis component of the assay takes about 2 hours. Other methods of validating the presence of editing events are described in length in Zischewski (2017), Biotechnology Advances 1(1):95-104.

In some embodiments of the method of the invention, the method further comprises validating the genome ploidy level of the male and female gametes produced by the parent plant. In some embodiments, the validating comprises particle size detection, for instance high-throughput measurement of pollen size by means of a Multisizer particle size detector. In some embodiments, the validating comprises Alexander staining of pollen and imaging so as to identify viable unreduced pollen grains. In some embodiments, the validating comprises meiotic chromosome spreading, which allows checking chromosome number and observing their behaviour. In some embodiments, the validating comprises chromosome counting from root tip and/or meiotic cells (meiocytes). In some embodiments, the validating comprises flow cytometry of leaf nuclei stained with DAPI. In some embodiments, the validating comprises whole genome sequencing and bioinformatic analysis to check for presence of genetic markers.

In some embodiments of the method of the invention, the method further comprises vegetatively propagating the parent plant. For instance, the vegetative propagation may be by means of a stem cutting, side-shoot, or tuber, instead of by sexual reproduction.

In a further aspect, the invention provides a parent plant obtainable by the method as described above. Also provided by the invention is a plant part or plant cell of such a parent plant.

As used herein, the term "plant" refers to any of various photosynthetic, eukaryotic multicellular organisms of the kingdom Plantae, characteristically producing embryos, containing chloroplasts, having cellulose cell walls and lacking locomotion. As used herein, a "plant" includes any plant or part of a plant at any stage of development, including seeds, suspension cultures, plant cells, embryos, meristematic regions, callus tissue, leaves, roots, shoots, gametophytes, sporophytes, pollen, microspores, and progeny thereof. Also included are cuttings, and cell or tissue cultures. As used herein, plants that are capable of apomeiosis can produce clonal spores and gametes with no reduction in genome ploidy (with respect parental plant genotype and ploidy). This is in contrast to normal sexual plants where meiosis takes place and homologous chromosomes recombine and a reduction of ploidy occurs (e.g. a diploid parent produces haploid gametes that have recombined chromosomes). Apomeiosis skips these two hallmarks of meiosis (e.g. a diploid parent produces diploid gametes that are genetically identical to the parent). In some plants capable of apomeiosis, both male and female reproduction can lead to functional clonal spores and gametes, whilst in other plants only male or female reproduction leads to functional clonal spores and gametes. A "MiMe plant", as referred to herein, is an example of a plant that is capable of apomeiosis. MiMe plants typically produce clonal, unreduced male and female gametes.

"Plant tissue" includes, for example, whole plants, plant cells, plant organs, e.g., leave, stems, roots, meristems, plant seeds, protoplasts, callus, cell cultures, and any groups of plant cells organized into structural and/or functional units. "Plant parts", as used herein, include differentiated and undifferentiated tissues including, but not limited to root (including tuber), rootstock, stem, scion, shoot, fruit, leaf, microspore, pollen, seed, tumour tissue, and various forms of cells and culture (e.g. single cells, protoplasts, embryos, embryonic cells, and callus tissue). In some particular embodiments, the plant part is a tuber, for instance a potato tuber, or a fruit, such as a tomato fruit. In other embodiments, the plant part is a seed. The term "seed" as used herein refers to a unit of reproduction of a flowering plant capable of developing into another such plant.

The "plant cell", as used herein, may be from any plant tissue such as leaf disks, pollen, embryos, cotyledons, hypocotyls, megagametophytes, callus tissue, existing meristematic tissue (e.g. apical meristem, axillary buds, and root meristems), and induced meristem tissue (e.g. cotyledon meristem and hypocotyl meristem). In any of the embodiments of the present invention, the parent plant, plant, plant part, plant cell, protoplast, microspore, or seed may not exclusively be obtained by means of an essentially biological process.

In some embodiments of the method of the invention, the method further comprises producing a microspore from the parent plant. In some of these embodiments, the microspore is treated with a chromosome-doubling agent such as, but not limited to, colchicine. Such methods for doubling the ploidy of cells are well known in the art.

In some embodiments of the method of the invention, the method further comprises producing a seed from the parent plant, wherein the seed has a genome ploidy level that is higher than, optionally double, the ploidy level of the parent plant. In such embodiments, the seed can be produced by selfing the parent plant. In alternative embodiments, the seed can be produced by crossing the parent plant with a second, different parent plant also having a reduced level or activity of the endogenous REC8, TAM, and SPO11-1, SPO11-2, MTOPVIB, PRD1, PRD2, PRD3, or DFO polypeptides. In any of these embodiments, the first and second, different parent plants can both be diploid hybrid plants, and the seed may comprise an embryo having a genome which comprises four genetically distinct genome haplotypes.

In some embodiments of the method of the invention, the method further comprises producing a seed from the parent plant by crossing the parent plant with a second, different parent plant, wherein the second, different plant does not have a reduced level or activity of the endogenous REC8, TAM, and SPO11-1, SPO11-2, MTOPVIB, PRD1, PRD2, PRD3, or DFO polypeptides and is: diploid; triploid; or tetraploid.

In some embodiments of the method of the invention, the method further comprises producing a seed from the parent plant by pollinating the parent plant with a haploid inducer plant, wherein the seed has a genome ploidy level that is the same as the ploidy level of the parent plant. Haploid inducer plants are known in the art. In such embodiments, the haploid inducer plant may a reduced level or activity of an endogenous DMP polynucleotide, so as to inhibit gamete fusion. In some embodiments, the endogenous DMP polynucleotide: has at least 90% sequence identity to a polynucleotide selected from the group consisting of: SEQ ID NOs.: 145, 146, 148, 149, 151, 152, 154, and 155. In some embodiments, the endogenous DMP polynucleotide: encodes a polypeptide having at least 90% sequence identity to a polypeptide selected from the group consisting of: SEQ ID NOs.: 147, 150, 153, and 156. In some embodiments, the haploid inducer comprises a genome modification in the endogenous DMP polynucleotide. In some embodiments, the guide RNAs of SEQ ID NOs: 157 and 158 are used to introduce the genome modification into the endogenous DMP polynucleotide in tomato. In some embodiments, the guide RNAs of SEQ ID NOs: 159 and 160 are used to introduce the genome modification into the endogenous DMP polynucleotide in potato.

In another aspect, the invention provides a microspore or seed obtainable by the method described above.

In a further aspect, the invention provides a parent plant capable of apomeiosis, so as to produce viable clonal male and female gametes having the same genome ploidy level as the parent plant, having: a reduced level or activity of an endogenous REC8 polypeptide, so as to ablate sister chromatid cohesion; a reduced level or activity of an endogenous TAM polypeptide, so as to prevent entry to the second meiotic division; and a reduced level or activity of an endogenous SPO11-1, SPO11-2, MTOPVIB, PRD1, PRD2, PRD3, or DFO polypeptide, so as to ablate meiotic recombination; wherein the parent plant is from the Solanaceae family. As indicated elsewhere herein, the term *"plant"* refers to whole plants, grafted plants, ancestors and progeny of the plants, plant organs, plant tissues, and *"plant parts". "Plant parts",* as used herein, include differentiated and undifferentiated tissues including, but not limited to root (including tuber), rootstock, stem, scion, shoot, fruit, leaf, microspore, pollen, seed, tumour tissue, and various forms of cells and culture (e.g. single cells, protoplasts, embryos, embryonic cells, and callus tissue). In some particular embodiments, the plant part is a tuber, for instance a potato tuber, or a fruit, such as a tomato fruit. In other embodiments, the plant part is a seed. The term "seed" as used herein refers to a unit of reproduction of a flowering plant capable of developing into another such plant. The "plant cell" may be from any plant tissue such as leaf disks, pollen, embryos, cotyledons, hypocotyls, megagametophytes, callus tissue, existing meristematic tissue (e.g. apical meristem, axillary buds, and root meristems), and induced meristem tissue (e.g. cotyledon meristem and hypocotyl meristem). In any of the embodiments of the present invention, the parent plant, plant, plant part, plant cell, protoplast, microspore, or seed may not exclusively be obtained by means of an essentially biological process.

In some embodiments of the parent plant of the invention, the parent plant is a tomato plant. In some embodiments, the endogenous REC8 polypeptide: has at least 90%, 91%, 92%, 93%, 94%, 95%, 96%, 97%, 98%, 99%, or complete sequence identity to the polypeptide of SEQ ID NO.: 6, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 4 and 5, or a fragment thereof. In some embodiments, the endogenous TAM polypeptide: has at least 90%, 91%, 92%, 93%, 94%, 95%, 96%, 97%, 98%, 99%, or complete sequence identity to the polypeptide of SEQ ID NO.: 9, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 7 and 8, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 3, 12, 15, 18, 21, 24, and 27, or fragment thereof In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 1, 2, 10, 11, 13, 14, 16, 17, 19, 20, 22, 23, 25, and 26, or a fragments thereof.

In some embodiments of the parent plant of the invention, the tomato plant comprises a genome selected from the group consisting of: *Solarium lycopersicum, Solarium pimpinellifolium, Solarium galapagense, Solarium cheesmaniae, Solarium arcanum, Solanum chmielewskii, Solanum neorickii, Solanum chilense, Solanum corneliomulleri, Solanum habrochaites, Solanum huaylasense, Solanum peruvianum,* and *Solanum pennellii.* In some embodiments, the tomato plant comprises a genome selected from any other species in the *Solanum* genus that can be hybridized with any of the species listed above (such as *Solanum lycopersicoides*). In some embodiments, the tomato plant is a hybrid of any of the above, in the first F1 hybrid generation or any generation thereafter.

In some embodiments of the parent plant of the invention, the parent plant is a potato plant. In some embodiments, the endogenous REC8 polypeptide: has at least 90%, 91%, 92%, 93%, 94%, 95%, 96%, 97%, 98%, 99%, or complete sequence identity to the polypeptide of SEQ ID NO.: 33, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 31 and 32, or a fragment thereof. In some embodiments, the endogenous TAM polypeptide: has at least 90%, 91%, 92%, 93%, 94%, 95%, 96%, 97%, 98%, 99%, or complete sequence identity to the polypeptide of SEQ ID NO.: 36, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 34 and 35, or a fragment thereof In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 30, 39, 42, 45, 48, 51, and 54, or a fragment thereof In some embodiments, the endogenous SPO11-1, SPO11-2, MTOPVIB, PRD1, PRD2, PRD3, or DFO polypeptide is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 28, 29, 37, 38, 40, 41, 43, 44, 46, 47, 49, 50, 52, and 53, or a fragment thereof.

In some embodiments of the parent plant of the invention, the potato plant comprises a genome selected from the group consisting of: *Solanum tuberosum, Solanum tuberosum* group stenotomum, *Solanum tuberosum* group phureja, *Solanum tuberosum* group goniocalyx, *Solanum tuberosum* group ajanhuiri, *Solanum candolleanum, Solanum pinnatisectum, Solanum andreanum, Solanum burkartii, Solanum lignicaule, Solanum buesii, Solanum multiinterruptum, Solanum brevicaule, Solarium jamesii, Solarium piurae, Solarium morelliforme, Solarium chomatophilum, Solarium paucissectum, Solarium sogarandinum, Solarium vernei, Solarium chacoense, Solarium commersonii, Solarium boliviense, Solarium bulbocastanum, Solarium cajamarquense, Solarium neorossii, Solarium gourlayi, Solarium spegazzinii, Solarium avilesii, Solarium berthaultii, Solarium venturii, Solarium stoloniferum, Solarium hougasii,* and *Solarium demissum.* In some embodiments, the potato plant comprises a genome of any other species in the *Solarium* genus that can be hybridized with any of the species listed above (such as *Solarium etuberosum*)*.* In some embodiments, the potato plant is a hybrid of any of the above, in the first F1 hybrid generation or any generation thereafter.

In some embodiments of the parent plant of the invention, the parent plant is an eggplant plant. In some embodiments, the endogenous REC8 polynucleotide: has at least 90%, 91%, 92%, 93%, 94%, 95%, 96%, 97%, 98%, 99%, or complete sequence identity to the polypeptide of SEQ ID NO.: 60, or a fragment thereof. In some embodiments, the endogenous REC8 polynucleotide: is encoded by a polynucleotide having at least 90%, 91%, 92%, 93%, 94%, 95%, 96%, 97%, 98%, 99%, or complete sequence identity to a polynucleotide selected from SEQ ID NOs.: 58 and 59, or a fragment thereof. In some embodiments, the endogenous TAM polypeptide: has at least 90%, 91%, 92%, 93%, 94%, 95%, 96%, 97%, 98%, 99%, or complete sequence identity to a polypeptide of SEQ ID NO.: 63, or a fragment thereof. In some embodiments, the endogenous TAM polynucleotide: is encoded by a polynucleotide having at least 90%, 91%, 92%, 93%, 94%, 95%, 96%, 97%, 98%, 99%, or complete sequence identity to a polynucleotide selected from SEQ ID NOs.: 61 and 62, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 57, 66, 69, 72, 75, 78, and 81, or a fragment thereof In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 55, 56, 64, 65, 67, 68, 70, 71, 73, 74, 76, 77, 79, and 80, or a fragment thereof.

In some embodiments of the parent plant of the invention, the eggplant plant comprises a genome selected from the group consisting of: *Solarium melongena, Solarium macrocarpon, Solarium aethiopicum, Solarium aculeatissimum, Solarium anguivi, Solarium atropurpureum, Solarium campylacanthum, Solarium capense, Solarium capsicoides, Solarium dasyphylium, Solarium elaeagnifolium, Solarium ferox, Solarium incanum, Solarium indicum, Solarium insanum, Solarium lasiocarpum, Solarium linnaeanum, Solarium pectinatum, Solarium pseudocapsicum, Solarium quitonese, Solarium repandum, Solarium rostratum, Solarium supinum, Solarium sessiliflorum, Solarium sisymbriifolium, Solarium stramoniifolium, Solarium torvum, Solarium viarum, Solarium violaceum, Solarium virginianum,* and *Solarium xanthocarpum.* In some embodiments, the eggplant plant comprises a genome of any other species in the *Solanum* genus that can be hybridized with any of the species listed above. In some embodiments, the eggplant plant is a hybrid of any of the above, in the first F1 hybrid generation or any generation thereafter.

In some embodiments of the parent plant of the invention, the parent plant is a pepper plant. In some embodiments, the endogenous REC8 polypeptide: has at least 90%, 91%, 92%, 93%, 94%, 95%, 96%, 97%, 98%, 99%, or complete sequence identity to a polypeptide of SEQ ID NO.: 87, or a fragment thereof. In some embodiments, the endogenous REC8 polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 85 and 86, or a fragment thereof. In some embodiments, the endogenous TAM polypeptide: has at least 90%, 91%, 92%, 93%, 94%, 95%, 96%, 97%, 98%, 99%, or complete sequence identity to a polypeptide of SEQ ID NO.: 90, or a fragment thereof. In some embodiments, the endogenous TAM polypeptide: is encoded by a polynucleotide having at least 90%, 91%, 92%, 93%, 94%, 95%, 96%, 97%, 98%, 99%, or complete sequence identity to a polynucleotide selected from SEQ ID NOs.: 88 and 89, or a fragment thereof. In some embodiments, the endogenous SPO11-1, SPO11-2, MTOPVIB, PRD1, PRD2, PRD3, or DFO polypeptide: has at least 90%, 91%, 92%, 93%, 94%, 95%, 96%, 97%, 98%, 99%, or complete sequence identity to a polypeptide selected from the group consisting of SEQ ID NOs.: 84, 93, 96, 99, 102, 105, and 108, or a fragment thereof. In some embodiments, the endogenous SPO11-1, SPO11-2, MTOPVIB, PRD1, PRD2, PRD3, or DFO polypeptide: is encoded by a polynucleotide having at least 90%, 91%, 92%, 93%, 94%, 95%, 96%, 97%, 98%, 99%, or complete sequence identity to a polynucleotide selected from the group consisting of SEQ ID NOs.: 82, 83, 91, 92, 94, 95, 97, 98, 100, 101, 103, 104, 106, and 107, or a fragment thereof.

In some embodiments of the parent plant of the invention, the pepper plant comprises a genome selected from the group consisting of: *Capsicum annuum, Capsicum baccatum, Capsicum chinense, Capsicum frutescens, Capsicum pubescens, Capsicum buforum, Capsicum campylopodium, Capsicum cardenasii, Capsicum chacoense, Capsicum coccineum, Capsicum cornutum, Capsicum dimorphum, Capsicum dusenii, Capsicum eximium, Capsicum flexuosum, Capsicum galapagoense, Capsicum geminifolium, Capsicum hookerianum, Capsicum lanceolatum, Capsicum leptopodum, Capsicum lycianthoides, Capsicum minutiflorum, Capsicum mirabile, Capsicum mositicum, Capsicum parvifolium, Capsicum rhomboideum, Capsicum schottianum, Capsicum scolnikianum, Capsicum tovarii,* and *Capsicum villosum.* In some embodiments, the pepper plant comprises a genome of any other species in the *Capsicum* genus that can be hybridized with any of the species listed above. In some embodiments, the pepper plant is a hybrid of any of the above, in the first F1 hybrid generation or any generation thereafter.

In any one of the embodiments of the parent plant of the invention, the parent plant may be haploid, diploid, or polyploid. When the parent plant is polyploid, it may be triploid, tetraploid, or hexaploid.

In any one of the embodiments of the parent plant of the invention, the parent plant may be a hybrid plant.

In any one of the embodiments of the parent plant of the invention, the level of the endogenous REC8, TAM, and/or SPO11-1, SPO11-2, MTOPVIB, PRD1, PRD2, PRD3, or DFO polypeptide/s in the parent plant may be reduced by about 10%, 20%, 30%, 40%, 50%, 60%, 70%, 80%, 90%, or completely, as compared to in a control plant (such as a wild type equivalent plant).

In any one of the embodiments of the parent plant of the invention, the activity of the endogenous REC8, TAM, and/or SPO11-1, SPO11-2, MTOPVIB, PRD1, PRD2, PRD3, or DFO polypeptide/s in the parent plant is reduced by about 10%, 20%, 30%, 40%, 50%, 60%, 70%, 80%, 90%, or completely, as compared to in a control plant (such as a wild type equivalent plant).

In some of these embodiments, a "control" plant or plant cell, or wild type equivalent plant or cell, is one in which the activity of the endogenous REC8, TAM, and/or SPO11-1, SPO11-2, MTOPVIB, PRD1, PRD2, PRD3, or DFO polypeptide/s is not reduced. In some embodiments, a "control" plant or plant cell, or wild type equivalent plant or cell, lacks the genome modifications in the polynucleotides encoding the endogenous REC8, TAM, and/or SPO11-1, SPO11-2, MTOPVIB, PRD1, PRD2, PRD3, or DFO polypeptide/s.

In any one of the embodiments of the parent plant of the invention, the parent plant may comprise genome modifications in the endogenous polynucleotides encoding the REC8, TAM, and/or SPO11-1, SPO11-2, MTOPVIB, PRD1, PRD2, PRD3, or DFO polypeptide/s. In such embodiments, the genome modifications may be selected from the group consisting of: a nucleotide insertion; a nucleotide deletion; an insertion-deletion (indel); an inversion; a nucleotide substitution; and any combination the foregoing.

In another aspect, the invention provides a plant part, such as a side-shoot or tuber, or plant cell of the parent plant as described above.

In a further aspect, the invention provides a microspore or seed of the parent plant as described above. Also provided by the invention is a plant grown from such a microspore or seed, or from a tuber as described above. Further provided by the invention are plant plant parts or plant cells of any of the foregoing.

In some embodiments, the plant, plant part, or plant cell of the invention is characterised by a phenotype such as, but not limited to: of improved plant vigour, improved herbicide resistance, improved pathogen resistance, improved insect resistance, improved mite resistance, improved nematode resistance, improved parasitic plant resistance, improved herbivory resistance, improved nitrogen fixation, improved heat tolerance, improved wind tolerance, improved heavy metal tolerance, improved flooding and hypoxic stress tolerance, improved ozone tolerance, improved yield, improved lodging resistance, altered plant height and stature, increased biomass, lower utilization of fertiliser, faster life-cycle, higher nutritional content, improved taste, improved colour, improved performance as a rootstock, and/or longer shelf-life. In some embodiments, the phenotype is "heritable", so can be passed on to subsequent generations, for instance by classical plant breeding.

### Examples

The following examples are illustrative and not considered to limit the scope of the invention.

### Materials and Methods

### Seed harvesting and Seed germination

For seed harvesting, the collected seeds from fresh ripen fruits were first fixed with 2% HCl for 30 minutes, followed by washing with fully desalinated water. Thereafter, the harvested seeds will be dried under room temperature for serval weeks and then restored at cold room (4°C). Seed germination was performed in vitro by dealing with following steps: incubate seeds into 1.5mL Sterile MilliQ Water (Millipore) for 2 hours. Subsequently, incubate them into 1.5mL saturated Na₃PO₄ buffer for 20 minutes, followed by washing seeds through Millipore for three times, and then incubate seeds into 1.5mL 2.7% Sodium hypochlorite (NaClO) for 20 minutes and three times' washes by using Millipore. Afterward, transfer the seeds into 0.8% Agar under 25°C at culture room and then transfer the seedlings with roots into 1/2MS solid medium in cultural room or soil in greenhouse.

### Materials and plant growth

All the plants including control plants (wild type: *MicroTom, MbTMV; Maxeza* F1, *Funtelle* F1 and hybrid line *MbTMV-MT* F1) and mutants are growth under the condition of 25°C at day time and 19°C at night time (16 hours light and 8 hours dark, 53% relative humidity) in Bronson Chamber, Percival Chamber and Greenhouse. Tomato hybridization was accomplished through manual castration and hand pollination.

### Seed imaging

The seed populations between wild type and *Sltam* mutants were captured using advanced imaging with Leica M205 FA Digital Stereomicroscope (Leica Microsystems, Germany). Thereafter, the Leica LAS X software was used to get the original seed pictures.

### Vector construction of CRIPSR-Cas9

An efficient CRISPR-Cas9 system vector pDIRECT_22C purchased from Addgene plasmid#91135 (https://www.addgene.org/91135/) was used to knock out SPO11-1, REC8, TAM, OSD1 and DMP by manipulating the Csy-type (CRISPR system Yersinia) ribonuclease 4 (Csy4) and tRNA processing enzymes (Čermák, T. *et al.* A multipurpose toolkit to enable advanced genome engineering in plants. *Plant Cell* 29, 1196-1217 (2017)). Meanwhile, the specific guide RNAs (gRNAs) designed by CRISPR-P v2.0 (http://crispr.hzau.edu.cn/CRISPR2/) were selected to knock out the candidate genes with low rate of off-target in tomato (Liu, H. et al. CRISPR-P 2.0: An Improved CRISPR-Cas9 Tool for Genome Editing in Plants. Mol. Plant 10, 530-532 (2017)). To make sure the specificity of the targeted sgRNAs, tomato long read assemblies databases, including BGV006775, BGV006865, BGV007931, BGV007989, Brandywine, Floradade, EA00371, EA00990, LYC1410, PAS014479, PI169588 and PI303721, were used for genomic DNA blast through UCSC Genome Browser (https://genome.ucsc.edu/cgi-bin/hgBlat?command=start). The golden gate assembly approach was executed to combine multiple sgRNAs into the destination expression vector (Engler, C., Kandzia, R. & Marillonnet, S. A One Pot, One Step, Precision Cloning Method with High Throughput Capability. PLoS One 3, e3647 (2008)).

### Agrobacterium-mediated transformation in tomato

The GV3101 *Agrobacterium* strain was used for transformation with the 10 µg/ml gentamycin, 50 µg/ml kanamycin plus 20 µg/ml rifampicin antibiotics selection. Four-weeks-old fresh tomato leaves were collected to perform the transformation according to the previous method (Sun, H. J., Uchii, S., Watanabe, S. & Ezura, H. A highly efficient transformation protocol for Micro-Tom, a model cultivar for tomato functional genomics. Plant Cell Physiol. 47, 426-431 (2006)). Briefly, cut the fresh leaves in approximately 6 x 6 mm and grow them on MS-I medium (4.3 g/L MS-salt including vitamins, 100 mg/L Myo-inositol, 30 g/L Saccharose, 7 g/L PhytoAgar, 2 mL 1 mg/mL Zeatin riboside, 1 mL 73 mg/mL Acetosyringon, 5 µL 20 mg/mL IAA, pH 5.9) overnight at 25° C in the dark. Meanwhile, prepare the Agrobacterium seed culture overnight at 28° C with 200rpm shaking in advance, when the OD₆₀₀ reaches within 0.4-0.6 on next day and dilute the liquid culture 1:20 by using liquid LB without antibiotics. Incubate the leaves for 15 min with occasional middle shaking, and the incubated leaves were transferred to MS-I solid medium for 48 hours in the dark at 25° C. Transfer the leaf pieces on MS-II plates and place them in long day conditions to produce new callus at 25° C. After that, transfer the developed stems generated from callus into MS-III rooting medium with carbenicillin and kanamycin resistance. Thereafter, make the final screening for the positive transgenic plants and transfer them into greenhouse.

### Alexander staining and scanning electron microscopy (SEM)

Alexander staining was performed to identify the pollen variability of tomato plants (Alexander, M. P. Differential Staining of Aborted and Nonaborted Pollen. http://dx.doi.org/10.3109/10520296909063335 44, 117-122 (2009)). The matured pollen of opening flowers was collected by the pollen pollinator tool. The photos were taken by Zeiss Axioplan 2 Imaging Fluorescence Microscope with ZEISS Axiocam 208 Color Microscope Camera and the data of Alexander staining and pollen viability was analyzed by software Zeiss Labscope v3.1 and Gridfree software (Hu, Y. & Zhang, Z. GridFree: a python package of imageanalysis for interactive grain counting and measuring. Plant Physiol. 186, 2239-2252 (2021)).

The method of Scanning electron microscopy (SEM) was performed as previously (Gul, S. et al. Foliar epidermal anatomy of Lamiaceae with special emphasis on their trichomes diversity using scanning electron microscopy. Microsc. Res. Tech. 82, 206-223 (2019)). Pollen grains of mature opening flowers were collected into 2 mL Eppendorf tubes and then coated with palladium-gold by a Polaron Sputter Coater SC 7600 (Quorum Technologies Ltd, Britain). The pollens were spread on 25.4 mm specimen mounts (or stubs) (Plano: Product number G399F) using 25 mm conductive Carbon adhesive tabs (Plano: Product number G3348). The results were examined using a field emission scanning electron microscope (Supra 40 VP, Zeiss, Germany) with an acceleration voltage of 3 kV.

### Ploidy checking and flow cytometry analysis

The ploidy checking of plants was analyzed by flow cytometry as following steps in brief: One piece of fresh young tomato leaf was chopped by sharp razor blade within 550 µL Galbraith's buffer (45 mM MgCl2, 30 mM sodium citrate, 20 mM MOPS, 0.1% (v/v) Triton X-100, pH7.0) (Galbraith, D. W. et al. Rapid flow cytometric analysis of the cell cycle in intact plant tissues. Science 220, 1049-1051 (1983)). And then transfer the tissue materials into CellTrics disposable filters-green with 30 µm pore size (REF: 04-0042-2316, Sysmex). Add 20 µL DAPI (100 µg/mL) into 500 µL filtered sample for incubating 15 minutes and check the sample according to the manual of CytoFLEX V5-B5-R3 Flow Cytometer. The daily quality control was performed by using CytoFLEX Daily QC Fluorospheres (B53230). For the data collection, 10000 events per sample were required for each independent measurement at fast model. The final data was analyzed by CytExpert software (Beckmann Counter, Germany) and FCS Express 7 software.

### DNA extraction and Next-generation sequencing (NGS)

The total genomic DNA extraction was conducted by using the DNeasy Plant Mini Kit (Qiagen, cat. Nos. 69106). The exact mutation sites and ratios of the transgenic plants were analyzed by Illumina sequencing. The protocol of NGS was slightly modified from previous description (Brinkman, E. K., Chen, T., Amendola, M. & Van Steensel, B. Easy quantitative assessment of genome editing by sequence trace decomposition. Nucleic Acids Res. 42, (2014)). The bridge sequences and barcoding primers of target-specific PCR and barcoding PCR are designed on the basics of M13F (TGTAAAACGACGGCCAGT) and M13R (CAGGAAACAGCTATGAC). After the process of DNA purification, the library construction was carried out and the sequencing run condition was based on 2x 150 bp (paired end read). The final data was analyzed by CLC Main Workbench 21.0.5 software according the reference of Tomato Genome version SL4.0 and Annotation ITAG4.0. After importing the raw data by Illumina high-throughput sequencing, demultiplexing reads was achieved to prepare sequencing data and then map reads to reference (resequencing analysis). In addition, fixed ploidy variant detection was performed to visualize the specific mutation sites of each gene among different chromosomes. The required variant probability parameter is more than 96% and the coverage and count filter with 5% minimum frequency.

### In vitro culture and embryo rescue (ER)

The suitable fresh shoots of the plants were selected and cut in Super-clean beach. Sterilize them for 15 minutes in a 1:4 dilution of commercial bleach with 0.02% Tween, and then wash them 3 times in sterile water. Transplant them on 0.5 MS 10 medium and got sub-cultured on the same medium after 4-5 weeks. For the embryo rescue, the inventors sterilized the crossed fruit from greenhouse around 24 days after pollination (DAP) for 20 minutes in a 1:2 dilution of commercial bleach with 0.2% Tween. Then wash the fruits with sterile water for 3 times and isolate the whole embryos from fruits. Sub-culture the separated embryos in 0.5 MS medium with 15g/L sucrose and 7g /L agar under long day conditions (25°C, 16 hours light and 8 hours dark)

### Chromosome spreading

The unopened flower buds (meiotic stages) were collected into 1.5 mL Carnoy's fixation buffer (absolute ethanol: acetic acid, 3:1, v/v) for vacuumizing 20 minutes. Refresh the Carnoy's fixation buffer and place materials overnight until the tissue turns white at room temperature. Replace the fixation buffer with 75% ethanol and restore the materials into 4° C. The chromosome spreading process were performed as described by previous protocol with the following modifications (Wang, Y., Jiang, L., Zhang, T., Jing, J. & He, Y. ZmCom1 is required for both mitotic and meiotic recombination in maize. Front. Plant Sci. (2018) doi:10.3389/fpls.2018.01005; Ross, K. J., Fransz, P. & Jones, G. H. A light microscopic atlas of meiosis in Arabidopsis thaliana. Chromosom. Res. 4, 507-516 (1996)). Separate the individual anthers (length of anthers arranges from 1.5 mm to 3 mm at meiotic stages) from the flowers buds and digest the anthers with enzyme solution (0.3% (w/v) cellulase RS, 0.3% (w/v) pectolyase Y23, 0.3% (w/v) cytohelicase in citrate buffer, pH 4.7) for 2 hours at 37° C. Meiocytes were released from two or three anthers by crushing via tweezers within 45% acetic acid, and cover the coverslip without bubbles. Put the slides into liquid nitrogen until no sound is heard and remove the coverslip. The slides were dried by 70% ethanol, 85% ethanol and 100% ethanol for 5 minutes. Finally, 6 µl DAPI (10 µg/mL) was added to stain and indicate the chromosome after the slides dry out. The data was captured by Zeiss Axio Imager Z2 Upright Microscope and the data was analyzed by ZEN software.

### Homologous protein sequence searching and phylogenetic tree analysis

The protein sequence of AtSPO11-1 (AT3G13170), AtREC8/SYN1 (AT5G05490), AtCYCLIN A1;2 (AT1G77390), AtOSD1 (AT3G57860) and AtDMP8 (AT1G09157)/AtDMP9 (AT5G39650) were acquired from the Arabidopsis database TAIR (The Arabidopsis Information Resource, https://www.arabidopsis.org/) and then blast against the phytozome database (https: //phytozome-next, jgi.doe.gov/), UniProt protein database (https://www.uniprot.org/balst) and NCBI database (https://blast.ncbi.nlm.nih.gov/Blast.cgi) to get the homologue protein sequence in other species. The multiple protein sequences were analyzed by Clustal X2 software and phylogenetic tree was generated by MEGA11 software.

### Whole genome sequencing (WGS)

The small amount (around 1 cm x 1 cm) of leaf samples was collected into 96 well format and then the DNA were isolated by Genomic Center at Max Planck institute for Plant Breeding Research based on CTAB extraction protocol. The pooled libraries were sent to Novogene by using the Illumina NovaSeq 6000, and obtain around 20 Gbp data per sample.

### In vitro regeneration of tomato plants

The young branches of the plants in greenhouse were selected to perform the regeneration *in vitro.* In brief, a few suitable fresh shoots were cut and sterilized for 15 minutes in a commercial bleach or 12% Natriumhypochlorit (NaClO) (1:4 dilution) combing with 0.02% Tween, and then wash them for 3-4 times in sterile water. After that, transfer them into 0.5 MS-10 solid medium. Plants will grow in culture room in long day conditions (16 hours' light/ 8 hours' dark) and got sub-cultured on the same medium after 4-5 weeks.

### High through-put pollen size analysis

The Multisizer 4e particle sizer and counter (https://www.beckman.de/cell-counters-and-analyzers/multisizer-4e, BECKMAN Counter, Germany) was used to measure the particle diameter and volume of single pollen from single opening flower (De Storme, N., Zamariola, L., Mau, M., Sharbel, T. F. & Geelen, D. Volume-based pollen size analysis: an advanced method to assess somatic and gametophytic ploidy in flowering plants. Plant Reprod. 26, 65-81 (2013)). All the pollen was collected from single opening flower by a modified Oral-B electric toothbrush into 10 mL Coulter ISOTON II Diluent contained by 25 x 25 mL vials and caps (Coulter Counter Accuvette ST, smart technology). The calibration step of the instrument was performed by using nominal 20 µm latex beads (Counter CC sizer Standard L20, REF6602798). The related run settings data are as follows: aperture tube: GLWR Custom Aperture Tube 100 µm (Product No: A36394); aperture current: 800 µA; preamp gian: 4; Flow rate: approximately 39 µL/s; three different replications with 1500 µL analytic volume. The pollen size measurement was performed by MS4e User's Manual. The final data were analyzed by Multisizer 4e software and GraphPad Prism 9 software, and p-values were calculated using an ordinary one-way ANOVA.

### Websites for gene and protein analysis

The website for prediction of transmembrane helices in proteins:
https://services.healthtech.dtu.dk/service.php?TMHMM-2.0.

The gene structure analysis website: http://wormweb.org/exonintron.

The gene annotation website of phytozome 13: https://phytozome-next.jgi.doe.gov/.

The database for tomato reference and blast is: https://solgenomics.net/tools/blast/.

The website of conserved domain database is: https://www.ebi.ac.uk/interpro/.

The website for protein sequence analysis and coiled coil prediction:
https://toolkit.tuebingen.mpg.de/tools/marcoil.

The website of the Solanaceae Genomics Network database is:
https://solgenomics.net/.

For homologue proteins searching against NCBI database, the inventors selected the maximum number (250) of aligned sequences to display.

### Parental genome assembly

Haplotype-resolved assemblies of Funtelle and Maxeza F1 hybrids were generated by running Hifiasm (Cheng, H., Concepcion, G. T., Feng, X., Zhang, H. & Li, H. Haplotype-resolved de novo assembly using phased assembly graphs with hifiasm. Nat. Methods 2021 182 18, 170-175 (2021)) on HiFi reads with default settings. The alignment of raw contigs against the MbTMV reference genome was generated using minimap2 (Li, H. Minimap2: pairwise alignment for nucleotide sequences. Bioinformatics 34, 3094-3100 (2018)) and visualized using D-GENIES. For each haplotype, contigs were anchored and scaffolded to chromosome-scale pseudomolecules by Ragtag (Alonge, M. et al. Automated assembly scaffolding using RagTag elevates a new tomato system for high-throughput genome editing. Genome Biol. 23, 1-19 (2022)). K-mer analysis and genome size estimation was done using genomescope (Vurture, G. W. et al. GenomeScope: fast reference-free genome profiling from short reads. Bioinformatics 33, 2202 (2017)).

### Marker detection

The inventors first identified and assigned SNP markers into two haplotypes. For detecting markers between MbTMV and Micro-Tom, the inventors first aligned the Illumina and HiFi reads of both parental genomes against MbTMV (Rengs, W. M. J. van et al. A chromosome scale tomato genome built from complementary PacBio and Nanopore sequences alone reveals extensive linkage drag during breeding. Plant J. (2022) doi:10.1111/TPJ.15690) using bwa-mem (Li, H. Aligning sequence reads, clone sequences and assembly contigs with BWA-MEM. (2013)) and minimap2 (Cheng, H., Concepcion, G. T., Feng, X., Zhang, H. & Li, H. Haplotype-resolved de novo assembly using phased assembly graphs with hifiasm. Nat. Methods 2021 182 18, 170-175 (2021)), respectively. SNPs were then detected using GATK HaplotypeCaller and hard-filtering (Poplin, R. et al. Scaling accurate genetic variant discovery to tens of thousands of samples. bioRxiv 201178 (2017) doi:10.1101/201178). The inventors retrieved homozygous Micro-Tom SNPs detected using both HiFi and Illumina read and do not match any SNP in the MbTMV sample, separating ambiguous markers. The inventors also selected markers that differs between MbTMV-MT and Funtelle by selecting homozygous Funtelle SNPs that do not overlap any MbTMV and MT SNPs. Similar approach is used to detect segregating markers between MbTMV-MT and Maxeza.

For identifying unique markers in each of the four different haplotypes in the tetraploid tomato, the inventors initially needed to determine the polymorphisms between the haplotypes of Funtelle and Maxeza. The inventors separately selected the markers in the *MiMe* tetraploids crossed with Funtelle haplotype from those crossed with Maxeza haplotypes. Both follow the same method. The succeeding steps describe the selection of markers on MiMe tetraploids with Funtelle haplotypes. Both scaffolded assemblies of Funtelle haplotypes the inventors aligned against MbTMV by minimap2 and were then processed by MuMmer *dnadiff* (Kurtz, S. et al. Versatile and open software for comparing large genomes. Genome Biol. 5, 1-9 (2004)) to identify SNPs. To validate that SNPs are heterozygous between the two Funtelle haplotypes, the inventors retrieved the assembly-based SNPs that matched heterozygous SNPs reported from Funtelle HiFi and Illumina reads.

Afterwards, the inventors retrieved those that do not overlap any SNPs from MbTMV and MT. The resulting set of SNPs uniquely identify Funtelle haplotypes from both MbTMV and MT. For unique MT SNPs, the inventors retrieved homozygous SNPs not overlapping any MbTMV and Funtelle SNPs. For unique MbTMV SNPs, the inventors reported the genomic positions that shows homozygous SNPs for both MicroTom and Funtelle. To reduce false markers, the inventors excluded the TMV region. The resulting set of SNPs are considered as unique parental markers.

### Checking crossover and aneuploids

Trimming and quality checking of Illumina reads is done using trimgalore (https://github.com/FelixKrueger/TrimGalore). The alignment of reads and SNP calling were performed using bwa-mem and GATK HaplotypeCaller. For each sample, the inventors computed the average allele frequency in a sliding 1-Mb window with 50 kb step size. The inventors visually inspected the deviations from the expected frequency to differentiate F2 from F1 and *MiMe* samples and to infer presence or absence recombination. The inventors also used the allele frequency information to confirm that some of the samples are triploid. Some of the samples are showing unexpected allele frequencies in some genomic regions or chromosomes so the inventors decided to check deviations from the average read coverage to infer chromosomes fragmentation or aneuploidy. Lastly, to determine if all four haplotypes are present in a tetraploid tomato, the inventors counted the number of unique parental markers that can be observed in each sample and compared it against the control hybrids.

### Counting gene dosage

The inventors selected specific genes to count alleles or gene dosage. For gene with known causative mutations, the inventors checked them from the both the assemblies and Illumina reads. Syri was used to detect rearrangement relative to the MbTMV reference and to find haplotypes with introgressions (Goel, M., Sun, H., Jiao, W. B. & Schneeberger, K. SyRI: finding genomic rearrangements and local sequence differences from whole-genome assemblies. Genome Biol. 20, 1-13 (2019)). SNP density was computed to also infer regions of introgressions.

### Example 1 - Unreduced male and female gametes in sltam mutants and a Mitosis instead of Meiosis system in inbred tomato

In order to establish a *MiMe* system in tomato, the inventors first set out to identify genetic factors that control entry to the second meiotic division in tomato. The inventors identified that UVI4 and OSD1 (the plant-specific inhibitors of Anaphase-promoting complex/cyclosome (APC/C)) are single copy in tomato (Solyc10g080400), unlike Arabidopsis and rice where the function is split between two genes.

Meanwhile, there are nine A-type cyclins in tomato with only a single cyclin A1 gene (*SlTAM,* Solyc11g005090), with a predicted specific role in meiosis and highly similarity to AtCYCA1;2 in Arabidopsis. Two different CRISPR/Cas9 constructs targeting *SlOSD1* and one CRISPR/Cas9 construct targeting *SITAM* (Fig. 1a) were generated and transformed into the tomato cultivar Micro-Tom (SCOTT, J. W. & HARBAUGH, B. K. Micro-tom. A miniature dwarf tomato. Circ. - Univ. Florida. Agric. Exp. Station. (1989)). Despite five independent transformation attempts, the inventors were unable to generate null diploid *slosd1* mutants which is consistent with a recent report about SlUVI4/SlOSD1 (Di, S. et al. Tomato UVI4 homologue modulates cell expansion to participate heat-stimulated hypocotyl elongation. Environ. Exp. Bot. 201, 104963 (2022)), and suggests SlOSD1 has essential functions in mitotic division. In contrast, the inventors isolated 33 To diploid plants with the construct targeting *SITAM,* and went on to further characterize five different alleles (Fig. 1a).

All five *sltam* homozygous mutants exhibited normal vegetative development and could produce between 28.53%-47.75% viable pollen grains that were larger than wild type according to Scanning electron microscopy (SEM) data and Alexander staining result (Fig. 1b). Using high-throughput particle size detection, the inventors quantified the diameter and volume of single wild-type and *sltam* pollen, and found a monomodal size distribution in wild type pollen, whereas all five *sltam* lines produced pollen with a bimodal size distribution (Fig.1c). Cytological analysis of the *sltam-1* allele demonstrated many meiocytes stall at the dyad stage and do not enter meiosis II. All five *sltam* mutants produced lighter fruits that contained less seeds compared to wild-type controls. All five *sltam* mutants had a high percentage (11.23%-42.19%) of undeveloped abnormal seeds (Fig.1d) but could also produce seeds that were considerably larger than wild type seeds which gave rise to tetraploid offspring (Fig.1e, 1f). Taken together, *sltam* mutants can skip the second meiotic division in both male and female meiosis and generate the unreduced gametes.

To engineer a complete *Mitosis instead of Meiosis* system in tomato, the inventors developed a CRISPR/Cas9 construct for simultaneously mutating *SISPO11-1* (expected to ablate meiotic recombination initiation), *SlREC8* (expected to abolish sister chromatid cohesion) and *SITAM* (Fig.1g). 27 T₀ lines were regenerated and a subset were backcrossed to wild-type to remove the transgene and used for conventional genetic analysis. In total the inventors isolated two *slspo11-1* alleles, two *slrec8* alleles, one further *sltam* allele and higher order double and triple mutant combinations. All lines were self-sterile apart from the *sltam* single mutants and the *slspo11-1 slrec8 sltam* triple mutant (Fig.1h, j). Meiotic defects in *slspo11-1* and *slrec8* were consistent with the respective absence of meiotic recombination and sister chromatid cohesion, whereas the *slspo11-1 slrec8 sltam* triple mutant exhibited a large number of cells at the dyad stage, consistent with *Mitosis instead of Meiosis.* Unlike single *sltam* mutants, the *putative MiMe* line produced only unreduced or dead pollen grains (Fig.1i), consistent with the idea that any meiocytes that enter meiosis II lead to non-viable spores/gametes. Like the single *sltam* mutants, the *MiMe* line produced smaller fruits with few tetraploid seeds (Fig.1k, 1i). These results demonstrate that a MiMe system (apomeiosis) could be established in inbred tomato through mutation of *SISPO11-1, SlREC8* and *SITAM.*

### Example 2 - Mitosis instead of Meiosis in three hybrid tomato genotypes

The inventors implemented the MiMe system in three hybrid tomato genotypes using a streamlined CRISPR/Cas9 construct (Fig.2a). In total the inventors regenerated 32 transgenic lines in the model tomato hybrid Moneyberg-TMV x MicroTom, 40 transgenic lines in the date-tomato commercial hybrid "Funtelle" and 37 transgenic lines in the truss tomato commercial hybrid "Maxeza". Of these the inventors identified 6 MbTMV-MT, 3 Funtelle and 3 Maxeza lines that had biallelic mutations on *SlSPO11-1, SlREC8* and *SlTAM.* The inventors focused on one putative hybrid *MiMe* line per hybrid (Fig.2b) and found all were capable of producing unreduced pollen (Fig.2c, d).

The inventors performed the chromosome spreading on these three different *MiMe* lines for cytological analysis of meiosis (Fig.2e, f). In wild type, during meiosis I homologous chromosomes replicate, pair, synapse and subsequently generate twelve highly condensed paired bivalents that subsequently segregate (Fig.2e). During meiosis II, sister chromatids segregate and leading to tetrads (Fig.2e). In contrast, the hybrid *MiMe* mutants went through a mitotic-like cell division. At prophase, the 24 univalents were identifiable at diakinesis and the inventors observed the balanced segregation of the 24 chromatids to produce dyads in *MiMe* mutants (Fig.2f). These findings illustrate the possibility of obtaining unreduced clonal gametes in hybrid *MiMe* mutants.

Compared with MbTMV-MT F1 hybrid, diploid hybrid *MiMe* mutants produced the slightly smaller fruits that contained few seeds (Fig.2g, h). However, theseds were bigger than wild type seeds and gave rise to tetraploid offspring at high penetrance (Fig. 2i, j).

### Example 3 - Stable inheritance of genome-wide heterozygosity and plant phenotypes through seeds

The inventors sequenced the hybrid *MiMe* offspring and used polymorphic genetic markers between the parental genomes (MbTMV and MT) to compute allele frequency and infer the presence or absence of recombination. As expected F1 plants were heterozygous across the genome while F2 plants derived from F1 plants showed divergence from the heterozygous state, indicating the presence of crossovers along the chromosomes (Fig.3a). In contrast, tetraploid *MiMe* offspring demonstrated the same pattern as F1 hybrid controls without any meiotic recombination (Fig.3a). In a subset (3/12) of the tetraploid plants the inventors identified potential loss of local chromosome regions (Fig.3a). Next the inventors extensively explored the phenotypic behavior of hybrid *MiMe* offspring and control F1 and F2 plants.

For the phenotyping experiments, the inventors focused on plant height (28 days, 42 days, 56 days and 70 days after transplanting), fruit weight and seed number of the first ten ripened fruits, and morphology on the eighth leaf (Fig.3b, c). The inventors found highly diversity phenotypes among the MbTMV-MT F2 offspring (due to random segregation and recombination), while highly consistent phenotypes occurred among the F1 hybrid and *MiMe* clonal offspring (Fig.3b, c).

To provide proof-of-concept that the *MiMe* system could be used for synthetic apomixis, the inventors made use of the *sldmp* maternal haploid induction system (Zhong, Y. et al. In vivo maternal haploid induction in tomato. Plant Biotechnol. J. (2021) doi:10.1111/PBI.13755). The inventors isolated four independent *sldmp* alleles which exhibited normal vegetative development and pollen viability, but dramatically reduced seed setting. Consistent with the previous report, the inventors identified haploid plants in selfing and backcrossed (*sldmp* mutants as pollen donor) offspring, yet also found embryo rescue could increase the haploid induction rate (6/157, 3.82% haploid induction rate). Towards clonal reproduction the inventors pollinated hybrid MiMe plants with *sldmp* pollen and rescued embryos leading to one clonal embryo and plant (190 crosses led to 186 fruits, 17 rescued embryos, of which one was a clone) (Fig. 3d, Fig.5). The single clonal plant was fully consistent with the maternal genotype - it was diploid and exhibited genome-wide heterozygosity (Fig.3a). The pollen viability/diameter and leaf morphology were also fully consistent with the mother (Fig.3a, e, f, g and h), indicating it was a true clone.

### Example 4 - Polyploid genome design and generation of tetraploid hybrid plants containing four unique genome haplotypes

The inventors set out to harness the MiMe system to generate "4-Haplotype" (4-H) plants that contained the complete genetic repertoire of their four inbred grandparents. To this end the inventors designed two sets of crosses between hybrid MiMe plants (MbTMV-MT*^{MiMe}* x Maxeza*^{MiMe}*; MbTMV-MT*^{MiMe}* x Funtelle*^{MiMe}*) to generate tetraploid hybrid offspring with four unique genome haplotypes (Fig.4a). The inventors made use of platinum grade assemblies of inbred grandparental lines MbTMV and Micro-Tom, and developed haplotype-resolved assemblies of the hybrid parental lines (Funtelle and Maxeza) to identify unique SNPs for each haplotype in each cross (i.e., MbTMV Vs Micro-Tom Vs Funtelle^{hap1} Vs Funtelle^{hap2}; MbTMV Vs Micro-Tom Vs Maxeza^{hap1} Vs Maxeza^{hap2}).

Next, the inventors germinated seeds from the two crosses and set out to fully characterize the distinct haplotypes in a set of 18 putative 4-H plants (13 from MbTMV-MT*^{MiMe}* x Maxeza*^{MiMe}* and 5 from MbTMV-MT*^{MiMe}* x Funtelle*^{MiMe}*) using whole genome sequencing. Using the haplotype-specific SNPmarkers, the presence of all four parental genomes in each 4H plant was validated (Fig.4b, c). In addition, the inventors found that each 4H plant inherited mutations on the MiMe target genes that were distinct to the respective parents. Furthermore, cytological analysis of a subset of lines demonstrated the 4-H plants had the expected 48 chromosomes (Fig.4d and Fig.6).

By examining allele frequency, the inventors confirmed equal dosage from both parents in 16 of 18 4-Haplotype (4-H) plants analyzed, while MbTMV-MT-Maxeza*^{MiMe-8}* had one truncated chromosome and MbTMV-MT-Funtelle*^{MiMe-4}* appeared to have additional chromosome copies (Fig.8). Remarkably, phenotyping tests of 4-H plants demonstrated normal vegetative growth and the production of well-organized branches with seedless fruits (Fig.4e, f, and Fig.7).

The Funtelle and Maxeza assemblies contain genomic regions that are distinct from MbTMV and Micro-Tom due to recent introgression of resistance genes from wild tomato species into elite germplasm. In the inventor's analysis of the 4-H plants it was found that multiple favorable alleles can be present, which the inventors tallied by checking allele identity in all parental haplotypes, SNP distributions in regions of interest or by whole genome alignment and synteny analysis (Fig.4g). Genome comparison revealed the introgression of *Meloidogyne incognita* (Mi) resistance in Funtelle-1 (Fig.4h) and its absence in Maxeza. Moreover, MbTMV-MT-Maxeza*^{MiMe}* plants have more Tomato Mosaic Virus (TMV) resistance alleles than MbTMV-MT-Funtelle*^{MiMe}*. In summary, it was found that the 4-H plants contain four genomes directly descended from their four inbred grandparents, essentially doubling the genetic space for introgression breeding and providing novel opportunities for crop improvement.

### Example 5 - Engineering Mitosis instead of Meiosis

The inventors developed a Mitosis instead of Meiosis (*MiMe*) system in tomato, and applied it in the context of clonal reproduction, via combination with maternal haploid induction, and polyploid genome design, via the hybridization of independent hybrid *MiMe* plants. The *MiMe* system the inventors developed in tomato differs from the favored systems in Arabidopsis and rice that rely on the mutation of *OSD1* for avoiding entry of the second meiotic division. In Arabidopsis and rice, there are two copies of the *OSD1* gene where it appears that one gene copy is largely meiosis-specific, and genetic analysis in Arabidopsis has shown double mutants are embryo lethal (Iwata, E. et al. GIGAS CELL1, a novel negative regulator of the anaphase-promoting complex/cyclosome, is required for proper mitotic progression and cell fate determination in Arabidopsis. Plant Cell 23, 4382-4393 (2011).

In many plant species, including tomato, there is just a single *OSD1* gene which the inventors believe will have essential functions during early plant development. Therefore, in this study, the inventors generated *tam* mutants for the first time in a crop species. Consistent with previous results from Arabidopsis the inventors show that the mutation of *tam* can restore the sterility of *spo11-1 rec8* double mutants, and lead to a mitosis-like division in place of meiosis. While the *MiMe* system can lead to clonal, unreduced gametes in both male and female reproduction, it was found that some chromosome fragmentation in the tetraploid offspring which could be explained by incomplete penetrance of *slspo11-1, slrec8* or *sltam.* For example, the inventors observed a low frequency of unbalanced segregation during meiosis I in spo11-1rec8 double mutants, and also found some defects during meiosis I in *tam* single mutants. Therefore, it's necessary to screen novel players for cell cycle regulation and skip the second cell division at full penetration. Moreover, the different tomato genetic backgrounds may influence the fertility of *MiMe* mutants; MbTMV-MT*^{MiMe}* exhibited highert fertility compared with Funtelle*^{MiMe}* and Maxeza*^{MiMe}*. Similar results were also found in rice *MiMe* mutants where Chunyou84 (CY84), Hwayoung and Nipponbare backgrounds showed different levels of fertility (Mieulet, D. et al. Turning rice meiosis into mitosis. Nat. Publ. Gr. 26, 1242-1254 (2016); Wang, C. et al. Clonal seeds from hybrid rice by simultaneous genome engineering of meiosis and fertilization genes. Nat. Biotechnol. 37, 283-286 (2019)).

### Example 6 - Synthetic apomixis in tomato

The inventors made use of our *MiMe* system to establish approaches for clonal embryo formation in tomato. In this study, the inventors pollinated hybrid *MiMe* plants with pollen from the maternal haploid inducer *Sldmp* and subsequently performed embryo rescues as the inventors expected failed endosperm development due to absence of a 2:1 maternal:paternal ratio. Despite a large number of crosses the inventors were able to isolate just a single clone of the *MiMe* mother genotype. This can be cumulatively explained by the reduced fertility of both parental lines, the low haploid induction rate of *Sldmp* and potentially also an important role for functional endosperm development early in tomato embryonic development.

Unlike rice where a high seed-setting rate is crucial for commercial success of apomictic varieties (Wei *et al*., 2023; Vernet *et al*., 2022), in crops like tomato or potato where the seed is not the final plant product, the inventors believe even moderately penetrant synthetic apomixis systems could be employed commercially when combined with strict seed quality screening measures at the seed production stage. As such the inventors propose that the proof-of-principle the inventors provide here for clonal embryo and offspring formation in tomato may represent the first step towards synthetic apomixis in this important vegetable crop.

### Example 7 - Polyploid genome design

The inventors provided a blueprint for the generation of tetraploid hybrid plants that contain the full genetic inheritance of their four inbred grandparents. This represents the first step towards controlled polyploid genome design and has the potential to maximize genetic heterozygosity and thereby allow autopolyploid heterosis to be actually exploited in agriculture. The implementation of polyploid genome design in plant breeding will require the development of four-way heterotic groups which could be driven by using genomic selection to identify higher-order combining abilities between grandparental lines. Beyond this, the genetic space for introgression of wild alleles is essentially doubled in tetraploids meaning that breeders could incorporate more unique characteristics (e.g., abiotic and biotic stress tolerance) in elite lines that were previously abandoned due to polygenic inheritance or prohibitive linkage drag (Zhang, C. et al. Genome design of hybrid potato. Cell 184, 3873-3883.e12 (2021); Gao, C. Genome engineering for crop improvement and future agriculture. Cell 184, 1621-1635 (2021); Markel, K. & Shih, P. M. From breeding to genome design: A genomic makeover for potatoes. Cell 184, 3843-3845 (2021)). For example, our blueprint could even facilitate the introgression of a complete "wild" genome in a background with three "domesticated" genomes. Further still, four genomes from four distinct tomato species could be combined within a single plant. Given that some important pathways for plant defense involve a plethora of genes this could lead to the development of highly resistant plant genotypes. Furthermore, polyploidy can allow the more acute control of genes that are very sensitive to gene dosage due to the buffering epistatic effect of a genome containing four complete haplotypes.

From a wider perspective the inventors propose that polyploid genome design can allow the controlled engineering of organisms with three or more sets of chromosomes with pre-defined DNA sequences. Hence, the inventors propose that maximizing genetic diversity whist increasing genome ploidy through polyploid genome design has the potential to unleash heterosis in a myriad of crops and lead to completely novel crop breeding schemes.

### Example 8 - Unreduced clonal gamete formation and polyploid genome design

### Exemplary Mutagenesis Protocol

1) Identification of *SPO11-1, REC8* and *TAM* genes in the species of interest based on phylogenetic analysis and sequence alignment. Sequences (genomic DNA, cDNA and proteins) from tomato, potato, eggplant and pepper are presented herein.
2) Mutagenesis of the identified genes individually or in combination in inbred or hybrid plant varieties using
   i) approaches based on targeted mutation of genes by engineered nucleases including CRISPR/Cas9, CRISPR-Cas12a (cpf1), Base editors (e.g., cytidine base editors), Prime editors (e.g., fusion proteins between Cas9 H840A nickase and a reverse transcriptase enzyme), TALENs, ZFNs, Meganucleases or other targetable nucleases. Example guide RNA sequences for the mutation of *SPO11-1, REC8* and *TAM* genes in tomato and potato by CRISPR/Cas9 are presented in the sequence list. Targeted mutation of genes by engineered nucleases can be carried out by stable genetic transformation (e.g., agrobacterium or particle gun) of recombinant DNA containing cassettes that express engineered nucleases, or by transient delivery of protein/ribonucleoprotein engineered nuclease complexes into plant protoplasts/cells/tissues/organs.
   ii) approaches based on traditional mutagenesis using chemicals (e.g., Ethyl methanesulfonate), irradiation (e.g., gamma irradiation), transposon activation (e.g., Mutator elements), or similar.
3) DNA extraction from mutagenized plants, or offspring thereof. This can be carried out using standard methods (e.g., CTAB method) or high throughput methods (e.g., Kingfisher robot).
4) Screening of DNA from mutagenized plants, or offspring thereof, in order to identify plants that contain mutations predicted to ablate or reduce the function of the above-mentioned genes. For example, mutations in DNA can be identified by the amplification of said genes by PCR followed by DNA sequencing (e.g., Sanger/Illumina/PacBio) or enzymatic digestion (e.g., dCAPS). With respect to introduced genetic mutations this could include the introduction of a protein frame shift (e.g. any insertion or deletion that is not a multiple of three), failures in splicing (e.g. mutation of GT splice donor, a splicing branch point or an AG splice acceptor) or the introduction of a premature stop codon (TAA, TAG, TGA). Any other genetic insertions or deletions that cause modifications to the protein sequence may also lead to a considerable loss of function (e.g., due to the loss of even just a single critical amino acid).
5) If the mutagenesis was not carried out in a combinatorial manner (e.g., multiplex genome editing) but by individual mutagenesis of each gene then the different mutations can be combined by hybridization and genotyping (see part 3 and 4) to establish triple mutant plants.
6) Validation that plants that contain mutations in the *SPO11-1, REC8* and *TAM* genes are suitable for polyploid breeding can be carried out by checking the pollen for the presence of unreduced pollen grains. This can be carried out by alexander staining of pollen combined with imaging analysis to see the size of the viable pollen and/or through the detection of pollen size through automated methods (e.g., particle size detection by coulter counter). In addition, spreading of meiotic chromosomes combined with imaging analysis can be used to check for the replacement of a normal meiotic cell division with a mitotic-like cell division.
7) Plants that contain mutations in the *SPO11-1, REC8* and *TAM* genes that have been validated (step 6) can be vegetatively propagated indefinitely, for example by making new cuttings from young branches and/or via tubers.
8) Hybridization of independent hybrid plants that contain mutations in *SPO11-1, REC8* and *TAM* genes to generate tetraploid plants that maintain the full heterozygosity of the two parents.
9) Germination of tetraploid hybrid seeds and plant propagation. Check the ploidy level by (1) flow cytometry of leaf nuclei stained with DAPI and/or (2) chromosome counting from root tip and/or meiotic cells (meiocytes).
10) DNA extraction of tetraploid hybrid plants.
11) Genotyping analysis of tetraploid hybrid plants to determine whether the plants maintain the full heterozygosity of the two parents. This can be conventional genetic marker analysis and/or by high throughput sequencing and bioinformatic analysis.

### Exemplary Gene Knockdown Protocol

1) Identification of *SPO11-1, REC8* and *TAM* genes in the species of interest based on phylogenetic analysis and sequence alignment. Example sequences (genomic DNA, cDNA and proteins) from four nightshade species (tomato, potato, eggplant and pepper) are presented herein.
2) Reducing the expression level of the identified genes in meiotic cells in floral buds using RNAi approaches.
   i) including artificial microRNAs (amiRNAs), MiRNA-induced gene silencing (MIGS), Virus-induced gene silencing (VIGS), co-suppression constructs or other RNA interference-based approaches. Targeted knockdown of genes by amiRNAs can be carried out by stable genetic transformation (e.g., agrobacterium or particle gun) of recombinant DNA containing cassettes that express amiRNAs.
3) RNA extraction from meiotic stage floral buds from plants that contain knockdown constructs against *SPO11-1, REC8* and *TAM* genes.
4) cDNA production from RNA extracted in step 3.
5) qRT-PCR analysis of *SPO11-1, REC8* and *TAM* genes using cDNA from step 4 to identify plants with reduced expression of said genes compared to control plants.
6) Validation that plants that have reduced expression of *SPO11-1, REC8* and *TAM* genes are suitable for polyploid breeding can be carried out by checking the pollen for the presence of unreduced pollen grains. This can be carried out by alexander staining of pollen combined with imaging analysis to see the size of the viable pollen and/or through the detection of pollen size through automated methods (e.g., particle size detection by coulter counter). In addition, spreading of meiotic chromosomes combined with imaging analysis can be used to check for the replacement of a normal meiotic cell division with a mitotic-like cell division.
7) Plants that have reduced expression of *SPO11-1, REC8* and *TAM* genes that have been validated (step 6) can be vegetatively propagated indefinitely, for example by making new cuttings from young branches and/or via tubers.
8) Hybridization of independent hybrid plants that that have reduced expression of *SPO11-1, REC8* and *TAM* genes to generate tetraploid plants that maintain the full heterozygosity of the two parents.
9) Germination of tetraploid hybrid seeds and plant propagation. Check the ploidy level by (1) flow cytometry of leaf nuclei stained with DAPI and/or (2) chromosome counting from root tip and/or meiotic cells (meiocytes).
10) DNA extraction of tetraploid hybrid plants.
11) Genotyping analysis of tetraploid hybrid plants to determine whether the plants maintain the full heterozygosity of the two parents. This can be conventional genetic marker analysis and/or by high throughput sequencing and bioinformatic analysis.

### Core Reference List

1. Hochholdinger, F. & Baldauf, J. A. Heterosis in plants. Current Biology vol. 28 R1089-R1092 (2018).
2. ter Steeg, E. M. S., Struik, P. C., Visser, R. G. F. & Lindhout, P. Crucial factors for the feasibility of commercial hybrid breeding in food crops. Nat. plants (2022) doi: 10.1038/S41477-022-01142-W.
3. Washburn, J. D. & Birchler, J. A. Polyploids as a 'model system' for the study of heterosis. Plant Reprod. 27, 1-5 (2014).
4. Groose, R. W., Talbert, L. E., Kojis, W. P. & Bingham, E. T. Progressive heterosis in autotetraploid alfalfa: Studies using two types of inbreds. Crop Sci. 29, 1173-1177 (1989).
5. Levings, C. S., Dudley, J. W. & Alexander, D. E. Inbreeding and Crossing in Autotetraploid Maize. Crop Sci. 7, 72-73 (1967).
6. Mok, D. W. S. & Peloquin, S. J. Breeding value of 2n pollen (diplandroids) in tetraploid x diploid crosses in potatoes. Theor. Appl. Genet. 46, 307-314 (1975).
7. Mieulet, D. et al. Turning rice meiosis into mitosis. Nat. Publ. Gr. 26, 1242-1254 (2016).
8. d'Erfurth, I. et al. Turning Meiosis into Mitosis. PLoS Biol. 7, e1000124 (2009).
9. Wang, Y., van Rengs, W. M. J., Zaidan, M. W. A. M. & Underwood, C. J. Meiosis in crops: from genes to genomes. J. Exp. Bot. (2021) doi:10.1093/JXB/ERAB217.
10. Marimuthu, M. P. A. et al. Synthetic clonal reproduction through seeds. Science (80-. ). 331, 876 (2011).
11. Marimuthu, M. P. A. et al. Epigenetically mismatched parental centromeres trigger genome elimination in hybrids. Sci. Adv. 7, 1151 (2021).
12. Wang, C. et al. Clonal seeds from hybrid rice by simultaneous genome engineering of meiosis and fertilization genes. Nat. Biotechnol. 37, 283-286 (2019).
13. Khanday, I., Skinner, D., Yang, B., Mercier, R. & Sundaresan, V. A male-expressed rice embryogenic trigger redirected for asexual propagation through seeds. Nature 565, 91-95 (2019).
14. d'Erfurth, I. et al. The CYCLIN-A CYCA1;2/TAM Is Required for the Meiosis I to Meiosis II Transition and Cooperates with OSD1 for the Prophase to First Meiotic Division Transition. PLOS Genet. 6, e1000989 (2010).
15. Cifuentes, M. et al. TDM1 Regulation Determines the Number of Meiotic Divisions. PLoS Genet. 12, (2016).
16. Wang, Y. & Underwood, C. J. Apomixis. Curr. Biol. 33, R293-295 (2023).
17. Underwood, C. J. & Mercier, R. Engineering Apomixis: Clonal Seeds Approaching the Fields. https://doi.org/10.1146/annurev-arplant-102720-013958 73, (2022).
18. Stokstad, E. Unmixed blessing. Science (80-. ). 380, 684-687 (2023).
19. SCOTT, J. W. & HARBAUGH, B. K. Micro-tom. A miniature dwarf tomato. Circ. - Univ. Florida. Agric. Exp. Station. (1989).
20. Di, S. et al. Tomato UVI4 homologue modulates cell expansion to participate heat-stimulated hypocotyl elongation. Environ. Exp. Bot. 201, 104963 (2022).
21. Zhong, Y. et al. In vivo maternal haploid induction in tomato. Plant Biotechnol. J. (2021) doi:10.1111/PBI.13755.
22. Iwata, E. et al. GIGAS CELL1, a novel negative regulator of the anaphase-promoting complex/cyclosome, is required for proper mitotic progression and cell fate determination in Arabidopsis. Plant Cell 23, 4382-4393 (2011).
23. Wei, X. et al. Synthetic apomixis with normal hybrid rice seed production. Mol. Plant 0, (2023).
24. Vernet, A. et al. High-frequency synthetic apomixis in hybrid rice. Nat. Commun. 2022 131 13, 1-13 (2022).
25. Zhang, C. et al. Genome design of hybrid potato. Cell 184, 3873-3883.e12 (2021).
26. Gao, C. Genome engineering for crop improvement and future agriculture. Cell 184, 1621-1635 (2021).
27. Markel, K. & Shih, P. M. From breeding to genome design: A genomic makeover for potatoes. Cell 184, 3843-3845 (2021).
28. Čermák, T. *et al.* A multipurpose toolkit to enable advanced genome engineering in plants. *Plant Cell* 29, 1196-1217 (2017).
29. Liu, H. et al. CRISPR-P 2.0: An Improved CRISPR-Cas9 Tool for Genome Editing in Plants. Mol. Plant 10, 530-532 (2017).
30. Engler, C., Kandzia, R. & Marillonnet, S. A One Pot, One Step, Precision Cloning Method with High Throughput Capability. PLoS One 3, e3647 (2008).
31. Sun, H. J., Uchii, S., Watanabe, S. & Ezura, H. A highly efficient transformation protocol for Micro-Tom, a model cultivar for tomato functional genomics. Plant Cell Physiol. 47, 426-431 (2006).
32. Alexander, M. P. Differential Staining of Aborted and Nonaborted Pollen. http://dx.doi.org/10.3109/10520296909063335 44, 117-122 (2009).
33. Hu, Y. & Zhang, Z. GridFree: a python package of imageanalysis for interactive grain counting and measuring. Plant Physiol. 186, 2239-2252 (2021).
34. Gul, S. et al. Foliar epidermal anatomy of Lamiaceae with special emphasis on their trichomes diversity using scanning electron microscopy. Microsc. Res. Tech. 82, 206-223 (2019).
35. Galbraith, D. W. et al. Rapid flow cytometric analysis of the cell cycle in intact plant tissues. Science 220, 1049-1051 (1983).
36. Brinkman, E. K., Chen, T., Amendola, M. & Van Steensel, B. Easy quantitative assessment of genome editing by sequence trace decomposition. Nucleic Acids Res. 42, (2014).
37. Wang, Y., Jiang, L., Zhang, T., Jing, J. & He, Y. ZmCom1 is required for both mitotic and meiotic recombination in maize. Front. Plant Sci. (2018) doi:10.3389/fpls.2018.01005.
38. Ross, K. J., Fransz, P. & Jones, G. H. A light microscopic atlas of meiosis in Arabidopsis thaliana. Chromosom. Res. 4, 507-516 (1996).
39. De Storme, N., Zamariola, L., Mau, M., Sharbel, T. F. & Geelen, D. Volume-based pollen size analysis: an advanced method to assess somatic and gametophytic ploidy in flowering plants. Plant Reprod. 26, 65-81 (2013).
40. Cheng, H., Concepcion, G. T., Feng, X., Zhang, H. & Li, H. Haplotype-resolved de novo assembly using phased assembly graphs with hifiasm. Nat. Methods 2021 182 18, 170-175 (2021).
41. Li, H. Minimap2: pairwise alignment for nucleotide sequences. Bioinformatics 34, 3094-3100 (2018).
42. Alonge, M. et al. Automated assembly scaffolding using RagTag elevates a new tomato system for high-throughput genome editing. Genome Biol. 23, 1-19 (2022).
43. Vurture, G. W. et al. GenomeScope: fast reference-free genome profiling from short reads. Bioinformatics 33, 2202 (2017).
44. Rengs, W. M. J. van et al. A chromosome scale tomato genome built from complementary PacBio and Nanopore sequences alone reveals extensive linkage drag during breeding. Plant J. (2022) doi:10.1111 /TPJ.15690.
45. Li, H. Aligning sequence reads, clone sequences and assembly contigs with BWA-MEM. (2013).
46. Poplin, R. et al. Scaling accurate genetic variant discovery to tens of thousands of samples. bioRxiv 201178 (2017) doi:10.1101/201178.
47. Kurtz, S. et al. Versatile and open software for comparing large genomes. Genome Biol. 5, 1-9 (2004).
48. Goel, M., Sun, H., Jiao, W. B. & Schneeberger, K. SyRI: finding genomic rearrangements and local sequence differences from whole-genome assemblies. Genome Biol. 20, 1-13 (2019).

## Claims

1. A method of generating a parent plant capable of apomeiosis, so as to produce viable clonal male and female gametes having the same genome ploidy level as the parent plant, the method comprising:
(I) reducing the level or activity of an endogenous REC8 polypeptide in the parent plant or a cell thereof, so as to ablate sister chromatid cohesion;
(II) reducing the level or activity of an endogenous TAM polypeptide in the parent plant or a cell thereof, so as to prevent entry to the second meiotic division; and
(III) reducing the level or activity of an endogenous SPO11-1, SPO11-2, MTOPVIB, PRD1, PRD2, PRD3, or DFO polypeptide in the parent plant or a cell thereof, so as to ablate meiotic recombination;
wherein the parent plant is from the Solanaceae family.

2. The method of claim 1, wherein the parent plant is a tomato plant, and wherein:
(i) the endogenous REC8 polypeptide:
(a) has at least 90% sequence identity to the polypeptide of SEQ ID NO.: 6; and/or
(b) is encoded by a polynucleotide having at least 90% sequence identity to a polynucleotide selected from SEQ ID NOs.: 4 and 5;
(ii) the endogenous TAM polypeptide:
(a) has at least 90% sequence identity to the polypeptide of SEQ ID NO.: 9; and/or
(b) is encoded by a polynucleotide having at least 90% sequence identity to a polynucleotide selected from SEQ ID NOs.: 7 and 8; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 3, 12, 15, 18, 21, 24, and 27; and/or
(b) is encoded by a polynucleotide having at least 90% sequence identity to a polynucleotide selected from the group consisting of SEQ ID NOs.: 1, 2, 10, 11, 13, 14, 16, 17, 19, 20, 22, 23, 25, and 26.

3. The method of claim 1, wherein the parent plant is a potato plant, and wherein:
(i) the endogenous REC8 polypeptide:
(a) has at least 90% sequence identity to the polypeptide of SEQ ID NO.: 33; and/or
(b) is encoded by a polynucleotide having at least 90% sequence identity to a polynucleotide selected from SEQ ID NOs.: 31 and 32;
(ii) the endogenous TAM polypeptide:
(a) has at least 90% sequence identity to the polypeptide of SEQ ID NO.: 36; and/or
(b) is encoded by a polynucleotide having at least 90% sequence identity to a polynucleotide selected from SEQ ID NOs.: 34 and 35; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 30, 39, 42, 45, 48, 51, and 54; and/or
(b) is encoded by a polynucleotide having at least 90% sequence identity to a polynucleotide selected from the group consisting of SEQ ID NOs.: 28, 29, 37, 38, 40, 41, 43, 44, 46, 47, 49, 50, 52, and 53.

4. The method of claim 1, wherein the parent plant is an eggplant plant, and wherein:
(i) the endogenous REC8 polynucleotide:
(a) has at least 90% sequence identity to the polypeptide of SEQ ID NO.: 60; and/or
(b) is encoded by a polynucleotide having at least 90% sequence identity to a polynucleotide selected from SEQ ID NOs.: 58 and 59;
(ii) the endogenous TAM polypeptide:
(a) has at least 90% sequence identity to a polypeptide of SEQ ID NO.: 63; and/or
(b) is encoded by a polynucleotide having at least 90% sequence identity to a polynucleotide selected from SEQ ID NOs.: 61 and 62; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 57, 66, 69, 72, 75, 78, and 81; and/or
(b) is encoded by a polynucleotide having at least 90% sequence identity to a polynucleotide selected from the group consisting of SEQ ID NOs.: 55, 56, 64, 65, 67, 68, 70, 71, 73, 74, 76, 77, 79, and 80.

5. The method of claim 1, wherein the parent plant is a pepper plant, and wherein:
(i) the endogenous REC8 polypeptide:
(a) has at least 90% sequence identity to a polypeptide of SEQ ID NO.: 87; and/or
(b) is encoded by a polynucleotide having at least 90% sequence identity to a polynucleotide selected from SEQ ID NOs.: 85 and 86;
(ii) the endogenous TAM polypeptide:
(a) has at least 90% sequence identity to a polypeptide of SEQ ID NO.: 90; and/or
(b) is encoded by a polynucleotide having at least 90% sequence identity to a polynucleotide selected from SEQ ID NOs.: 88 and 89; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 84, 93, 96, 99, 102, 105, and 108; and/or
(b) is encoded by a polynucleotide having at least 90% sequence identity to a polynucleotide selected from the group consisting of SEQ ID NOs.: 82, 83, 91, 92, 94, 95, 97, 98, 100, 101, 103, 104, 106, and 107.

6. The method of any one of claims 1 to 5, wherein the method comprises reducing the level or activity of the endogenous REC8, TAM, and/or SPO11-1, SPO11-2, MTOPVIB, PRD1, PRD2, PRD3, or DFO polypeptide/s in the parent plant or a cell thereof by introducing genome modifications into the endogenous polynucleotides encoding the REC8, TAM, and/or SPO11-1, SPO11-2, MTOPVIB, PRD1, PRD2, PRD3, or DFO polypeptide/s.

7. The method of claim 6, wherein the genome modifications are introduced by:
(i) genome editing;
(ii) transposon activation; or
(iii) mutagenesis; optionally wherein the mutagenesis is by ethyl methanesulfonate or gamma irradiation.

8. The method of any one of claims 1 to 7, further comprising vegetatively propagating the parent plant, optionally vegetatively propagating using a side-shoot or tuber.

9. The method of any one of claims 1 to 7, further comprising producing a seed from the parent plant, wherein the seed has a genome ploidy level that is higher than, optionally double, the ploidy level of the parent plant;
optionally wherein the seed is produced:
(i) by selfing the parent plant; or
(ii) by crossing the parent plant with a second, different parent plant also having a reduced level or activity of the endogenous REC8, TAM, and SPO11-1, SPO11-2, MTOPVIB, PRD1, PRD2, PRD3, or DFO polypeptides;
further optionally wherein the first and second, different parent plants are both diploid hybrid plants, and wherein the seed comprises an embryo having a genome which comprises four genetically distinct genome haplotypes.

10. The method of any one of claims 1 to 7, further comprising producing a seed from the parent plant by crossing the parent plant with a second, different parent plant, wherein the second, different plant does not have a reduced level or activity of the endogenous REC8, TAM, and SPO11-1, SPO11-2, MTOPVIB, PRD1, PRD2, PRD3, or DFO polypeptides and is:
(i) diploid;
(ii) triploid; or
(iii) tetraploid.

11. The method of any one of claims 1 to 7, further comprising producing a seed from the parent plant by pollinating the parent plant with a haploid inducer plant, wherein the seed has a genome ploidy level that is the same as the ploidy level of the parent plant;
optionally wherein the haploid inducer plant has a reduced level or activity of an endogenous DMP polynucleotide, so as to inhibit gamete fusion;
further optionally wherein the endogenous DMP polynucleotide:
(i) has at least 90% sequence identity to a polynucleotide selected from the group consisting of: SEQ ID NOs.: 145, 146, 148, 149, 151, 152, 154, and 155; and/or
(ii) encodes a polypeptide having at least 90% sequence identity to a polypeptide selected from the group consisting of: SEQ ID NOs.: 147, 150, 153, and 156;
further optionally wherein the haploid inducer plant comprises a genome modification in the endogenous DMP polynucleotide.

12. A parent plant capable of apomeiosis, so as to produce viable clonal male and female gametes having the same genome ploidy level as the parent plant, having:
(I) a reduced level or activity of an endogenous REC8 polypeptide, so as to ablate sister chromatid cohesion;
(II) a reduced level or activity of an endogenous TAM polypeptide, so as to prevent entry to the second meiotic division; and
(III) a reduced level or activity of an endogenous SPO11-1, SPO11-2, MTOPVIB, PRD1, PRD2, PRD3, or DFO polypeptide, so as to ablate meiotic recombination;
wherein the parent plant is from the Solanaceae family.

13. The parent plant of claim 12, wherein the parent plant is:
(A) a tomato plant, and wherein:
(i) the endogenous REC8 polypeptide:
(a) has at least 90% sequence identity to the polypeptide of SEQ ID NO.: 6; and/or
(b) is encoded by a polynucleotide having at least 90% sequence identity to a polynucleotide selected from SEQ ID NOs.: 4 and 5;
(ii) the endogenous TAM polypeptide:
(a) has at least 90% sequence identity to the polypeptide of SEQ ID NO.: 9; and/or
(b) is encoded by a polynucleotide having at least 90% sequence identity to a polynucleotide selected from SEQ ID NOs.: 7 and 8; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 3, 12, 15, 18, 21, 24, and 27; and/or
(b) is encoded by a polynucleotide having at least 90% sequence identity to a polynucleotide selected from the group consisting of SEQ ID NOs.: 1, 2, 10, 11, 13, 14, 16, 17, 19, 20, 22, 23, 25, and 26;
(B) a potato plant, and wherein:
(i) the endogenous REC8 polypeptide:
(a) has at least 90% sequence identity to the polypeptide of SEQ ID NO.: 33; and/or
(b) is encoded by a polynucleotide having at least 90% sequence identity to a polynucleotide selected from SEQ ID NOs.: 31 and 32;
(ii) the endogenous TAM polypeptide:
(a) has at least 90% sequence identity to the polypeptide of SEQ ID NO.: 36; and/or
(b) is encoded by a polynucleotide having at least 90% sequence identity to a polynucleotide selected from SEQ ID NOs.: 34 and 35; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 30, 39, 42, 45, 48, 51, and 54; and/or
(b) is encoded by a polynucleotide having at least 90% sequence identity to a polynucleotide selected from the group consisting of SEQ ID NOs.: 28, 29, 37, 38, 40, 41, 43, 44, 46, 47, 49, 50, 52, and 53;
(C) an eggplant plant, and wherein:
(i) the endogenous REC8 polynucleotide:
(a) has at least 90% sequence identity to the polypeptide of SEQ ID NO.: 60; and/or
(b) is encoded by a polynucleotide having at least 90% sequence identity to a polynucleotide selected from SEQ ID NOs.: 58 and 59;
(ii) the endogenous TAM polypeptide:
(a) has at least 90% sequence identity to a polypeptide of SEQ ID NO.: 63; and/or
(b) is encoded by a polynucleotide having at least 90% sequence identity to a polynucleotide selected from SEQ ID NOs.: 61 and 62; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 57, 66, 69, 72, 75, 78, and 81; and/or
(b) is encoded by a polynucleotide having at least 90% sequence identity to a polynucleotide selected from the group consisting of SEQ ID NOs.: 55, 56, 64, 65, 67, 68, 70, 71, 73, 74, 76, 77, 79, and 80; or
(D) a pepper plant, and wherein:
(i) the endogenous REC8 polypeptide:
(a) has at least 90% sequence identity to a polypeptide of SEQ ID NO.: 87; and/or
(b) is encoded by a polynucleotide having at least 90% sequence identity to a polynucleotide selected from SEQ ID NOs.: 85 and 86;
(ii) the endogenous TAM polypeptide:
(a) has at least 90% sequence identity to a polypeptide of SEQ ID NO.: 90; and/or
(b) is encoded by a polynucleotide having at least 90% sequence identity to a polynucleotide selected from SEQ ID NOs.: 88 and 89; and
(iii) the endogenous SPO11-1, SPO11-2, MTOPVIB, PRD1, PRD2, PRD3, or DFO polypeptide:
(a) has at least 90% sequence identity to a polypeptide selected from the group consisting of SEQ ID NOs.: 84, 93, 96, 99, 102, 105, and 108; and/or
(b) is encoded by a polynucleotide having at least 90% sequence identity to a polynucleotide selected from the group consisting of SEQ ID NOs.: 82, 83, 91, 92, 94, 95, 97, 98, 100, 101, 103, 104, 106, and 107.

14. The parent plant of claim 12 or 13, wherein the parent plant comprises genome modifications in the endogenous polynucleotides encoding the REC8, TAM, and/or SPO11-1, SPO11-2, MTOPVIB, PRD1, PRD2, PRD3, or DFO polypeptide/s.

15. A plant part or plant cell of the parent plant of any one of claims 12 to 14, optionally wherein the plant part is a side-shoot, tuber, or a microspore or seed of the parent plant of any one of claims 12 to 14.
